(19) Europäisches Patentamt
European Patent Office
Office européen des brevets

(11) EP 0 558 570 B1

(12) EUROPÄISCHE PATENTSCHRIFT

(45) Veröffentlichungstag und Bekanntmachung des Hinweises auf die Patenterteilung:
**10.01.1996 Patentblatt 1996/02**

(21) Anmeldenummer: **91920342.2**

(22) Anmeldetag: **23.11.1991**

(51) Int Cl.[6]: **C07C 405/00**, A61K 31/557

(86) Internationale Anmeldenummer:
**PCT/DE91/00925**

(87) Internationale Veröffentlichungsnummer:
**WO 92/09573 (11.06.1992 Gazette 1992/13)**

(54) **CYCLOPENTANETHERDERIVATE, VERFAHREN ZU IHRER HERSTELLUNG UND IHRE PHARMAZEUTISCHE VERWENDUNG**

CYCLOPENTANE ETHER DERIVATIVES, METHODS FOR PREPARING THEM AND THEIR USE IN PHARMACEUTICALS

DERIVES D'ETHER DE CYCLOPENTANE, PROCEDE POUR LEUR FABRICATION ET LEUR APPLICATION PHARMACEUTIQUE

(84) Benannte Vertragsstaaten:
**AT BE CH DE DK ES FR GB GR IT LI LU NL SE**

(30) Priorität: **23.11.1990 DE 4037941**

(43) Veröffentlichungstag der Anmeldung:
**08.09.1993 Patentblatt 1993/36**

(73) Patentinhaber:
**SCHERING AKTIENGESELLSCHAFT**
**D-13342 Berlin (DE)**

(72) Erfinder:
- **REHWINKEL, Hartmut**
  **D-1000 Berlin 41 (DE)**
- **KLAR, Ulrich**
  **D-1000 Berlin 27 (DE)**
- **VORBRÜGGEN, Helmut**
  **D-1000 Berlin 7 (DE)**
- **THIERAUCH, Karl-Heinz**
  **D-1000 Berlin 37 (DE)**
- **VERHALLEN, Peter**
  **D-1000 Berlin 28 (DE)**

(56) Entgegenhaltungen:
- **Journal of Chemical Society, 1981, R.J. Cave et al.: "Total synthesis of prostaglandin-F2alpha, and the 9-O-benzyl derivatives of prostanglandins-F2alpha,-F1alpha, -D2, and -D1", Seiten 646-652, siehe den ganzen Artikel**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Cyclopentanether-Derivate, Verfahren zu ihrer Herstellung sowie ihre Verwendung als Hilfsstoffe für pharmakologische Untersuchungen und als Arzneimittel.

Cyclopentan-Derivate sind in den letzten Jahren intensiv bearbeitet worden, da vom Cyclopentan-System abgeleitete Prostaglandine wie z.B. $PGA_2$, $PGB_2$, $PGE_2$, 6-Oxo-$PGE_1$, $PGD_2$, $PGF_{2\alpha}$, $PGJ_2$ und ihre Analoga unterschiedlichste biologische Wirkungen z.B. auf das Herz-Kreislauf-, ZNS- oder Immun-System besitzen.

Aus Journ. of Chem. Soc., 1981, Seite 646-652 sind 9-0-Benzylderivate als Zwischenverbindungen bei der Synthese der natürlichen Protaglandine F und D bekannt, die möglicherweise die natürliche Prostaglandine antagonisieren.

Es wurde überraschenderweise gefunden, daß durch die Einführung eines Etherrestes in Position 9 (Prostaglandin-Zählweise) des Prostangerüstes in Kombination mit unterschiedlichsten Strukturmerkmalen in der unteren Kette sowie in Position 11 chemisch und metabolisch stabile Prostaglandin-Analoga erhalten werden, die in der Lage sind, die pharmakologischen Eigenschaften des instabilen Thromboxan-$A_2$ ($TXA_2$) bzw. $PGH_2$ sowie seiner stabilen Analoga wie z.B. U46619 oder U44069 am Rezeptor zu antagonisieren.

Die Verbindungen dieser Erfindung stellen deshalb wertvolle Hilfsmittel zur selektiven Therapie von Erkrankungen, die auf einen Überschuß an $TXA_2$ bzw. $PGH_2$ zurückzuführen sind, dar.

Die Erfindung betrifft Cyclopentanetherderivate der Formel I,

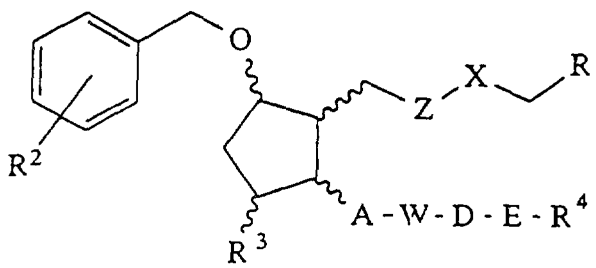

(I),

$R^1$ 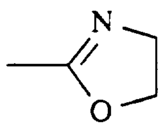 , 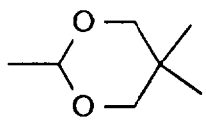 , 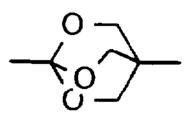 , 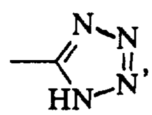,

$COOR^5$, wobei $R^5$ Wasserstoff oder gegebenenfalls durch Halogen, Phenyl, $C_1$-$C_4$-Alkoxy oder Di-($C_1$-$C_4$)alkylamino substituiertes $C_1$-$C_{10}$-Alkyl, $C_5$-$C_6$-Cycloalkyl, $C_7$-$C_{16}$-Aralkyl, durch Y substituiertes Phenacyl oder $C_6$-$C_{12}$-Aryl oder einen 5- oder 6- gliedrigen heterocyclischen Rest mit wenigstens einem N-, O- oder S-Atom bedeuten kann, oder $-CONHR^7$ mit $R^7$ in der Bedeutung Wasserstoff, $C_1$-$C_{10}$-Alkanoyl oder $C_1$-$C_{10}$-Alkansulfonyl sein kann,

Z eine Direktbindung, (Z)-CH=CH-,(E)-CH=CH-,-C≡C-,

X $-(CH_2)_p-$, $-CH_2-O-$, $-CH_2-S-$,

p 0 bis 5,

$R^2$ Y oder

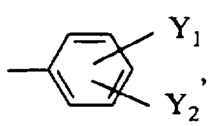,

$R^3$ Wasserstoff, F, $R^6$ oder $OR^6$,

A eine Direktbindung, (Z)-CH=CH-,(E)-CH=CH-,-C≡C-,

W eine Direktbindung, eine $-[(CH_2)_n-V]_q$-Gruppe oder eine $-(CH_2)_n-V-(CH_2)_q-V$-Gruppe, eine freie oder funktionell abgewandelte Hydroxymethylengruppe, eine freie oder funktionell abgewandelte

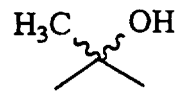 -Gruppe,

wobei die Hydroxygruppe jeweils α- oder β-ständig sein kann,

q 1 oder 2,

n 0 bis 2,

D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, $(CH_2)_m$-NH-$SO_2$-,

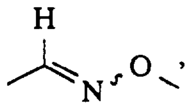 , 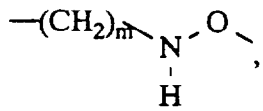 , 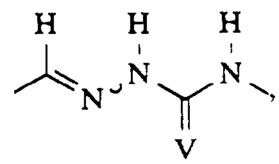 ,

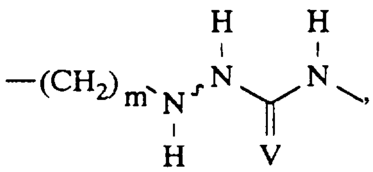 , 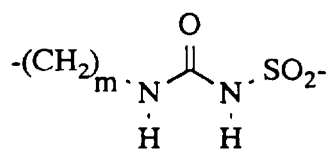 ,

m 0 bis 2

V ein O- oder S-Atom,

E eine Direktbindung, -C≡C- oder -CH=$CR^8$-, wobei $R^8$ Wasserstoff, $C_1$-$C_5$-Alkyl, Halogen oder Trifluormethyl,

AW , DE, unabhängig voneinander eine Direktbindung

$R^4$ durch Y substituiertes $C_1$-$C_{10}$-Alkyl, $C_3$-$C_{10}$-Cycloalkyl,

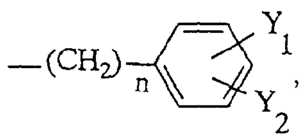 ,

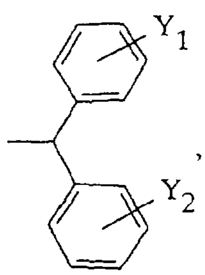 , 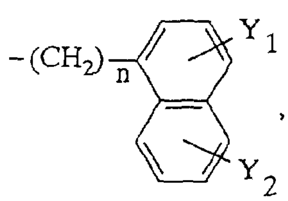 , 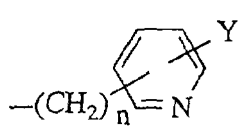 , 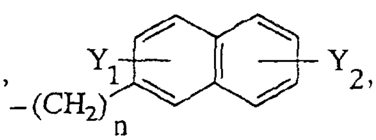

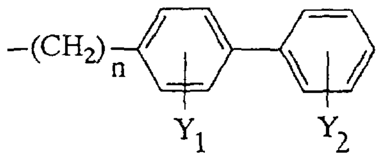, 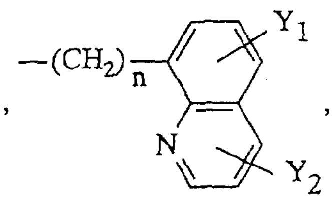, 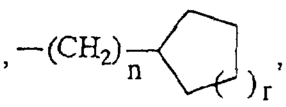

r 1 oder 2,

$Y_1$ und $Y_2$ gleich oder verschieden sind und Y bedeuten,

Y Wasserstoff, Halogen, CN, $N_3$, $CF_3$, $OR^6$, $NO_2$, $-CH_2-OR^6$, $COOR^6$ oder $C_1$-$C_{10}$-Alkyl,

$R^6$ Wasserstoff, $C_1$-$C_{10}$-Alkyl, durch Halogen substituiertes $C_6$-$C_{12}$-Aryl oder $C_7$-$C_{16}$- Aralkyl sein kann und, falls $R^5$ Wasserstoff bedeutet, deren Salze mit physiologisch verträglichen Basen , sowie die α-, β- oder γ-Cyclodextrinclathrate, sowie die mit Liposomen verkapselten Verbindungen der Formel I bedeuten;

wobei (9α, 11 α, 15S)-9-Benzyloxy-11,15-dihydroxy-5(Z), 13(E)-prostadiensäure, deren 15-Epi-Verbindung sowie 9 α-Benzyloxy-15-(t-butyldimethylsilyloxy)-11α-hydroxy-13(E)-prostensäure ausgeschlossen sind.

Die Definition 5- oder 6-gliedriger heterocyclischer Rest betrifft Heterocyclen, die wenigstens ein Heteroatom, vorzugsweise Stickstoff, Sauerstoff oder Schwefel enthalten und mono- oder bicyclisch sind. Beispielsweise seien genannt 2-Furyl, 3-Furyl, 2-Thienyl, 3-Thienyl, 2-Pyridyl, 3-Pyridyl, 4-Pyridyl, Chinolyl, Isochinolyl. Als Alkylgruppen $R^4$, $R^5$, $R^6$ und Y sind gerad- oder verzweigtkettige Alkylgruppen mit 1 - 10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl. Die Alkylgruppen $R^4$, $R^5$, $R^6$ und Y können substituiert sein durch Halogenatome, Hydroxygruppen, $C_1$-$C_4$-Alkoxygruppen, $C_6$-$C_{12}$-Arylgruppen, die durch Halogen substituiert sein können, Di-($C_1$-$C_4$)-Alkylamine und Tri-($C_1$-$C_4$)-Alkylammonium. Bevorzugt sind solche Alkylgruppen, die einfach substituiert sind. Als Substituenten seien beispielsweise genannt Fluor-, Chlor- oder Bromatome, Phenyl, Dimethylamino, Diethylamino, Methoxy, Ethoxy.

Als bevorzugte Alkylgruppen $R^4$, $R^5$, $R^6$ und Y sind solche mit 1 - 4 C-Atomen, wie z.B. Methyl, Ethyl, Propyl, Isobutyl, Butyl, zu nennen.

Als Arylgruppen $R^5$ und $R^6$ kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen eine Chlormethyl-, Fluormethyl-, Carboxyl-, $C_1$-$C_4$-Alkoxy- oder Hydroxygruppe. Bevorzugt ist die Substitution in 3- und 4-Stellung am Phenylring zum Beispiel durch Fluor, Chlor, $C_1$-$C_4$- Alkoxy oder Trifluormethyl oder in 4-Stellung durch Hydroxy.

Die Cycloalkylgruppen $R^4$ und $R^5$ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Cyclohexyl, Methylcyclopentyl, Methylcyclohexyl.

Die unter den Definitionen genannten $C_1$-$C_{10}$-Alkylgruppen sollen geradkettige oder verzweigte Alkylgruppen sein, wie sie für die vorstehenden Alkylgruppen bereits genannt wurden.

Die Hydroxygruppen in $R^2$, $R^3$ und Y können funktionell abgewandelt sein, beispielsweise durch Veretherung oder Veresterung, wobei die freie oder abgewandelte Hydroxygruppe in $R^3$ α- oder β-ständig sein kann, wobei freie Hydroxygruppen bevorzugt sind. Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen z.B. Acetyl, Propionyl, Butyryl, Benzoyl in Frage. Halogen in den Definitionen für $R^5$, $R^6$ und Y bedeutet Fluor, Chlor, Brom und Iod. Die Reste "$C_1$-$C_{10}$-Alkanoyl" oder "$C_1$-$C_{10}$-Alkansulfonyl" für $R^7$ entsprechen den bereits genannten Alkylgruppen gleicher Länge mit dem Unterschied, daß sie an eine Carboxylgruppe gebunden sind. Bevorzugt sind $C_1$-$C_4$-Alkanoyl bzw. -Alkansulfonyl. Zur Salzbildung mit den freien Säuren ($R^5$ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Bevorzugt werden die Verbindungen der Formel I, in denen

$R^1$ die Gruppe $COOR^5$,

$R^3$ Wasserstoff oder Hydroxyl,

$R^5$ Wasserstoff oder Methyl,

$R^7$ Methansulfonyl,

p 0 bis 4

n 0 oder 1 bedeuten.

Die anmeldungsgemäßen Verbindungen der Formel I lassen sich, wie nachfolgend näher beschrieben, herstellen:

A.

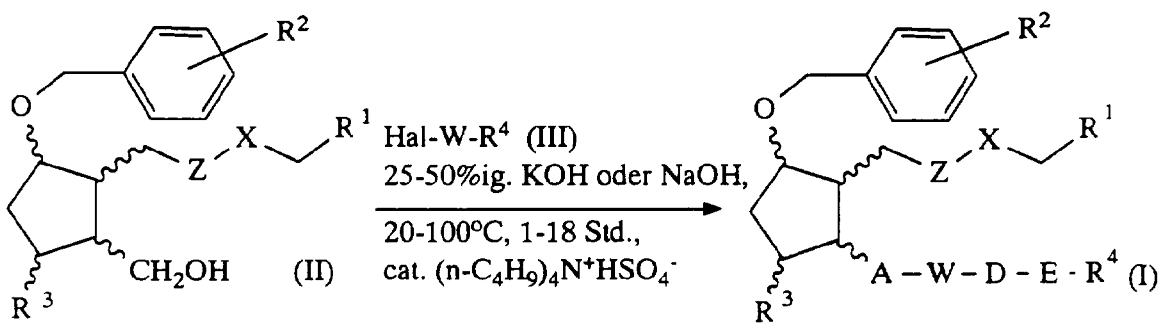


mit $R^2$, $R^3$, $R^4$, X, Z in den oben angegebenen Bedeutungen,

Hal als Brom oder Chlor,

A, D, E als Direktbindung,

$R^1$ in der Bedeutung eines $-COOR^5$-Esters.

B.

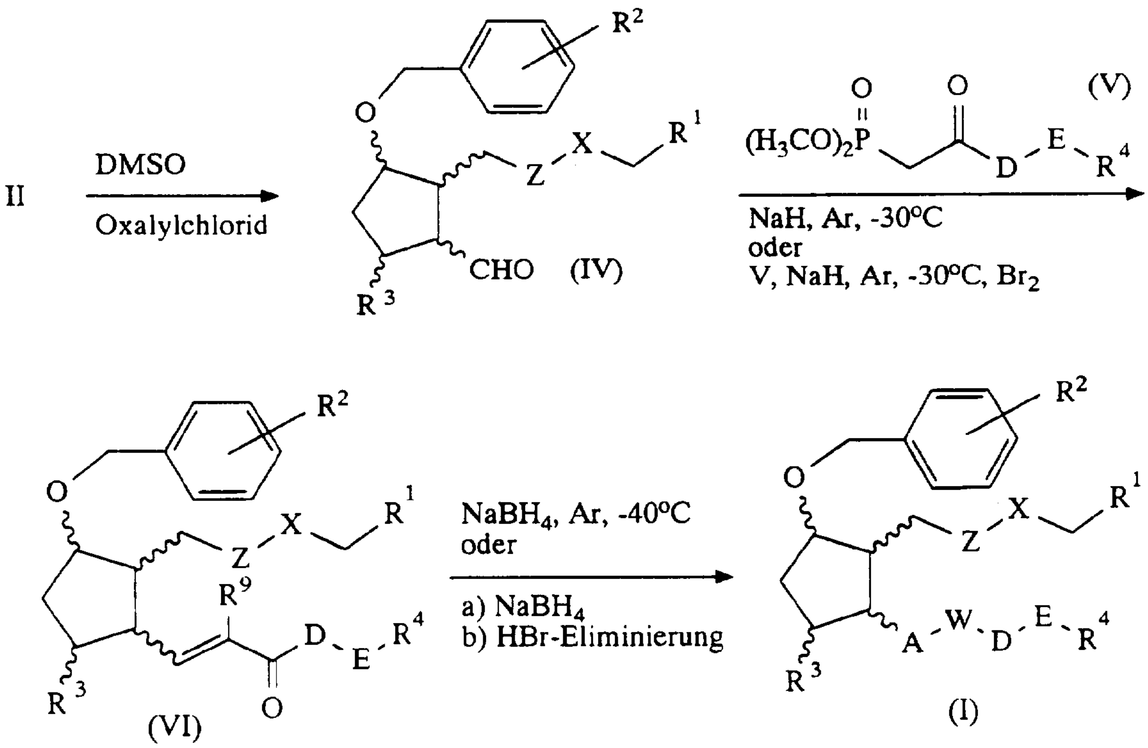

mit $R^2$, $R^3$, $R^4$, A, E, X, Z in den oben angegebenen Bedeutungen,

D als gegebenenfalls durch Alkyl substituiertes Alkylen,

$R^1$ in der Bedeutung eines $-COOR^5$-Esters,

$R^9$ als Wasserstoff oder Brom,

W -CH(OH)-, wobei für D-E-$R^4$ die Bedeutung $-(CH_2)_4CH_3$ ausgeschlossen ist, wenn -Z-X die Bedeutung (Z) $-CH{=}CH-(CH_2)_p-$ oder die Bedeutung Direktbindung-$(CH_2)_p-$ aufweist.

C.

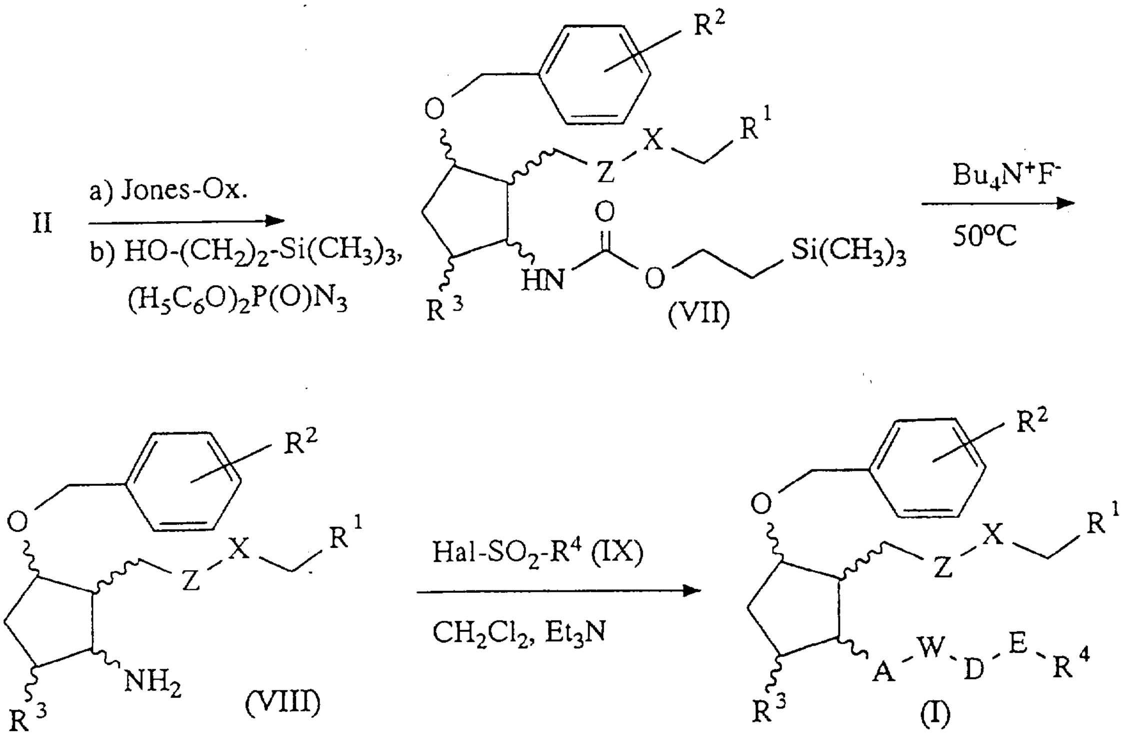

II
(VII)
(VIII)
(I)

mit $R^2$, $R^3$, $R^4$, X, Z, Hal in den oben angegebenen Bedeutungen,

A, W, D, E als Direktbindung

$R^1$ in der Bedeutung eines $-COOR^5$-Esters.

D.

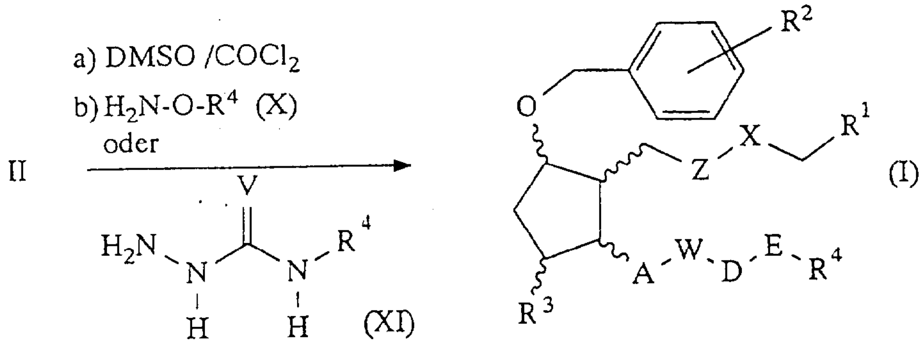

II
(XI)
(I)

mit $R^2$, $R^3$, $R^4$, X, Z in den oben angegebenen Bedeutungen,

A, W, E als Direktbindung

$R^1$ in der Bedeutung eines -$COOR^5$-Esters,

D in der Bedeutung

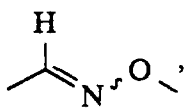
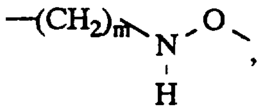
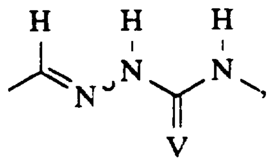

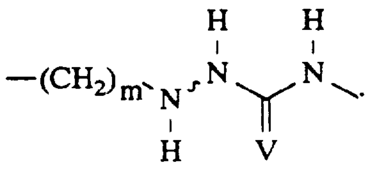

E.

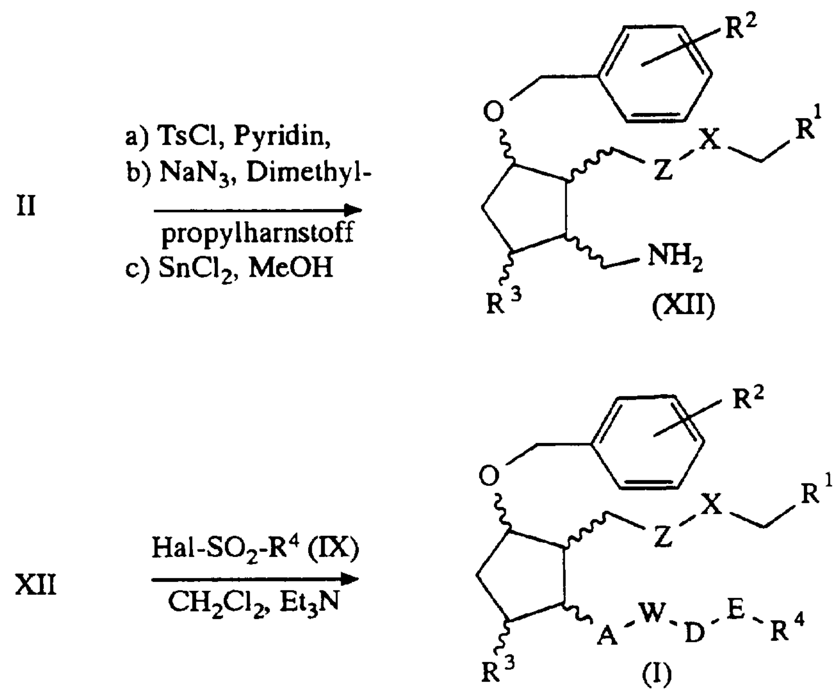


mit, $R^2$, $R^3$, X, Z in den oben angegebenen Bedeutungen,

A, W, D, E als Direktbindung

$R^1$ in der Bedeutung eines $COOR^5$-Esters.

Die Verbindungen der Formel I lassen sich nach Anspruch 3 entsprechend den oben beschriebenen Verfahrensalternativen herstellen. Die Ausgangsverbindungen der Formel II werden entsprechend den in den Beispielen 2a-2e angegebenen Vorschriften hergestellt. Die Verseifung der Ester in -$COOR^5$ erfolgt beispielsweise mit Alkali- oder Erdalkalicarbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkali-carbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Reaktionsbedingungen der nachfolgenden Verfahrensstufen sind:

1) II ⇒ I (Verfahren A) In Gegenwart von wässrigen Alkali- oder Erdalkalilösungen und unter Verwendung von Phasentransfer-Katalysatoren (wie z.B. Tetrabutylammoniumhydrogenphosphat oder -sulfat) werden Verbindungen der Formel II bei 20 bis 100°C in 1 bis 16 Std. mit dem Reaktanden III als organische Phase oder einer Lösung von III in einem inerten, nicht mit Wasser mischbaren organischen Lösungsmittel umgesetzt.

2) II ⇒ IV (Verfahren B) Die Oxidation von Verbindungen der Formel II erfolgt nach bekannten Verfahren, wie z.B. nach dem von Swern, Collins sowie unter Verwendung von Pyridiniumdichromat bzw. -chlorochromat in Lösungsmitteln wie Dichlormethan, Diethylether, Tetrahydrofuran, Benzol oder Toluol bei -80°C bis -50°C (Swern) bzw. bis +30°C (bei den übrigen Oxidationen) innerhalb von 10 Minuten bis 8 Stunden.

IV ⇒ VI (Verfahren B), VI ⇒ I (Verfahren B) Die Umsetzung der Verbindungen IV mit den Phosphonaten V sowie die sich anschließende Reduktion bzw. HBr-Eliminierung erfolgten analog den in der DE-OS 2845770 genannten Bedingungen.

3) II ⇒ VII (Verfahren C) Die Oxidation der Verbindungen der Formel II wird bevorzugt mit Jones-Reagenz oder Pyridiniumchlorochromat unter Beachtung der hierfür erforderlichen Reaktionsbedingungen durchgeführt. Anschließend wird die Umlagerung durch Erhitzen mit Phosphorsäurediphenylesterazid in einem inerten Lösungsmittel wie z.B. Toluol durchgeführt. Das Umlagerungsprodukt wird nach Zugabe von 2-(Trimethylsilyl)-ethanol als Verbindungen der Formel VII isoliert, die wie in den zugehörigen Beispielen beschrieben zu Verbindungen der Formel VIII umgesetzt werden.

4) VIII ⇒ I (Verfahren C) Die Umsetzung der Verbindungen der Formel VIII mit den Verbindungen der Formel IX erfolgt wie in den dafür genannten Beispielen.

5) II ⇒ I (Verfahren D) Die Umsetzung erfolgt in Analogie zu dem in WO 90/02740 (Verfahren C, S. 16) beschriebenen Verfahren.

6) II ⇒ I (Verfahren E) Die Umsetzung der Verbindungen der Formel II zu Verbindungen der Formel XII und weiter zu Verbindungen der Formel I erfolgt wie in den dafür genannten Beispielen.

Die Freisetzung der funktionell abgewandelten Hydroxygruppen $R^2$, $R^3$, $R^4$ und W erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.

Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid etc. geeignet. Die Abspaltung wird vorzugsweise bei Temperaturen zwischen 20°C und 80°C durchgeführt.

Die Verseifung der Acylgruppen und Prostaglandinester wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Einführung der Estergruppe $CO_2R^5$ für $R^1$ bzw. $CO_2R^6$ für Y, bei welcher $R^5$ bzw. $R^6$ eine Alkylgruppe mit 1-10-C-Atomen darstellt, erfolgt nach den dem Fachmann bekannten Methoden. Die Carboxyverbindungen ($R^5$ = H bzw. $R^6$ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der Carboxyverbindung, gelöst in dem gleichen oder in einem anderen ebenfalls inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe $CO_2R^5$ für $R^1$ bzw. $CO_2R^6$ für Y, bei welcher $R^5$ bzw. $R^6$ eine substituierte oder

unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, DMAP, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Prostaglandinderivate der Formel I mit $R^5$ bzw. $R^6$ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Säure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid etc., umsetzt.

Cyclodextrinclathrate werden analog der Vorschrift in WO 87/05294 erhalten.

Liposomen werden nach dem in "Pharmazie in unserer Zeit 11, 98 (1982)" beschriebenen Herstellungsverfahren hergestellt.

Alle stereoisomeren Formen gehören ebenfalls zum Gegenstand der Erfindung. Die vier mit dieser "geschlängelten Linie" gekennzeichneten Verbindungen am Fünfring der Formel I stellen keine Gemische dar, sondern sollen jeweils eine konkrete optische Form bedeuten, die R oder S konfiguriert sein kann.

Biologische Wirkung und Anwendungsbereich der neuen $TXA_2$-Antagonisten: Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind hervorragend geeignet zur Hemmung der Thrombozytenaktivierung. Folglich stellen die neuen $TXA_2$-Antagonisten der Formel I wertvolle pharmazeutische Wirkstoffe dar. Darüber hinaus zeichnen sich die Verbindungen durch höhere Selektivität, einer wesentlich längeren Wirksamkeit und einer größeren Stabilität verglichen mit ähnlichen $TXA_2$-Antagonisten aus. Die neuen $TXA_2$-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Senkung des systemischen Blutdrucks ohne zugleich Schlagvolumen und koronare Durchblutung zu senken, Förderung der Nierendurchblutung und der Durchblutung anderer peripherer Organe, Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen $TXA_2$-Antagonisten prinzipiell geeignet zur Behandlung des Schlaganfalles, der Prophylaxe und Therapie koronarer Herzerkrankungen, zum Beispiel der Koronarthrombose, zur Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns wie z.B. der Migräne, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks, des Asthmas und der allergischen Rhinitis. Sie können ferner eingesetzt werden, zur Inhibierung von Geburtswehen und zur Behandlung von Schwangerschaftstoxikosen.

Die neuen $TXA_2$-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.

Die neuen $TXA_2$-Antagonisten besitzen antiinflammatorische Eigenschaften und sind prinzipiell geeignet zur Therapie topischer Erkrankungen wie zum Beispiel der dermalen Ischämie oder von Dekubitus-ulcera.

Die neuen $TXA_2$-Antagonisten besitzen eine antimetastatische Wirkung und antiproliferative Eigenschaften. Sie sind prinzipiell geeignet zur Behandlung von Neoplasien. Die neuen $TXA_2$-Antagonisten können in Kombination, zum Beispiel mit Carbacyclinen, Prostacyclin und seinen Analoga, 7-Oxo-prostacyclinen, Prostaglandinen und deren Derivate und 6-Oxo-$PGE_1$- und 6-Oxo-9-Fluor-Prostaglandin-Derivaten, mit $TXA_2$-Synthetase-Inhibitoren, mit Phosphodiesterase-Inhibitoren, mit Antagonisten und Rezeptorantagonisten verschiedener Thrombozytenstimulatoren (z.B. ADP, Thrombin, Collagen, PAF, Adrenalin, Serotonin, Fibrinogen), mit Calciumantagonisten, mit Fibrinolytika und Thrombolytika, z.B. t-PA, mit Heparin und anderen Antikoagulanzien, mit Cyclooxygenaschemmem, z.B. Acetylsalicylsäure, mit Hemmstoffen der Lipoxygenasen sowie Antagonisten von Lipoxygenaseprodukten, mit Vasodilatatoren wie z.B. Nitroverbindungen, mit Antihypertensiva wie z.B. β-Blockem oder mit Diuretika Verwendung finden. Die erfindungsgemäßen Verbindungen können nicht nur $TXA_2$-antagonistisch, sondern auch bifunktionell, d.h. $TXA_2$-antagonistisch und gleichzeitig $PGI_2$-agonistisch wirken. Die Dosis der Verbindungen ist 0,1-500 mg/Tag, auch in mehreren Teildosierungen, wenn sie am menschlichen Patienten verabreicht werden. Die Einheitsdosis für den pharma-zeutisch akzeptablen Träger beträgt 0,1-100 mg. Für die parenterale Verabreichung werden sterile, injizierbare wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Der Einheitsdosisbereich für die Ampulle ist 0,1-100 mg, für die Tablette 0,1-100 mg.

Beispiel 1:

7-[(1 R,2S,5S)-2-(4-Phenylbenzyloxy)-5-(4-fluorphenylsulfonylamino)-cyclopentyl]-5(Z)-heptensäure:

Die Lösung von 55 mg (97 μmol) der nach Beispiel 2 dargestellten Verbindung in 1 ml Methanol versetzt man mit 0,5 ml einer ca. 5% igen Kaliumydroxidlösung und rührt 20 Stunden bei 23°C. Durch Zugabe von gesättigter Zitronensäure wird angesäuert, mit Wasser verdünnt und mehrfach mit Ethylacetat extrahiert. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an ca. 25g Kieselgel mit einem Gradientensystem aus n-Hexan und Ethylacetat. Isoliert werden 32,8 mg (59 μmol, 61%) der Titelverbindung als farbloses Öl.

IR (Film): 3600-2400, 3280, 3030, 3010, 2940, 2870, 1710, 1590, 1495, 1450, 1405, 1330, 1290, 1165, 1155, 1090, 1025, 915, 840, 765, 700, 670, 570 und 550 $cm^{-1}$.

Beispiel 2:

7-[(1R,2S,5S)-2-(4-Phenylbenzyloxy)-5-(4-fluorphenylsulfonylamino)-cyclopentyl]-5(Z)- heptensäuremethylester:

Die Lösung von 100 mg (245 μmol) des nach Beispiel 2a dargestellten Amins in 1,2 ml wasserfreiem Dichlormethan versetzt man bei 0°C mit 49,5 mg Triethylamin, 52,3 mg 4-Fluorsulfonsäurechlorid und rührt 16 Stunden bei 23°C unter einer Atmosphäre aus trockenem Argon. Man verdünnt mit Ethylacetat, wäscht mit 1n Salzsäure, 5%iger Natriumhydrogencarbonatlösung, Wasser, gesättigter Natriumchloridlösung und trocknet über Magnesiumsulfat. Den nach Filtration und Lösungsmittelabzug erhaltenen Rückstand reinigt man durch Chromatographie an ca. 20g Kieselgel. Isoliert werden 61 mg (108 μmol, 44%) der Titelverbindung als farbloses Öl.

IR (Film): 3280, 3060, 3030, 3010, 2950, 2870, 1735, 1715, 1595, 1495, 1440, 1335, 1295, 1230, 1165, 1155, 1090, 915, 760, 700 und 670 $cm^{-1}$.

Beispiel 2a:

7-[(1R,2S,5S)-2-(4-Phenylbenzyloxy)-5-amino-cyclopentyl]-5(Z)-heptensäuremethylester:

Die Lösung von 1,23 g (2,23 mmol) der nach Beispiel 2b dargestellten Verbindung versetzt man mit 11 ml einer 1M Lösung von Tetrabutylammoniumfluorid in Tetrahydrofuran und erhitzt 2,5 Stunden auf 50°C. Nach dem Erkalten versetzt man mit Diethylether, wäscht mit Wasser, gesättigter Natriumchloridlösung und trocknet über Magnesiumsulfat. Den nach Filtration und Lösungsmittelabzug erhaltenen Rückstand setzt man ohne Reinigung weiter um.

Beispiel 2b:

7-[(1R,2S,5S)-2-(4-Phenylbenzyloxy)-5-(trimethylsilylethoxycarhonylamino)-cyclopentyl]-5(Z)-heptensäuremethylester:

Die Lösung von 1,62 g (3,71 mmol) der nach Beispiel 2c dargestellten Säure in 3,7 ml wasserfreiem Toluol versetzt man unter einer Atmosphäre aus trockenem Argon mit 372 mg Triethylamin, 1,02 g Phosphorsäurediphenylesterazid und erhitzt 2,5 Stunden auf 90°C. Man versetzt mit 930 mg 2-(Trimethylsilyl)-ethanol und rührt weitere 18 Stunden bei 90°C. Nach dem Erkalten verdünnt man mit Diethylether, wäscht mit 10%iger Natriumhydroxidlösung, gesättigter Natriumchloridlösung und trocknet über Magnesiumsulfat. Den nach Filtration und Lösungsmittelabzug erhaltenen Rückstand reinigt man durch Chromatographie an 80 g Kieselgel mit einem Gradientensystem aus n-Hexan und Ethylacetat. Isoliert werden 1,23 g (2,23 mmol, 60%) der Titelverbindung als farbloses Öl. IR (Film): 3340, 3060, 3030, 3010, 2950, 2900, 1740-1690, 1600, 1530, 1490, 1250, 1170, 1060, 960, 860, 835, 760 und 700 $cm^{-1}$.

Beispiel 2c:

7-[(1R,2S,5S)-2-(4-Phenylbenzyloxy)-5-hydroxycarbonyl-cyclopentyl]-5(Z)-heptensäuremethylester:

Die Lösung von 2,0 g (4,73 mmol) des nach Beispiel 2d dargestellten Alkohols in 58 ml Aceton kühlt man auf -40°C, versetzt mit 2,84 ml einer standardisierten Chromschwefelsäurelösung (Jones-Reagenz), rührt 1,5 Stunden bei -40°C bis -15°C und zersetzt überschüssiges Oxidationsmittel durch Zugabe von 7 ml Isopropanol. Man verdünnt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 150g groben Kieselgel mit einem Gradientensystem aus n-Hexan und Ethylacetat. Isoliert werden 1,62 g (3,71 mmol, 79%) der Titelverbindung als farbloses Öl.

IR (Film): 3600-2400, 3030, 3010, 2950, 2860, 1735, 1700, 1605, 1485, 1435, 1340, 1310, 1240, 1165, 1075, 760 und 695 $cm^{-1}$.

Beispiel 2d:

7-[(1R,2S,5S)-2-(4-Phenylbenzyloxy)-5-hydroxymethyl-cyclopentyl]-5(Z)-heptensäuremethylester:

Die Lösung von 6,1 g (9,23 mmol) der nach Beispiel 2e dargestellten Verbindung in 120 ml wasserfreiem Tetrahydrofuran versetzt man mit 17 g Tetrabutylammoniumfluorid und rührt 17 Stunden bei 23°C unter einer Atmosphäre aus trockenem Argon. Man versetzt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 300g Kieselgel mit einem Gradientensystem aus n-Hexan und Ethylacetat. Isoliert werden 2,07 g (4,90 mmol, 53%) der Titelverbindung als farbloses Öl. IR (Film): 3600-3200, 3060, 3030, 3000, 2940, 2860, 1735, 1600, 1485, 1435, 1340, 1310, 1245, 1210, 1165, 1075, 825, 760 und 695 $cm^{-1}$.

Beispiel 2e:

7-[(1R,2S,5S)-2-(4-Phenylbenzyloxy)-5-(tert.-butyldiphenylsilyloxymethyl)-cyclopentyl]- 5(Z)-heptensäuremethylester:

4,0 g (8,09 mmol) 7-[(1R,2S,5S)-5-(tert.-butyldiphenylsilyloxymethyl)-2-hydroxy- cyclo-pentyl]-5(Z)-heptensäuremethylester ( siehe DE 40 24 347.8, Bsp. 1i ) löst man in 12ml Toluol, versetzt mit 5,0 g 4-Phenylhenzylbromid, 8 ml einer 50%igen Kaliumhydroxidlösung, 368 mg Tetrabutylammoniumhydrogensulfat und rührt 18 Stunden bei 23°C. Man säuert durch Zugabe von Zitronensäure an, verdünnt mit Ethylacetat, wäscht mit gesättigter Natriumchloridlösung und trocknet über Magnesiumsulfat. Den nach Filtration und Lösungsmittelabzug erhaltenen Rückstand reinigt man durch Chromatographie an ca. 160g Kieselgel mit einem Gradientensystem aus n-Hexan und Ethylacetat. Isoliert werden 3,05 g (4,61 mmol, 57%) der Titelverbindung als farbloses Öl. IR (Film): 3070, 3050, 3030, 3000, 2950, 2930, 2850, 1735, 1600, 1485, 1430, 1360, 1110, 1005, 825, 760, 740 und 700 $cm^{-1}$.

Beispiel 3:

7-[(1R,2S,5S)-2-(4-Phenylbenzyloxy)-5-phenylsulfonylamino-cyclopentyl]-5(Z)-heptensäure:

55,5 mg (101 µmol) der nach Beispiel 4 dargestellten Verbindung verseift man in Analogie zu Beispiel 1 und isoliert nach Aufarbeitung und Reinigung 38,7 mg (73 µmol, 72%) der Titelverbindung als farbloses Öl.

IR (Film): 3600-2400, 3270, 3060, 3030, 3010, 2940, 2870, 1705, 1605, 1490, 1450, 1325, 1160, 1095, 1075, 965, 830, 760, 725, 690, 590 und 565 $cm^{-1}$.

Beispiel 4:

7-[(1R,2S,5S)-2-(4-Phenylbenzyloxy)-5-phenylsulfonylamino-cyclopentyl]-5(Z)-heptensäuremethylester:

100 mg (245 µmol) des nach Beispiel 2a dargestellten Amins setzt man in Analogie zu Beispiel 2 unter Verwendung von Benzolsulfonsäurechlorid um und isoliert nach Aufarbeitung und Reinigung 85 mg (156 µmol, 63%) der Titelverbindung als farbloses Öl. IR (Film): 3280, 3060, 3030, 3010, 2950, 2870, 1735, 1600, 1490, 1445, 1330, 1160, 1095, 1070, 910, 760, 720 und 690 $cm^{-1}$.

Beispiel 5:

7-[(1R,2S,5S)-2-(4-Phenylbenzyloxy)-5-(4-methylphenylsulfonylamino)-cyclopentyl]-5(Z)- heptensäure:

39,2 mg (69 µmol) der nach Beispiel 6 dargestellten Verbindung verseift man in Analogie zu Beispiel 1 und isoliert nach Aufarbeitung und Reinigung 15,4 mg (28 µmol, 40%) der Titelverbindung als farbloses Öl.

IR (Film): 3600-2400, 2960, 2930, 2870, 1690, 1450, 1425, 1405, 1385, 1155, 1095, 1070, 900 und 760 $cm^{-1}$.

Beispiel 6:

7-[(1R,2S,5S)-2-(4-Phenylbenzyloxy)-5-(4-methylphenylsulfonylamino)-cyclopentyl]-5(Z)-heptensäuremethylester:

100 mg (245 µmol) des nach Beispiel 2a dargestellten Amins setzt man in Analogie zu Beispiel 2 unter Verwendung von 4-Methylphenylsulfonsäurechlorid um und isoliert nach Aufarbeitung und Reinigung 49 mg (87 µmol, 36%) der Titelverbindung als farbloses Öl. IR (Film): 3280, 2950, 2870, 1735, 1600, 1450, 1425, 1405, 1385, 1155, 1095, 1070, 900 und 760 $cm^{-1}$.

Beispiel 7:

7-[(1R,2S,5S)-2-(4-Phenylbenzyloxy)-5-(2,5-dichlorphenylsulfonylamino)-cyclopentyl]-5(Z)-heptensäure:

43,8 mg (71 µmol) der nach Beispiel 8 dargestellten Verbindung verseift man in Analogie zu Beispiel 1 und isoliert nach Aufarbeitung und Reinigung 31 mg (51 µmol, 73%) der Titelverbindung als farbloses Öl.

IR (Film): 3600-2400, 3300, 3090, 3060, 3030, 3010, 2940, 2870, 1710, 1600, 1490, 1450, 1375, 1340, 1245, 1165, 1100, 1075, 1045, 920, 895, 825, 760, 700, 680, 600, 585 und 515 $cm^{-1}$.

Beispiel 8:

7-[(1R,2S,5S)-2-(4-Phenylbenzyloxy)-5-(2,5-dichlorphenylsulfonylamino)-cyclopentyl]5(Z)-heptensäuremethylester:

100 mg (245 µmol) des nach Beispiel 2a dargestellten Amins setzt man in Analogie zu Beispiel 2 unter Verwendung von 2,5-Dichlorphenylsulfonsäurechlorid um und isoliert nach Aufarbeitung und Reinigung 83,5 mg (135 µmol, 55%) der Titelverbindung als farbloses Öl.

IR (Film): 3300, 3090, 3070, 3030, 3010, 2950, 2870, 1735, 1715, 1600, 1490, 1450, 1375. 1340, 1245, 1220, 1165,

1100, 1075, 1040, 825, 765, 700 und 680 $cm^{-1}$.

Beispiel 9:

7-[(1R,2S,5S)-2-(4-Phenylbenzyloxy)-5-(chinon-8-ylsulfonylamino)-cyclopentyl]-5(Z)-heptensäure:

35,3 mg (59 µmol) der nach Beispiel 10 dargestellten Verbindung verseift man in Analogie zu Beispiel 1 und isoliert nach Aufarbeitung und Reinigung 13,9 mg (24 µmol, 40%) der Titelverbindung als farbloses Öl.

IR (Film): 3600-2400, 3260, 3060, 3030, 3010, 2940, 2870, 1735, 1705, 1615, 1595, 1565, 1490, 1435, 1330, 1240, 1215, 1165, 1145, 1070, 900, 835, 790, 760, 700, 675 und 605 $cm^{-1}$.

Beispicl 10:

7-[(1R,2S,5S)-2-(4-Phenylbenzyloxy)-5-(chinon-8-ylsulfony lamino)-cyclopentyl]-5(Z)-heptensäuremethylester:

100 mg (245 µmol) des nach Beispiel 2a dargestellten Amins setzt man in Analogie zu Beispiel 2 unter Verwendung von Chinon-8-ylsulfonsäurechlorid um und isoliert nach Aufarbeitung und Reinigung 35,3 mg (59 µmol, 24%) der Titelverbindung als farbloses Öl. IR (Film): 3270, 3060, 3030, 3010, 2940, 2870, 1735, 1610, 1595, 1565, 1490, 1435, 1330, 1240, 1215, 1165, 1145, 1070, 900, 835, 790, 760, und 700 $cm^{-1}$.

Beispiel 11:

7-[(1R,2S,5S)-2-(4-Phenylbenzyloxy)-5-(naphth-2-ysulfonylamino)-cyclopentyl]-5(Z)-heptensäure:

29,5 mg (49 µmol) der nach Beispiel 12 dargestellten Verbindung verseift man in Analogie zu Beispiel 1 und isoliert nach Aufarbeitung und Reinigung 18,2 mg (31 µmol, 64%) der Titelverbindung als farbloses Öl.

IR (Film): 3600-2400, 3270, 3060, 3030, 3010, 2940, 2870, 1705, 1590, 1490, 1450, 1435, 1410, 1325, 1155, 1130, 1075, 910, 820, 760, 695, 660, 615, 565, 550 und 475 $cm^{-1}$.

Beispiel 12:

7-[(1R,2S,5S)-2-(4-Phenylbenzyloxy)-5-(naphth-2-ylsulfonylamino)-cyclopentyl]-5(Z)-heptensäuremethylester:

100 mg (245 µmol) des nach Beispiel 2a dargestellten Amins setzt man in Analogie zu Beispiel 2 unter Verwendung von Naphth-2-ylsulfonsäurechlorid um und isoliert nach Aufarbeitung und Reinigung 41,8 mg (70 µmol, 29%) der Titelverbindung als farbloses Öl. IR (Film): 3290, 3060, 3030, 3010, 2940, 2870, 1735, 1590, 1490, 1450, 1435, 1410, 1325, 1155, 1130, 1075, 910, 820, 760, und 700 $cm^{-1}$.

Beispiel 13:

7-[(1R,2S,5S)-2-(4-Phenylbenzyloxy)-5-(4-fluorphenylsulfonylaminomethyl)-cyclopentyl]-5(Z)-heptensäure:

50 mg (86 µmol) der nach Beispiel 14 dargestellten Verbindung verseift man in Analogie zu Beispiel 1 und isoliert nach Aufarbeitung und Reinigung 37 mg (65 µmol, 76%) der Titelverbindung als farbloses Öl.

IR (Film): 3680-2400, 3280, 2930, 1710, 1595, 1495, 1330, 1240, 1155, 1090, 840, 760, 700 und 550 $cm^{-1}$.

Beispiel 14:

7-[(1R,2S,5S)-2-(4-Phenylbenzyloxy)-5-(4-fluorphenylsulfonylaminomethyl)-cyclopentyl]-5(Z)-heptensäuremethylester:

139 mg (329 µmol) des nach Beispiel 14a dargestellten Amins setzt man in Analogie zu Beispiel 2 unter Verwendung von 4-Fluorphenylsulfonsäurechlorid um und isoliert nach Aufarbeitung und Reinigung 50 mg (86 µmol, 26%) der Titelverbindung als farbloses Öl. IR (Film): 3280, 3060, 3030, 3010, 2950, 2860, 1735, 1595, 1490, 1435, 1410, 1335, 1290, 1235, 1165, 1155, 1090, 840, 760, 700 und 670 $cm^{-1}$.

Beispiel 14a:

7-[(1R,2S,5S)-2-(4-Phenylbenzyloxy)-5-aminomethyl-cyclopentyl]-5(Z)-heptensäuremethylester:

Zu der Lösung von 402 mg Zinnchlorid-Dihydrat in 6,5 ml wasserfreiem Methanol tropft man die Lösung von 532 mg (1,19 mmol) der nach Beispiel 14b dargestellten Verbindung in 6,5 ml Methanol und rührt 29 Stunden bei 23°C. Man engt ein, versetzt den Rückstand mit Natriumcarbonatlösung, extrahiert mehrfach mit Ethylacetat, wäscht die vereinigten organischen Extrakte mit gesättigter Natriumchloridlösung und trocknet über Magnesiumsulfat. Den nach Filtration und Lösungsmittelabzug erhaltenen Rückstand (419 mg) setzt man ohne Reinigung weiter um.

Beispiel 14b:

7-[(1R,2S,5S)-2-(4-Phenylbenzyloxy)-5-azidomethyl-cyclopentyl]-5(Z)-heptensäuremethylester:

760 mg (1,32 mmol) der nach Beispiel 14c dargestellten Verbindung löst man in 11,5 ml Dimethylpropylharnstoff, versetzt mit 1,55 g Natriumazid und rührt 5,5 Stunden bei 40°C unter einer Atmosphäre aus trockenem Argon. Man verdünnt mit Diethylether, wäscht mehrfach mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 532 mg (1,19 mmol, 90%) der Titelverbindung als farbloses Öl.

IR (Film): 3060, 3030, 3010, 2950, 2860, 2090, 1735, 1600, 1490, 1450, 1435, 1345, 1245, 1165, 1120, 1075, 825, 760 und 700 $cm^{-1}$.

Beispiel 14c:

7-[(1R,2S,5S)-2-(4-Phenylbenzyloxy)-5-(4-methylphenylsulfonyloxymethyl)-cyclopentyl]-5(Z)-heptensäuremethylester:

Die Lösung von 700 mg (1,66 mmol) der nach Beispiel 2d dargestellten Verbindung in 4,3 ml wasserfreiem Pyridin versetzt man mit 677 mg p-Toluolsulfonsäurechlorid und rührt 4 Stunden bei 23 °C unter einer Atmosphäre aus trockenem Argon. Man versetzt mit Wasser, verdünnt mit Ethylacetat, wäscht mit 10%iger Schwefelsäure, anschließend mit gesättigter Natriumhydrogencarbonatlösung und trocknet über Natriumsulfat. Den nach Filtration und Lösungsmittelabzug erhaltenen Rückstand reinigt man durch Chromatographie an Kieselgel unter Verwendung eines Gradientensystems aus n-Hexan und Ethylacetat. Isoliert werden 760 mg (1,32 mmol, 80%) der Titelverbindung als farbloses Öl.

IR (Film): 3060, 3030, 3010, 2950, 2870, 1735, 1600, 1490, 1450, 1335, 1360, 1245, 1190, 1175, 1095, 1080, 945, 830, 815, 760, 700 und 665 $cm^{-1}$.

Beispiel 15:

7-[(1R,2S,5S)-2-(4-Phenylbenzyloxy)-5-(4-methylphenylsulfonylaminomethyl)-cyclopentyl]-5(Z)-heptensäure:

66 mg (115 µmol) der nach Beispiel 16 dargestellten Verbindung verseift man in Analogie zu Beispiel 1 und isoliert nach Aufarbeitung und Reinigung 54 mg (95 µmol, 83%) der Titelverbindung als farbloses Öl.

IR (Film): 3680-2400, 3280, 2930, 1710, 1600, 1490, 1325, 1160, 815, 760, 700, 660 und 550 $cm^{-1}$.

Beispiel 16:

7-[(1R,2S,5S)-2-(4-Phenylbenzyloxy)-5-(4-methylphenylsulfonylaminomethyl)-cyclopentyl]-5(Z)-heptensäuremethylester:

139 mg (329 µmol) des nach Beispiel 14a dargestellten Amins setzt man in Analogie zu Beispiel 2 unter Verwendung von 4-Methylphenylsulfonsäurechlorid um und isoliert nach Aufarbeitung und Reinigung 72 mg (125 µmol, 38%) der Titelverbindung als farbloses Öl. IR (Film): 3280, 3060, 3030, 3010, 2950, 2870, 1735, 1600, 1490, 1435, 1330, 1160, 1090, 815, 760, 700 und 665 $cm^{-1}$.

Beispiel 17:

7-[(1R,2S,5S)-2-(4-Phenylbenzyloxy)-5-(2,5-dichlorphenylsulfonylaminomethyl)-cyclopentyl]-5(Z)-heptensäure:

35 mg (56 µmol) der nach Beispiel 18 dargestellten Verbindung verseift man in Analogie zu Beispiel und isoliert nach Aufarbeitung und Reinigung 25 mg (41 µmol, 73%) der Titelverbindung als farbloses Öl.

IR (Film): 3680-2400, 3300, 3080, 3000, 2920, 1710, 1490, 1450, 1340, 1165, 825, 760, 700 und 580 $cm^{-1}$.

Beispiel 18:

7-[(1R,2S,5S)-2-(4-Phenylbenzyloxy)-5-(2,5-dichlorphenylsulfonylaminomethyl)-cyclopentyl]-5(Z)-heptensäuremethylester:

139 mg (329 µmol) des nach Beispiel 14a dargestellten Amins setzt man in Analogie zu Beispiel 2 unter Verwendung von 2,5-Dichlorphenylsulfonsäurechlorid um und isoliert nach Aufarbeitung und Reinigung 35 mg (56 µmol, 17%) der Titelverbindung als farbloses Öl.

IR (Film): 3300, 3090, 3060, 3030, 3010, 2940, 2870, 1735, 1600, 1490, 1450, 1415, 1375, 1340, 1245, 1165, 1100, 1040, 825, 760, 700 und 680 $cm^{-1}$.

Beispiel 19:

7-[(1R,2S,5S)-2-(4-Phenylbenzyloxy)-5-(2,4-difluorphenylsulfonylamino)-cydopentyl]-5(Z)-heptensäure:

88 mg (151 µmol) der nach Beispiel 20 dargestellten Verbindung verseift man in Analogie zu Beispiel 1 und isoliert nach Aufarbeitung und Reinigung 36,1 mg (63 µmol, 32%) der Titelverbindung als farbloses Öl.

IR (Flsg.cap.): 3600-3200, 2940, 1710, 1605, 1490, 1430, 1340, 1275, 1160, 970, 760 und 700 $cm^{-1}$.

Beispiel 20:

7-[(1R,2S,5S)-2-(4-Phenylbenzyloxy)-5-(2,4-difluorphenylsulfonylamino)-cyclopentyl]-5(Z)-heptensäuremethylester:

257 mg (653 µmol) des nach Beispiel 2a dargestellten Amins setzt man in Analogie zu Beispiel 2 unter Verwendung von 2,4-Difluorphenylsulfonsäurechlorid um und isoliert nach Aufarbeitung und Reinigung 114 mg (200 µmol, 30%) der Titelverbindung als farbloses Öl.

IR (Film): 3400-3140, 2950, 1730, 1605, 1485, 1340, 11165, 1075, 970 und 850 $cm^{-1}$.

Beispiel 21:

7-[(1R,2S,5S)-2-(4-Phenylbenzyloxy)-5-(4-bromphenylsulfonylamino)-cyclopentyl] -5(Z)-heptensäure:

174 mg (278 µmol) der nach Beispiel 22 dargestellten Verbindung verseift man in Analogie zu Beispiel 1 und isoliert nach Aufarbeitung und Reinigung 99 mg (162 µmol, 58%) der Titelverbindung als farbloses Öl.

IR (Film): 3700-3140, 2940, 1710, 1575, 1490, 1330, 1160, 1070, 1010, 825, 760, 740 und 700 $cm^{-1}$.

Beispiel 22:

7-[(1R,2S,5S)-2-(4-Phenylbenzyloxy)-5-(4-bromphenylsulfonylamino)-cyclopentyl]-5(Z)-heptensäuremethylester:

257 mg (653 µmol) des nach Beispiel 2a dargestellten Amins setzt man in Analogie zu Beispiel 2 unter Verwendung von 4-Bromphenylsulfonsäurechlorid um und isoliert nach Aufarbeitung und Reinigung 174 mg (278 µmol, 43%) der Titelverbindung als farbloses Öl.

IR (Film): 3280, 2960, 1740, 1578, 1490, 1440, 1340, 1165, 1070, 1013, 830, 765, 745 und 705 $cm^{-1}$.

Beispiel 23:

7-[(1R,2S,5S)-2-(4-Phenylbenzyloxy)-5-(3,4-dibromphenylsulfonylamino)-cyclopentyl]-5(Z)-heptensäure:

114 mg (162 µmol) der nach Beispiel 24 dargestellten Verbindung verseift man in Analogie zu Beispiel 1 und isoliert nach Aufarbeitung und Reinigung 61 mg (91 µmol, 54%) der Titelverbindung als farbloses Öl.

IR (KBr): 3700 - 3100, 2930, 2860, 1705, 1630, 1445, 1330, 1110, 760, 700, 695 und 660 $cm^{-1}$.

Beispiel 24:

7-[(1R,2S,5S)-2-(4-Phenylbenzyloxy)-5-(3,4-dibromphenylsulfonylamino)-cyclopentyl]-5(Z)-heptensäuremethylester:

257 mg (653 µmol) des nach Beispiel 2a dargestellten Amins setzt man in Analogie zu Beispiel 2 unter Verwendung von 3,4-Dibromphenylsulfonsäurechlorid um und isoliert nach Aufarbeitung und Reinigung 114 mg (162 µmol, 26%) der Titelverbindung als farbloses Öl.

IR (Film): 3280, 2950, 1730, 1487, 1445, 1340, 1165, 1075, 785, 760 und 700 $cm^{-1}$.

Beispiel 25:

7-[(1R,2S,4R,5R)-2-(4-Phenylbenzyloxy)-4-hydroxy-5-(3,4-dibromphenylsulfonylamino)-cyclopentyl]-5(Z)-heptensäure:

95 mg (115 µmol) der nach Beispiel 26 dargestellten Verbindung verseift man in Analogie zu Beispiel 1 und isoliert nach Aufarbeitung und Reinigung 66 mg (95 µmol, 81%) der Titelverbindung als farbloses Öl.

IR (KBr): 3600-3360, 3360-3100, 2930, 1705, 1570, 1485, 1450, 1330, 1100, 915, 820, 760 und 700 $cm^{-1}$.

Beispiel 26:

7-[(1R,2S,4R,5R)-2-(4-Phenylbenzyloxy)-4-benzoyloxy-5-(3,4-dibromphenylsulfonylamino)-cydopentyl]-5(Z)-heptensäuremethylester:

212 mg (412 µmol) des nach Beispiel 26a dargestellten Amins setzt man in Analogie zu Beispiel 2 unter Verwendung von 3,4-Dibromphenylsulfonsäurechlorid um und isoliert nach Aufarbeitung und Reinigung 95 mg (115 µmol, 32%) der Titelverbindung als farbloses Öl.

IR (Film): 3400-3120, 2940, 1715, 1645, 1600, 1485, 1450, 1345, 1270, 1165, 1020, 930, 760, 715 und 700 $cm^{-1}$.

Beispiel 26a:

7-[(1R,2S,4R,5R)-2-(4-Phenylbenzyloxy)-4-benzoyloxy-5-amino-cyclopentyl]-5(Z)-heptensäuremethylester:

Die Lösung von 1,34 g (1,99 mmol) der nach Beispiel 26b dargestellten Verbindung in THF versetzt man mit 9 ml einer 1M Lösung von Tetrabutylammoniumfluorid in Tetrahydrofuran und erhitzt 2,75 Stunden auf 50°C. Nach dem Abkühlen versetzt man mit Diethylether, wäscht mit Wasser, gesättigter Natriumchloridlösung und trocknet über Magnesiumsulfat. Den nach Filtration und Lösungsmittelabzug erhaltenen Rückstand setzt man ohne Reinigung weiter um.

Beispiel 26b:

7-[(1R,2S,4R,5R)-2-(4-Phenylbenzyloxy)-4-benzoyloxy-5-(trimethylsilylethoxycarbonyl]-amino)-cyclopentyl]-5(Z) -heptensäuremethylester:

Die Lösung von 1,62 g (3,04 mmol) der nach Beispiel 26c dargestellten Säure in 3 ml wasserfreiem Toluol versetzt man unter einer Atmosphäre aus trockenem Argon mit 307 mg Triethylamin, 836 mg Phosphorsäurediphenylesterazid und erhitzt 2 Stunden auf 90°C. Man versetzt mit 754 mg 2-(Trimethylsilyl)-ethanol und rührt weitere 23 Stunden bei 90°C. Nach dem Erkalten verdünnt man mit Diethylether, wäscht mit 10%iger Natriumhydroxidlösung, gesättigter Natriumchloridlösung und trocknet über Magnesiumsulfat. Den nach Filtration und Lösungsmittelabzug erhaltenen Rückstand reinigt man durch Chromatographie an Kieselgel mit einem Gradientensystem aus n-Hexan und Ethylacetat. Isoliert werden 1,34 g (1,99 mmol, 66%) der Titelverbindung als farbloses Öl.

IR (Film): 3460-3200, 2950, 1720, 1535, 1455, 1275, 1250, 1120, 1070, 860, 840, 765 und 715 $cm^{-1}$.

Beispiel 26c:

7-[(1R,2S,4R,5R)-2-(4-Phenylbenzyloxy)-4-benzoyloxy-5-hydroxycarbonyl-cyclopentyl]-5(Z)-heptensäuremethylester:

Die Lösung von 2,42 g (4,46 mmol) des nach Beispiel 26d dargestellten Alkohols in 55 ml Aceton kühlt man auf -46°C, versetzt mit 2,67 ml einer standardisierten Chromschwefel säurelösung (Jones-Reagenz), rührt 1,5 Stunden bei -40°C bis -10°C und zersetzt überschüssiges Oxidationsmittel durch Zugabe von 6,6 ml Isopropanol. Man verdünnt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an Kieselgel mit einem Gradientensystem aus n-Hexan und Ethylacetat. Isoliert werden 1,69 g (3,04 mmol, 68%) der Titelverbindung als farbloses Öl.

IR (Film): 3680-2800, 1740-1710, 1605, 1490,1450, 1275, 1115, 765 und 715 $cm^{-1}$.

Beispiel 26d:

7-[(1R,2S,4R,5S)-2-(4-Phenylbenzyloxy)-4-benzoyloxy-5-hydroxymethyl-cyclopentyl]-5(Z)-heptensäuremethylester:

4,29 g (5,49 mmol) 7-[(1R,2S,4R,5S)-2-(4-Phenylbenzyloxy)-4-benzoyloxy-5-(tert. butyldiphenylsilyloxymethyl) -cyclopentyl]-5(Z)-heptensäuremethylester ( Darstellung nach M. Shibasaki et al. THL 25, 1067 (1984), Bsp. 2e, J. S. Bindra, R. Bindra, Prostaglandin Synthesis, Academic Press 1977 ) gelöst in 70 ml wasserfreiem Tetrahydrofuran, versetzt man mit 8,7 g Tetrabutylammoniumfluorid und rührt 1,25 Stunden unter einer Atmosphäre aus trockenem Argon. Man versetzt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an Kieselgel mit einem Gradientensystem aus n-Hexan und Ethylacetat. Isoliert werden 2,42 g (4,46 mmol, 81%) der Titelverbindung als farbloses Öl.

IR (Film): 3640-3200, 2940, 1740-1715, 1605, 1490, 1450, 1315, 1275, 1120, 1070, 1025, 760 und 715 $cm^{-1}$.

Beispiel 27:

7-[(1R,2S,4R,5R)-2-(4-Phenylbenzyloxy)-4-hydroxy-5-(2,4-difluorphenylsulfonylamino)-cyclopentyl]-5(Z)-heptensäure:

86 mg (121 µmol) der nach Beispiel 28 dargestellten Verbindung verseift man in Analogie zu Beispiel 1 und isoliert nach Aufarbeitung und Reinigung 44 mg (76 µmol, 62%) der Titelverbindung als farbloses Öl.

IR (KBr): 3520, 3280, 3025, 2930, 1725, 1600, 1490, 1425, 1340, 1165, 1075, 1020, 860, 760 und 700 $cm^{-1}$.

Beispiel 28:

7-[(1R,2S,4R,5R)-2-(4-Phenylbenzyloxy)-4-benzoyloxy-5-(2,4-difluorphenylsulfonylamino)-cyclopentyl]-5(Z)-heptensäuremethylester:

212 mg (412 µmol) des nach Beispiel 26a dargestellten Amins setzt man in Analogie zu Beispiel 2 unter Verwendung

von 2,4-Difluorphenylsulfonsäurechlorid um und isoliert nach Aufarbeitung und Reinigung 86 mg (121 µmol, 35%) der Titelverbindung als farbloses Öl. IR (Film): 3700-3130, 2940, 1715, 1605, 1490, 1450, 1345, 1275, 1165, 1120, 970, 855, 765 und 715 $cm^{-1}$.

Beispiel 29:

7-[(1R,2S,4R,5R)-2-(4-Phenylbenzyloxy)-4-hydroxy-5-(4-fluorphenylsulfonylamino)-cyclopentyl]-5(Z)-heptensäure:

101 mg (147 µmol) der nach Beispiel 30 dargestellten Verbindung verseift man in Analogie zu Beispiel 1 und isoliert nach Aufarbeitung und Reinigung 38 mg (67 µmol, 45%) der Titelverbindung als farbloses Öl.

IR (KBr): 3500, 3330, 2930, 1730, 1590, 1490, 1445, 1330, 1240, 1155, 1090, 840, 760 und 700 $cm^{-1}$.

Beispiel 30:

7-[(1R,2S,4R,5R)-2-(4-Phenylbenzyloxy)-4-benzoyloxy-5-(4-fluorphenylsulfonylamino)-cyclopentyl]-5(Z)-heptensäuremethylester:

212 mg (412 µmol) des nach Beispiel 26a dargestellten Amins setzt man in Analogie zu Beispiel 2 unter Verwendung von 4-Fluorphenylsulfonsäurechlorid um und isoliert nach Aufarbeitung und Reinigung 101 mg (147 µmol, 42%) der Titelverbindung als farbloses Öl. IR (Film): 3400-3120, 2940, 1715, 1595, 1495, 1450, 1340, 1270, 1155, 840, 765 und 715 $cm^{-1}$.

Beispiel 31:

7-[(1R,2S,4R,5R)-2-(4-Phenylbenzyloxy)-4-hydroxy-5-(4-methylphenylsulfonylamino)-cyclopentyl]-5(Z)-heptensäure:

86 mg (126 µmol) der nach Beispiel 32 dargestellten Verbindung verseift man in Analogie zu Beispiel 1 und isoliert nach Aufarbeitung und Reinigung 21 mg (37 µmol, 29%) der Titelverbindung als farbloses Öl.

IR (KBr): 3600-2800, 3495, 3300, 2940, 1730, 1710, 1600, 1490, 1340, 1320, 1160, 1090, 920, 820, 760 und 700 $cm^{-1}$.

Beispiel 32:

7-[(1R,2S,4R,5R)-2-(4-Phenylbenzyloxy)-4-benzoyloxy-5-(4-methylphenylsulfonylamino)-cyclopentyl]-5(Z)-heptensäuremethylester:

212 mg (412 µmol) des nach Beispiel 26a dargestellten Amins setzt man in Analogie zu Beispiel 2 unter Verwendung von 4-Methylphenylsulfonsäurechlorid um und isoliert nach Aufarbeitung und Reinigung 86 mg (126 µmol, 36%) der Titelverbindung als farbloses Öl. IR (Film): 3400-3120, 2950, 1740-1710, 1605, 1490, 1450, 1270, 1160, 910, 815, 765 und 715 $cm^{-1}$.

Beispiel 33:

7-[(1R, 2S,4R,5R)-2-(4-Phenylbenzyloxy)-4-hydroxy-5-(4-bromphenylsulfonylamino)-cyclopentyl]-5(Z)-heptensäure:

53 mg (71 µmol) der nach Beispiel 34 dargestellten Verbindung verseift man in Analogie zu Beispiel 1 und isoliert nach Aufarbeitung und Reinigung 23 mg (37 µmol, 52%) der Titelverbindung als farbloses Öl.

IR (KBr): 3600-2700, 3500, 3320, 2940, 1730, 1710, 1575, 1390, 1330, 1110, 1090, 1070, 920, 760, 740 und 700 $cm^{-1}$.

Beispiel 34:

7-[(1R,2S,4R,5R)-2-(4-Phenylbenzyloxy)-4-benzoyloxy-5-(4-bromphenylsulfonylamino)-cyclopentyl]-5(Z)-heptensäuremethylester:

212 mg (412 µmol) des nach Beispiel 26a dargestellten Amins setzt man in Analogie zu Beispiel 2 unter Verwendung von 4-Bromphenylsulfonsäurechlorid um und isoliert nach Aufarbeitung und Reinigung 53 mg (71 µmol, 20%) der Titelverbindung als farbloses Öl. IR (Film): 3400-3120, 2950, 1715, 1575, 1490, 1455, 1275, 1165, 1010, 825, 765, 740,und 715 $cm^{-1}$.

Beispiel 35:

6-[(1R,2S,5S)-2-(4-Phenylbenzyloxy)-5-(phenylsulfonylaminomethyl)-cyclopentyl]-4(Z)-hexensäure:

93 mg (170 µmol) der nach Beispiel 36 dargestellten Verbindung verseift man in Analogie zu Beispiel 1 und isoliert nach Aufarbeitung und Reinigung 69 mg (130 µmol, 76%) der Titelverbindung als farbloses Öl.

IR (Flsg.cap.): 3700-2700, 3280, 2920, 1710, 1490, 1450, 1330, 1160, 1090, 760 und 690 $cm^{-1}$.

Beispiel 36:

6-[(1R,2S,5S)-2-(4-Phenylbenzyloxy)-5-(phenylsulfonylaminomethyl)-cyclopentyl]-4(Z)-hexensäuremethylester:

193 mg (470 µmol) des nach Beispiel 36a dargestellten Amins setzt man in Analogie zu Beispiel 2 unter Verwendung von Phenylsulfonsäurechlorid um und isoliert nach Aufarbeitung und Reinigung 93 mg (170 µmol, 36%) der Titelverbindung als farbloses Öl. IR (Film): 3280, 2950, 1720, 1600, 1490, 1435, 1160, 1090, 765 und 700 $cm^{-1}$.

Beispiel 36a:

6-[(1R,2S,5S)-2-(4-Phenylbenzyloxy)-5-aminomethyl-cyclopentyl]-4(Z)-hexensäuremethylester:

Zu der Lösung von 798 mg Zinnchlorid-Dihydrat in 14 ml wasserfreiem Methanol tropft man die Lösung von 1,025 g (2,36 mmol) der nach Beispiel 36b dargestellten Verbindung in 7 ml Methanol und rührt 18 Stunden bei 23°C. Man engt ein, versetzt den Rückstand mit Natriumcarbonatlösung, extrahiert mehrfach mit Ethylacetat, wäscht die vereinigten organischen Extrakte mit gesättigter Natriumchloridlösung und trocknet über Magnesiumsulfat. Den nach Filtration und Lösungsmittelabzug erhaltenen Rückstand (790 mg) setzt man ohne Reinigung weiter um.

Beispiel 36b:

6-[(1R,2S,5S)-2-(4-Phenylbenzyloxy)-5-azidomethyl-cyclopentyl]-4(Z)-hexensäuremethylester:

1,38 mg (2,45 mmol) der nach Beispiel 36c dargestellten Verbindung löst man in 14 ml Dimethylpropylharnstoff, versetzt mit 2,50 g Natriumazid und rührt 16 Stunden bei 40°C unter einer Atmosphäre aus trockenem Argon. Man verdünnt mit Diethylether, wäscht mehrfach mit Wasser und gesättigter Natriumchloridlösung und trocknet über Magnesiumsulfat. Den nach Filtration und Lösungsmittelabzug erhaltenen Rückstand reinigt man durch Chromatographie. Isoliert werden 1,025 g (2,36 mmol, 97%) der Titelverbindung als farbloses Öl.

IR (Film): 2950, 2860, 2100,1740, 1490, 1450, 1345, 1170, 1080, 760 und 700 $cm^{-1}$.

Beispiel 36c:

6-[(1R,2S,5S)-2-(4-Phenylbenzyloxy)-5-(4-methylphenylsulfonyloxymethyl)-cyclopentyl]-4(Z)-hexensäuremethylester:

Die Lösung von 2,1 g (5,14 mmol) der nach Beispiel 36d dargestellten Verbindung in 13,3 ml wasserfreiem Pyridin versetzt man mit 2,1 g p-Toluolsulfonsäurechlorid und rührt 16 Stunden bei 23 °C unter einer Atmosphäre aus trockenem Argon. Man versetzt mit Wasser, verdünnt mit Ethylacetat, wäscht mit 10%iger Schwefelsäure, anschließend mit gesättigter Natriumhydrogencarbonatlösung und trocknet über Natriumsulfat. Den nach Filtration und

Lösungsmittelabzug erhaltenen Rückstand reinigt man durch Chromatographie an Kieselgel unter Verwendung eines Gradientensystems aus n-Hexan und Ethylacetat. Isoliert werden 1,38 g (2,45 mmol, 48%) der Titelverbindung als farbloses Öl.

IR (Film): 3060, 3030, 3005,2945, 2870, 1735, 1605, 1490, 1450, 1360, 1245, 1190, 1175, 1095, 945, 825, 760 und 700 $cm^{-1}$.

Beispiel 36d:

6-[(1R,2S,5S)-2-(4-Phenylbenzyloxy)-5-hydroxymethyl-cyclopentyl]-4-(Z)-hexensäuremethylester:

12,9 g (20 mmol) 6-[(1R,2S,5S)-2-(4-Phenylbenzyloxy)-5-(tert.-butyldiphenylsilyloxymethyl)-cyclopentyl]-4 (Z) -hexensäuremethylester ( siehe PCT/DE91/00604, Bsp.78g )werden in 140 ml wasserfreiem Tetrahydrofuran gelöst, mit 33,4 ml einer 1 M Tetrabutylammoniumfluoridlösung in Tetrahydrofuran versetzt und 16 Stunden bei 23°C unter einer Atmosphäre aus trockenem Argon gerührt. Man versetzt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie mit einem Gradientensystem aus n-Hexan und Ethylacetat. Isoliert werden 7,25 g (17,7 mmol, 89%) der Titelverbindung als farbloses Öl.

IR (Flsg. cap.): 3700-3120, 3000, 2960, 2870, 1740, 1490, 1440, 1345, 1170, 1080, 760 und 700 $cm^{-1}$.

Beispiel 37:

6-[(1R,2S,5S)-2-(4-Phenylbenzyloxy)-5-(4-methylphenylsulfonylaminomethyl)-cyclopentyl]-4(Z)-hexensäure:

126 mg (220 µmol) der nach Beispiel 38 dargestellten Verbindung verseift man in Analogie zu Beispiel 1 und isoliert nach Aufarbeitung und Reinigung 88 mg (163 µmol, 72%) der Titelverbindung als farbloses Öl.

IR (Flsg.cap.): 3420-2800, 3280, 2930, 1710, 1600, 1490, 1330, 1160, 1090, 815, 760 und 700 $cn^{-1}$.

Beispiel 38:

6-[(1R,2S,5S)-2-(4-Phenylbenzyloxy)-5-(4-methylphenylsulfonylaminomethyl)-cyclopentyl]-4(Z)-hexensäuremethylester:

193 mg (470 µmol) des nach Beispiel 36a dargestellten Amins setzt man in Analogie zu Beispiel 2 unter Verwendung von 4-Methylphenylsulfonylchlorid um und isoliert nach Aufarbeitung und Reinigung 126 mg (220 µmol, 48%) der Titelverbindung als farbloses Öl. IR (Film): 3280, 3060, 3025, 2950, 1730, 1600, 1490, 1435, 1360, 1330, 1190, 1080, 830, 760 und 700 $cm^{-1}$.

Beispiel 39:

6-[(1R,2S,5S)-2-(4-Phenylbenzyloxy)-5-(4-fluorphenylsulfonylaminomethyl)-cyclopentyl]-4(Z)-hexensäure:

115 mg (200 µmol) der nach Beispiel 40 dargestellten Verbindung verseift man in Analogie zu Beispiel 1 und isoliert nach Aufarbeitung und Reinigung 84 mg (152 µmol, 74%) der Titelverbindung als farbloses Öl.

IR (Flsg.cap.): 3600-2700, 3280, 2920, 1710, 1590, 1490, 1330, 1150, 1090,840, 760 und 700 $cn^{-1}$.

Beispiel 40:

6-[(1R,2S,5S)-2-(4-Phenylbenzyloxy)-5-(4-fluorphenylsulfonylaminomethyl)-cyclopentyl]4(Z)-hexensäuremethylester:

193 mg (470 µmol) des nach Beispiel 36a dargestellten Amins setzt man in Analogie zu Beispiel 2 unter Verwendung von 4-Fluorphenylsulfonylchlorid um und isoliert nach Aufarbeitung und Reinigung 115 mg (200 µmol, 43%) der Titelverbindung als farbloses Öl IR (Film): 3280, 3060, 3025, 2950, 1730, 1595, 1490, 1435, 1335, 1290, 1235,

1160, 1090, 760 und 700 $cm^{-1}$.

Beispiel 41:

6-[(1R,2S,5S)-2-(4-Phenylbenzyloxy)-5-(2,4-difluorphenylsulfonylaminomethyl)-cyclopentyl]-4(Z)-hexensäure:

144 mg (250 µmol) der nach Beispiel 42 dargestellten Verbindung verseift man in Analogie zu Beispiel 1 und isoliert nach Aufarbeitung und Reinigung 76 mg (133 µmol, 54%) der Titelverbindung als farbloses Öl.

IR (Flsg. cap.): 3700-2700, 3300, 2920, 1710, 1600, 1485, 1430, 1340, 1275, 1170, 1075, 970, 850, 760 und 700 $cm^{-1}$.

Beispiel 42:

6-[(1R,2S,5S)-2-(4-Phenylbenzyloxy)-5-(2,4-difluorphenylsulfonylaminomethyl)-cyclopentyl]-4(Z)-hexensäuremethylester:

193 mg (470 µmol) des nach Beispiel 36a dargestellten Amins setzt man in Analogie zu Beispiel 2 unter Verwendung von 2,4-Difluorphenylsulfonylchlorid um und isoliert nach Aufarbeitung und Reinigung 144 mg (250 µmol, 52%) der Titelverbindung als farbloses Öl. IR (Film): 3280, 3030, 2940, 1730, 1600, 1490, 1425, 1345, 1170, 1075 1030, 860, 760 und 700 $cm^{-1}$.

Beispiel 43:

6-[(1R,2S,5S)-2-(4-Phenylbenzyloxy)-5-(4-bromphenylsulfonylamino)-cyclopentyl]-4(Z)-hexensäure:

213 mg (350 µmol) der nach Beispiel 44 dargestellten Verbindung verseift man in Analogie zu Beispiel 1 und isoliert nach Aufarbeitung und Reinigung 178 mg (296 µmol, 86%) der Titelverbindung als farbloses Öl.

IR (KBr): 3600-2400, 3440, 3220, 2920, 2900, 1700, 1580, 1390, 1155, 1070, 820, 760 und 740 $cm^{-1}$.

Beispiel 44:

6-[(1R,2S,5S)-2-(4-Phenylbenzyloxy)-5-(4-bromphenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von 4-Bromphenylsulfonylchlorid um und isoliert nach Aufarbeitung und Reinigung 213 mg (350 µmol, 6%) der Titelverbindung als farbloses Öl. IR (Film): 3280, 2955, 1735, 1575, 1490, 1440, 1330, 1160,1070, 830, 765 und 700 $cm^{-1}$.

Beispiel 44a:

6-[(1R,2S,5S)-2-(4-Phenylbenzyloxy)-5-amino-cyclopentyl]-4(Z)-hexensäuremethylester:

Die Lösung von 2,1 g (3,91 mmol) der nach Beispiel 44b dargestellten Verbindung in Tetrahydrofuran versetzt man mit 18 ml einer 1M Lösung von Tetrabutylammoniumfluorid in Tetrahydrofuran und erhitzt 3 Stunden auf 50°C. Nach dem Erkalten versetzt man mit Methyl-tert.-butylether, wäscht mit Wasser, gesättigter Natriumchloridlösung und trocknet über Magnesiumsulfat. Den nach Filtration und Lösungsmittelabzug erhaltenen Rückstand setzt man ohne Reinigung weiter um.

Beispiel 44b:

6-[(1R,2S,5S)-2-(4-Phenylbenzyloxy)-5-(trimethylsilylethoxycarbonylamino)-cyclopentyl]-4(Z)-hexensäuremethylester:

Die Lösung von 2,4 g (5,7 mmol) der nach Beispiel 44c dargestellten Säure in 5,6 ml wasserfreiem Toluol versetzt man unter einer Atmosphäre aus trockenem Argon mit 0,78 ml Triethylamin, 1,22 ml Phosphorsäurediphenylesterazid und erhitzt 2 Stunden auf 90°C. Man versetzt mit 1,7 ml 2-(Trimethylsilyl)-ethanol und rührt weitere 22 Stunden

bei 90°C. Nach dem Erkalten verdünnt man mit Methyl-tert.-butylether, wäscht mit 10%iger Natriumhydroxidlösung, gesättigter Natriumchloridlösung und trocknet über Magnesiumsulfat. Den nach Filtration und Lösungsmittelabzug erhaltenen Rückstand reinigt man durch Chromatographie an Kieselgel mit einem Gradientensystem aus n-Hexan und Ethylacetat. Isoliert werden 2,10 g (3,91 mmol, 69%) der Titelverbindung als gelbliches Öl.

IR (Film): 3340, 3060, 2955, 2900, 1740, 1690, 1530, 1490, 1250, 1060, 860,760 und 700 $cm^{-1}$.

<u>Beispiel 44c</u>:

6-[(1R,2S,5S)-2-(4-Phenylbenzyloxy)-5-hydroxycarbonyl-cyclopentyl]-4(Z)-hexensäuremethylester:

Die Lösung von 1,81 g (4,42 mmol) des nach Beispiel 36d dargestellten Alkohols in 50 ml Aceton kühlt man auf -40°C, versetzt mit 2,66 ml einer standardisierten Chromschwefelsäurelösung (Jones-Reagenz), rührt 2 Stunden bei -40°C bis -15°C und zersetzt überschüssiges Oxidationsmittel durch Zugabe von Isopropanol. Man verdünnt mit Wasser, extrahiert mehrfach mit Diethylether, wäscht die vereinigten organischen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an groben Kieselgel mit einem Gradientensystem aus n-Hexan und Ethylacetat. Isoliert werden 1,14 g (2,7 mmol, 61%) der Titelverbindung als gelbliches Öl. IR (Film): 3600-2500, 1735, 1700, 1600, 1485, 1340, 1300, 1235, 760 und 700 $cm^{-1}$.

<u>Beispiel 45</u>:

6-[(1R,2S,5S)-2-(4-Phenylbenzyloxy)-5-(4-trifluormethylphenylsulfonylamino)-cyclopentyl]-4(Z)-hexensäure:

90 mg (150 µmol) der nach Beispiel 46 dargestellten Verbindung verseift man in Analogie zu Beispiel 1 und isoliert nach Aufarbeitung und Reinigung 63 mg (110 µmol, 73%) der Titelverbindung als farbloses Öl.

IR (KBr): 3600-2400, 3440, 3280, 2920, 1710, 1410, 1330, 1160, 1070 und 715 $cm^{-1}$.

<u>Beispiel 46</u>:

6-[(1R,2S,5S)-2-(4-Phenylbenzyloxy)-5-(4-trifluormethylphenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von 4-Trifluormethylphenylsulfonylchlorid um und isoliert nach Aufarbeitung und Reinigung 90 mg (150 µmol, 32%) der Titelverbindung als farbloses Öl.

IR (Film): 3280, 3030, 2930, 1730, 1490, 1330, 1160, 1070, 760 und 700 $cm^{-1}$.

<u>Beispiel 47</u>:

6-[(1R,2S,5S)-2-(4-Phenylbenzyloxy)-5-(2,4-difluorphenylsulfonylamino)-cyclopentyl]-4(Z)-hexensäure:

1,55 mg (270 µmol) der nach Beispiel 48 dargestellten Verbindung verseift man in Analogie zu Beispiel 1 und isoliert nach Aufarbeitung und Reinigung 138 mg (250 µmol, 92%) der Titelverbindung als farbloses Öl.

IR (Flsg. cap.): 3720-2400, 3280, 2930, 1710, 1600, 1490, 1340, 1160, 1070, 970, 760 und 700 $cm^{-1}$.

<u>Beispiel 48</u>:

6-[(1R,2S,5S)-2-(4-Phenylbenzyloxy)-5-(2,4-difluorphenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von 2,4-Difluorphenylsulfonylchlorid um und isoliert nach Aufarbeitung und Reinigung 155 mg (270 µmol, 59%) der Titelverbindung als farbloses Öl. IR (Film): 3280, 3025, 2920, 1735, 1595, 1490, 1345, 1170, 1030,860 und 760 $cm^{-1}$.

Beispiel 49:

6-[(1R,2S,5S)-2-(4-Phenylbenzyloxy)-5-(4-fluorphenylsulfonylamino)-cyclopentyl]-4(Z)-hexensäure:

133 mg (240 µmol) der nach Beispiel 50 dargestellten Verbindung verseift man in Analogie zu Beispiel 1 und isoliert nach Aufarbeitung und Reinigung 128 mg (239 µmol, 98%) der Titelverbindung als farbloses Öl.

IR (Flsg. cap.): 3600-2800, 3280, 2930, 1710, 1590, 1490, 1330, 1240, 1150, 840, 760 und 700 $cm^{-1}$.

Beispiel 50:

6-[(1R,2S,5S)-2-(4-Phenylbenzyloxy)-5-(4-fluorphenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von 4-Fluorphenylsulfonylchlorid um und isoliert nach Aufarbeitung und Reinigung 133 mg (240 µmol, 52%) der Titelverbindung als farbloses Öl. IR (Film): 3280, 3055, 3010, 2940, 1735, 1595, 1490, 1435, 1230, 1165, 1090, 760 und 700 $cm^{-1}$.

Beispiel 51:

6-[(1R,2S,5S)-2-(4-Phenylbenzyloxy)-5-(4-methylphenylsulfonylamino)-cyclopentyl]-4(Z)-hexensäure:

131 mg (238 µmol) der nach Beispiel 52 dargestellten Verbindung verseift man in Analogie zu Beispiel 1 und isoliert nach Aufarbeitung und Reinigung 29 mg (53 µmol, 22%) der Titelverbindung als farbloses Öl.

IR (Flsg. cap.): 3600-2800, 3280, 2930, 1710, 1490, 1425 1330, 1155 und 760 $cm^{-1}$.

Beispiel 52:

6-[(1R,2S,5S)-2-(4-Phenylbenzyloxy)-5-(4-methylphenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von 4-Methylphenylsulfonylchlorid um und isoliert nach Aufarbeitung und Reinigung 131 mg (238 µmol, 51%) der Titelverbindung als farbloses Öl. IR (Film): 3280, 2940, 1735, 1590, 1450, 1425, 1380, 1155, 1095, 900, 760 und 700 $cm^{-1}$.

Beispiel 53:

6-[(1R,2S,5S)-2-(4-Phenylbenzyloxy)-5-(phenylsulfonylamino)-cyclopentyl]-4(Z)-hexensäure:

156 mg (290 µmol) der nach Beispiel 54 dargestellten Verbindung verseift man in Analogie zu Beispiel 1 und isoliert nach Aufarbeitung und Reinigung 96 mg (184 µmol, 63%) der Titelverbindung als farbloses Öl.

IR (Flsg. cap.): 3680-2400, 3290, 2920, 2850, 1710, 1490, 1450, 1330, 1160, 1070, 910, 760 und 700 $cm^{-1}$.

Beispiel 54:

6-[(1R,2S,5S)-2-(4-Phenylbenzyloxy)-5-(phenylsulfonylamino)-cyclopentyl]-4(Z)-hexensäuremethylester:

182 mg (460 µmol) des nach Beispiel 44a dargestellten Amins setzt man in Analogie zu Beispiel 2 unter Verwendung von Phenylsulfonylchlorid um und isoliert nach Aufarbeitung und Reinigung 156 mg (290 µmol, 63%) der Titelverbindung als farbloses Öl.

IR (Film): 3280, 2950, 1730, 1600, 1455, 1425, 1155, 1070, 900 und 760 $cm^{-1}$.

Beispiel 55:

7-[(1R,2S,4R,5S)-2-(4-Phenylbenzyloxy)-4-hydroxy-5-(diphenylmethoxyiminomethyl)-cyclopentyl]-5(Z)-heptensäure:

88,1 mg (142 µmol) der nach Beispiel 56 dargestellten Verbindung verseift man in Analogie zu Beispiel 1 und isoliert nach Aufarbeitung und Reinigung 45,8 mg (75 µmol, 53%) der Titelverbindung

IR (Flsg. cap.): 3700-2400, 3030, 2930, 1705, 1600, 1485, 1450, 1420, 1345, 1125, 1075, 1020, 920, 760 und 700 $cm^{-1}$.

Beispiel 56:

7-[(1R,2S,4R,5S)-2-(4-Phenylbenzyloxy)-4-hydroxy-5-(diphenylmethoxyiminomethyl)cyclopentyl]-5(Z)-heptensäuremethylester:

132,2 mg (188 µmol) der nach Beispiel 57 dargestellten Verbindung löst man in 1,5 ml absolutem Ethanol, versetzt mit 4,8 mg Pyridinium-p-toluolsulfonat und rührt 3 Stunden bei 50°C. Das Lösungsmittel wird abgezogen und der Rückstand durch Chromatographie gereinigt. Isoliert werden 88,1 mg (142 µmol, 76%) der gewünschten Verbindung als farbloses Öl.

IR (Film): 3700-3160, 3040, 2940, 1740, 1605, 1495, 1455, 1350, 1130, 1080, 765 und 700 $cm^{-1}$.

Beispiel 57:

7-[(1R,2S,4R,5S)-2-(4-Phenylbenzyloxy)-4-(tetrahydropyranyloxy)-5-diphenylmethoxyiminomethyl)-cyclopentyl]-5 (Z)-heptensäuremethylester:

120 mg (230 µmol) der nach Beispiel 57a dargestellten Verbindung löst man in 1,7 ml absolutem Ethanol, versetzt mit 124,1 mg (623 µmol) Hydroxylamindiphenylmethylether, einigen Tropfen Pyridin und rührt 20 Stunden bei Raumtemperatur. Man engt ein, verdünnt mit Ethylacetat, wäscht mehrfach mit gesättigter Natriumchloridlösung und trocknet über Natriumsulfat. Den nach Filtration und Lösungsmittelrückstand erhaltenen Rückstand reinigt man durch Chromatographie. Isoliert werden 132,2 mg (188 mol,82%) der gewünschten Verbindung als farbloses Öl.

IR (Film): 3070, 2940, 1740, 1605, 1490, 1405, 1345, 1245, 1130, 1080, 1025, 920, 765 und 700 $cm^{-1}$.

Beispiel 57a:

7-[(1R,2S,4R,5S)-2-(4-Phenylbenzyloxy)-4-(tetrahydropyranyloxy)-5-formyl-cyclopentyl]-5(Z)-heptensäuremethylester:

399 mg (3,143 mmol) Oxalylchlorid, verdünnt mit 3,9 ml absolutem Methylenchlorid, kühlt man auf -60°C unter Argon. Zu dieser Lösung tropft man 549 mg (6,464 mmol) Dimethylsulfoxid in 3,9 ml absolutem Methylenchlorid innerhalb von 10 Minuten und rührt 10 Minuten nach. Eine Lösung von 1 g (1,921 mmol) der nach Beispiel 57b dargestellten Verbindung in 3,9 ml absolutem Methylenchlorid tropft man innerhalb von 10 Minuten zu dieser Mischung und rührt 1,25 Stunden bei -60°C nach. Man versetzt den Ansatz bei -30°C mit 689 mg (6,808 mmol) Triethylamin, läßt auf Raumtemperatur kommen und rührt 15 Minuten nach. Das Reaktionsgemisch verdünnt man mit 200 ml Ether und 10 ml Wasser, wäscht mehrfach mit gesättigter Natriumchloridlösung und trocknet über Natriumsulfat. Den nach Filtration und Lösungsmittelabzug erhaltenen Rückstand setzt man ohne Reinigung weiter um.

Beispiel 57b:

7-[(1R,2S,4R,5S)-2-(4-Phenylbenzyloxy)-4-(tetrahydropyranyloxy)-5-(hydroxymethyl)-cyclopentyl]-5(Z)-heptensäuremethylester:

Die Lösung von 9,31 g (17,8 mmol) 7-[(1R,2S,4R,5S)-2-(4-Phenylbenzyloxy)-4-(tetrahydropyranyloxy)-5-(tert.-butyldiphenylsilyloxymethyl)-cyclopentyl]-5-(Z)-heptensäuremethylester ( M. Shibasaki et al., THL 25, 1067 (1984), Bsp. 2e, J.S. Bindra, R. Bindra, Prostaglandin Synthesis, Academic Press, 1977 )in 235 ml wasserfreiem Tetrahydrofuran versetzt man mit 16,8 g Tetrabutylammoniumfluorid und rührt 15,5 Stunden bei 23°C unter einer Atmosphäre aus trockenem Argon. Man versetzt mit Wasser, extrahiert mehrfach mit Diethylether, wäscht die vereinigten organischen Extrakte mit Wasser und gesättigter Natriumchloridlösung und trocknet über Magnesiumsulfat.

Den nach Filtration und Lösungsmittelabzug erhaltenen Rückstand reinigt man durch Chromatographie an ca. 400g Kieselgel mit einem Gradientensystem aus n-Hexan und Ethylacetat. Isoliert werden 3,71 g (7,1 mmol, 49%) der Titelverbindung als farbloses Öl. IR (Film): 3600-3200, 3020, 2940, 1735, 1600, 1490, 1315, 1245, 1070, 760 und 700 $cm^{-1}$.

Beispiel 58:

7-[(1R,2S,4R,5S)-2-(4-Phenylbenzyloxy)-4-hydroxy-5-(4-fluorbenzyloxyiminomethyl) cyclopentyl]-5(Z)-heptensäure:

75 mg (134 µmol) der nach Beispiel 59 dargestellten Verbindung verseift man in Analogie zu Beispiel 1 und isoliert nach Aufarbeitung und Reinigung 44,2 mg (79 µmol, 61%) der Titelverbindung.

IR (Flsg.cap.): 3600-2500, 2830, 1705, 1600, 1510, 1490, 1410, 1345, 1225, 1155, 1075, 1040, 825, 760 und 700 $cm^{-1}$.

Beispiel 59:

7-[(1R,2S,4R,5S)-2-(4-Phenylbenzyloxy)-4-hydroxy-5-(4-fluorbenzyloxyiminomethyl)-cyclopentyl]-5(Z)-heptensäuremethylester:

125,5 mg (194 mol) der nach Beispiel 60 dargestellten Verbindung setzt man in Analogie zu Beispiel 56 um und isoliert nach Aufarbeitung und Reinigung 75 mg (134 µmol,69%) der Titelverbindung als farbloses Öl.

IR (Film): 3700-3140, 3010, 2940, 2870, 1735, 1605, 1510, 1440, 1350, 1225, 835, 765 und 700 $cm^{-1}$.

Beispiel 60:

7-[(1R,2S,4R,5S)-2-(4-Phenylbenzyloxy)-4-(tetrahydropyranyloxy)-5-(4-fluorbenzyloxyiminomethyl)-cyclopentyl]-5 (Z)-heptensäuremethylester:

120 mg (230 µmol) der nach Beispiel 57a dargestellten Verbindung setzt man in Analogie zu Beispiel 57 unter Verwendung von Hydroxylamin-4-fluorbenzylether um und isoliert nach Aufarbeitung und Reinigung 125,3 mg (194 µmol,84%) der gewünschten Verbindung als farbloses Öl.

IR (Film): 2940, 2870, 1740, 1605, 1510, 1440, 1225, 1155, 1130, 1030, 825, 765 und 700 $cm^{-1}$.

Beispiel 61:

7-[(1R,2S,4R,5S)-2-(4-Phenylbenzyloxy)-4-hydroxy-5-(cyclohexylmethoxyiminomethyl)-cyclopentyl]-5(Z)-heptensäure:

60,1 mg (109 µmol) der nach Beispiel 62 dargestellten Verbindung verseift man in Analogie zu Beispiel 1 und isoliert nach Aufarbeitung und Reinigung 43,5 mg (81 µmol, 74%) der Titelverbindung.

IR (Flsg. cap.): 3700-2400, 2920, 2850, 1710, 1490, 1450, 1410, 1345, 1125, 1080, 1035, 760 und 700 $cm^{-1}$.

Beispiel 62:

7-[(1R,2S,4R,5S)-2-(4-Phenylbenzyloxy)-4-hydroxy-5-(cyclohexylmethoxyiminomethyl)-cyclopentyl ]-5(Z)-heptensäuremethylester:

115,6 mg (183 mol) der nach Beispiel 60 dargestellten Verbindung setzt man in Analogie zu Beispiel 56 um und isoliert nach Aufarbeitung und Reinigung 60,1 mg (109 µmol,59%) der Titelverbindung als farbloses Öl.

IR (Film): 3700-3200, 2930, 2860, 1740, 1490, 1455, 1350, 1220, 1175, 1080, 1040, 765 und 700 $cm^{-1}$.

Beispiel 63:

7-[(1R,2S,4R,5S)-2-(4-Phenylbenzyloxy)-4-(tetrahydropyranyloxy)-5-(cyclohexylmethoxyiminomethyl)-cyclopentyl]-5(Z)-heptensäuremethylester:

120 mg (230 µmol) der nach Beispiel 57a dargestellten Verbindung setzt man in Analogie zu Beispiel 57 unter Verwendung von Hydroxylamin-(cyclohexylmethyl)-ether um und isoliert nach Aufarbeitung und Reinigung 124,6 mg (196 µmol,86%) der gewünschten Verbindung als farbloses Öl.

IR (Film): 2930, 2855, 1740, 1450, 1350, 1130, 1080, 1030, 765 und 700 $cm^{-1}$.

Beispiel 64:

7-[(1R,2S,4R,5S)-2-(4-Phenylbenzyloxy)-4-hydroxy-5-(benzyloxyiminomethyl)-cyclopentyl]-5(Z)-heptensäure:

65,6 mg (121 µmol) der nach Beispiel 65 dargestellten Verbindung verseift man in Analogie zu Beispiel 1 und isoliert nach Aufarbeitung und Reinigung 50,3 mg (96 µmol, 79%) der Titelverbindung.

IR (Flsg. cap.): 3700-2400, 3030, 2930, 1705, 1490, 1455, 1410, 1395, 1125, 1075, 1040, 915, 760, 735 und 700 $cm^{-1}$.

Beispiel 65:

7-[(1R,2S,4R,5S)-2-(4-Phenylbenzyloxy)-4-hydroxy-5-(benzyloxyiminomethyl)-cyclopentyl]-5(Z)-heptensäuremethylester:

108,3 mg (173 mol) der nach Beispiel 66 dargestellten Verbindung setzt man in Analogie zu Beispiel 56 um und isoliert nach Aufarbeitung und Reinigung 65,6 mg (121 µmol,70%) der Titelverbindung als farbloses Öl.

IR (Film): 3700-3140, 3030, 2935, 2885, 1735, 1490, 1455, 1440, 1350, 1215, 1125, 1075, 760 und 700 $cm^{-1}$.

Beispiel 66:

7-[(1R,2S,4R,5S)-2-(4-Phenylbenzyloxy)-4-(tetrahydropyranyloxy)-5-(benzyloxyimino methyl)-cyclopentyl]-5(Z) -heptensäuremethylester:

100 mg (191 µmol) der nach Beispiel 57a dargestellten Verbindung setzt man in Analogie zu Beispiel 57 unter Verwendung von Hydroxylaminbenzylether um und isoliert nach Aufarbeitung und Reinigung 108,3 mg (173 µmol,90%)der gewünschten Verbindung als farbloses Öl.

IR (Film): 2940, 2870, 1740, 1605, 1510, 1440, 1350, 1225, 1030, 835, 765 und 700 $cm^{-1}$.

Beispiel 67:

7-[(1R,2S,4R,5S)-2-(4-Phenylbenzyloxy)-4-hydroxy-5-(4-trifluormethylbenzyloxyiminomethyl)-cyclopentyl]-5(Z) -heptensäure:

61,3 mg (100 µmol) der nach Beispiel 68 dargestellten Verbindung verseift man in Analogie zu Beispiel 1 und isoliert nach Aufarbeitung und Reinigung 46,4 mg (78 µmol, 78%) der Titelverbindung.

IR (Flsg. cap.): 3700-2400, 2930, 1710, 1490, 1415, 1325, 1165, 1125, 1065, 1030, 825, 760 und 700 $cm^{-1}$.

Beispiel 68:

7-[(1R,2S,4R,5S)-2-(4-Phenylbenzyloxy)-4-hydroxy-5-(4-trifluormethylbenzyloxyiminomethyl)-cyclopentyl]-5(Z) -heptensäuremethylester:

108,5 mg (156 mol) der nach Beispiel 69 dargestellten Verbindung setzt man in Analogie zu Beispiel 56 um und

isoliert nach Aufarbeitung und Reinigung 61,3 mg (100 µmol,64,3%) der Titelverbindung als farbloses Öl.

IR (Film): 3700-3200, 3010, 2940, 2870, 1735, 1605, 1510, 1490, 1350, 1225, 1010, 825, 765 und 700 $cm^{-1}$.

Beispiel 69:

7-[(1R,2S,4R,5S)-2-(4-Phenylbenzyloxy)-4-(tetrahydropyranyloxy)-5-(4-trifluormethylbenzyloxyiminomethyl)-cyclopentyl]-5(Z)-heptensäuremethylester:

100 mg (191 µmol) der nach Beispiel 57a dargestellten Verbindung setzt man in Analogie zu Beispiel 57 unter Verwendung von Hydroxylamin-4-trifluormethylbenzylether um und isoliert nach Aufarbeitung und Reinigung 108,5 mg (156 µmol,81%)der gewünschten Verbindung als farbloses Öl

IR (Film): 2940, 2870, 1740, 1620, 1490, 1440, 1325, 1165, 1125, 1065, 820, 765 und 700 $cm^{-1}$.

Beispiel 70:

7-[(1R,2S,4R,5S)-2-(4-Phenylbenzyloxy)-4-hydroxy-5-(1-naphthylmethoxyiminomethyl)-cyclopentyl]-5(Z)-heptensäure:

84,7 mg (143 µmol) der nach Beispiel 71 dargestellten Verbindung verseift man in Analogie zu Beispiel 1 und isoliert nach Aufarbeitung und Reinigung 57,7 mg (100 µmol, 70%) der Titelverbindung.

IR (Flsg. cap.): 3700-2400, 3400, 3070, 3030, 3010, 1705, 1600, 1510, 1490, 1410, 1385, 1345, 1235, 1125, 1070, 915, 760 und 700 $cm^{-1}$.

Beispiel 71:

7-[(1R,2S,4R,5S)-2-(4-Phenylbenzyloxy)4-hydroxy-5-(1-naphthylmethoxyiminomethyl)cyclopentyl]-5(Z)-heptensäuremethylester:

124,1 mg (189 mol) der nach Beispiel 72 dargestellten Verbindung setzt man in Analogie zu Beispiel 56 um und isoliert nach Aufarbeitung und Reinigung 84,7 mg (143 µmol,78%) der Titelverbindung als farbloses Öl.

IR (Film): 3660-3120, 3010, 2935, 2870, 1735, 1600, 1515, 1490, 1435, 1375, 1345, 1220, 1170, 1080, 1010, 800, 795, 780, 760 und 700 $cm^{-1}$.

Beispiel 72:

7-[(1R,2S,4R,5S)-2-(4-Phenylbenzyloxy)-4-(tetrahydropyranyloxy)-5-(1-naphthylmethoxyiminomethyl)-cyclopentyl]-5(Z)-heptensäuremethylester:

100 mg (191 µmol) der nach Beispiel 57a dargestellten Verbindung setzt man in Analogie zu Beispiel 57 unter Verwendung von Hydroxylamin-1-(naphthylmethyl)ether um und isoliert nach Aufarbeitung und Reinigung 124,1 mg (183 µmol,96%)der gewünschten Verbindung als farbloses Öl.

IR (Film): 2940, 2870, 1640, 1605, 1510, 1440, 1350, 1235, 1040, 835, 765 und 700 $cm^{-1}$.

Beispiel 73:

7-[(1R,2S,4R,5S)-2-(4-Phenylbenzyloxy)-4-hydroxy-5-(ω-phenylsemicarbazonomethyl)-cyclopentyl]-5(Z)-heptensäure:

48,2 mg (85 µmol) der nach Beispiel 74 dargestellten Verbindung verseift man in Analogie zu Beispiel 1 und isoliert nach Aufarbeitung und Reinigung 26,5 mg (48 µmol, 56%) der Titelverbindung.

IR (Flsg. cap.): 3700-2400, 3370, 2930, 1710-1630, 1590, 1540, 1450, 1235, 1135, 1100, 1075, 760 und 695 $cm^{-1}$.

Beispiel 74:

7-[(1R,2S,4R,5S)-2-(4-Phenylbenzyloxy)-4-hydroxy-5-(ω-phenylsemicarbazonomethyl)-cyclopentyl]-5(Z)-heptensäuremethylester:

101,4 mg (155 µmol) der nach Beispiel 75 dargestellten Verbindung löst man in 3 ml eines Gemisches aus Eisessig, Wasser und Tetrahydrofuran und rührt 22 Stunden bei Raumtemperatur. Das Lösungsmittel wird abgezogen, der Rückstand viermal mit Toluol versetzt und jeweils trockengezogen. Nach Chromatographie erhält man 65,5 mg (115 µmol,74%) der gewünschten Verbindung als farbloses Öl.

IR (Film): 3700-2700, 3380, 2940, 1735, 1680-1660, 1595, 1535, 1500, 1315, 1230, 1125, 760 und 695 $cm^{-1}$.

Beispiel 75:

7-[(1R,2S,4R,5S)-2-(4-Phenylbenzyloxy)-4-(tetrahydropyranyloxy)-5-(ω-phenylsemicarbazonomethyl)-cyclopentyl]-5(Z)-heptensäuremethylester:

200 mg (384 µmol) der nach Beispiel 57a dargestellten Verbindung setzt man in Analogie zu Beispiel 57 unter Verwendung von ω-Phenylsemicarbazid um und isoliert nach Aufarbeitung und Reinigung 187,7 mg (287 µmol,75%) der gewünschten Verbindung als farbloses Öl.

IR (Film): 3600-2800, 3380, 3210, 2950, 1740, 1695, 1600, 1535, 1500, 1360, 1320, 1225, 1135, 1035, 1025, 760 und 700 $cm^{-1}$.

Beispiel 76:

(8R,9S,11R,12R,15S)-9-(4-Phenylbenzyloxy)-11,15-dihydroxy-5(Z),13(E)-prostadiensäure:

24,6 mg (46 µmol) der nach Beispiel 77 dargestellten Verbindung verseift man in Analogie zu Beispiel 1 und isoliert nach Aufarbeitung und Reinigung 23,4 mg (45 µmol, 98%) der Titelverbindung als farbloses Öl.

IR (Flsg. cap.): 3700-2300, 2930, 2860, 1710, 1490, 1410, 1345, 1240, 1175, 970, 760 und 700 $cm^{-1}$.

Beispiel 77:

(8R,9S,11R,12R,15S)-9-(4-Phenylbenzyloxy)-11,15-dihydroxy-5(Z),13(E)-prostadiensäuremethylester:

83,8 mg (135 mol) der nach Beispiel 78 dargestellten Verbindung (II, polarer Fleck) setzt man in Analogie zu Beispiel 56 um und isoliert nach Aufarbeitung und Reinigung 24,6 mg (46 µmol,34%) der Titelverbindung als farbloses Öl.

IR (Film): 3700-3100, 2930, 2860, 1740, 1490, 1435, 1345, 1075, 970, 760 und 700 $cm^{-1}$.

Beispiel 78:

(8R,9S,11R,12R,15R)-9-(4-Phenylbenzyloxy)-11-(tetrahydopyranyloxy)-15-hydroxy-5(Z),-13(E)-prostadiensäuremethylester(I) und

(8R,9S,11R,12R,15S)-9-(4-Phenylbenzyloxy)-11-(tetrahydopyranyloxy)-15-hydroxy-5(Z),-13(E)-prostadiensäuremethylester(II):

1,15 g (1,858 mmol) der nach Beispiel 79 dargestellten Verbindung löst man in 21 ml wasserfreiem Methanol, kühlt auf -40°C und versetzt portionsweise mit 444 mg (11,728 mmol) Natriumborhydrid. Man rührt bei -40°C 50 Minuten nach, tropft 0,94 ml Eisessig zu und zieht das Methanol ab. Den Rückstand nimmt man in Methylenchlorid auf, wäscht mit gesättigter Natriumchloridlösung und trocknet über Natriumsulfat. Den nach Filtration und Lösungsmittelabzug erhaltenen Rückstand reinigt man durch Chromatographie. Isoliert werden 468,9 mg (758 µmol, 41%) der Titelverbindung I (unpolarer Fleck) und 424,6 mg (687 µmol, 37%) der Titelverbindung II (polarer Fleck) als farblose Öle.

IR (Film),I: 3700-3120, 2940, 2865, 1740, 1490, 1440, 1350, 1135, 1075, 1030, 975, 760 und 700 $cm^{-1}$.

IR Film), II: 3700-3160, 2935, 2860, 1740, 1490, 1440, 1350, 1205, 1135, 1025, 975, 765 und 700 $cm^{-1}$.

Beispiel 79:

(8R,9S,11R,12R)-9-(4-Phenylbenzyloxy)-11-(tetrahydropyranyloxy)-15-oxo-5(Z),13(E)-prostadiensäuremethylester:

Zu einer Suspension von 210 mg (4,371 mmol) 50 %igem Natriumhydrid in 25 ml Dimethoxyethan tropft man bei Raumtemperatur unter einer Atmosphäre aus Argon 979,5 mg (4,408 mmol) (2-Oxoheptyl)-phosphonsäuredimethylester, gelöst in 13 ml Dimethoxyethan.

Man fügt 187,5 mg (4,408 mmol) getrocknetes Lithiumchlorid zu und rührt eine Stunde bei Raumtemperatur. Nach dem Kühlen der Suspension auf -20°, tropft man 2 g (3,841 mmol) der nach Beispiel 57a dargestellten Verbindung, gelöst in Dimethoxyethan, zu und rührt anschließend 2 Stunden bei -20°C und 21 Stunden bei Raumtemperatur. Man kühlt auf -10°C, tropft 0,44 ml Eisessig zu, verdünnt mit Wasser und extrahiert mehrfach mit Ether. Die organischen Phasen werden mehrfach mit 4 %iger Natriumhydrogencarbonatlösung und gesättigter Natriumchloridlösung gewaschen. Den nach Filtration und Lösungsmittelabzug erhaltenen Rückstand reinigt man durch Chromatographie. Isoliert werden 1,16 g (1,86 mmol, 49 %) der Titelverbindung als farbloses Öl.

IR(Film): 2940, 2870, 1740, 1695, 1675, 1630, 1490, 1440, 1350, 1205, 1135, 1035, 765 und 700 $cm^{-1}$.

Beispiel 80:

(8R,9S,11R,12R,15R)-9-(4-Phenylbenzyloxy)-11,15-dihydroxy-5(Z),13(E)-prostadiensäure:

14 mg (26 µmol) der nach Beispiel 81 dargestellten Verbindung verseift man in Analogie zu Beispiel 1 und isoliert nach Aufarbeitung und Reinigung 13,3 mg (25 µmol, 98%) der Titelverbindung als farbloses Öl.

IR (Film): 3700-3200, 3500, 3360, 2930, 2860, 1710, 1675, 1490, 1410, 1345, 1270, 1050, 760 und 700 $cm^{-1}$.

Beispiel 81:

(8R,9S,11R,12R,15R)-9-(4-Phenylbenzyloxy)-11,15-dihydroxy-5(Z),13(E)-prostadiensäuremethylesler:

86,4 mg (139 µmol) der nach Beispiel 78 dargestellten Verbindung (I, unpolarer Fleck) setzt man in Analogie zu Beispiel 56 und isoliert nach Aufarbeitung und Reinigung 14 mg (26 µmol, 19%) der Titelverbindung als farbloses Öl.

IR (Film): 3700-3100, 2925, 2860, 1740, 1485, 1435, 1340, 1075, 970, 760 und 700 $cm^{-1}$.

Beispiel 82:

(8R,9S,12R,15S)-9-(4-Phenylbenzyloxy)-15-hydroxy-5(Z),13(E)-prostadiensäure:

58,2 mg (77 µmol) der nach Beispiel 83 dargestellten Verbindung löst man in 2 ml wasserfreiem Tetrahydrofuran, versetzt mit 292 mg Tetrabutylammoniumfluorid und rührt 17 Stunden bei Raumtemperatur. Nach dem Verdünnen mit Ether wäscht man mit gesättigter Natriumchloridlösung, trocknet über Natriumsulfat, filtriert und rotiert das Lösungsmittel an. Man reinigt durch Chromatographie an Kieselgel. Isoliert werden 14,4 mg (28 µmol, 37%) der Titelverbindung als farbloses Öl.

IR (Flsg. cap.): 3700-2300, 2930, 2860, 1710, 1490, 1455, 1410, 1340, 1240, 1075, 970, 760 und 700 $cm^{-1}$.

Beispiel 83:

(8R,9S,12R,15S)-9-(4-Phenylbenzyloxy)-15-(tert.-butyldiphenylsilyloxy)-5(Z),13(E)-prostadiensäuremethylester:

74,1 mg (80 µmol) der nach Beispiel 84 dargestellten Verbindung löst man in 0,7 ml Dimethoxyethan, versetzt mit

53,5 mg Zinkstaub, 62,1 mg Natriumjodid und 0,04 ml Wasser und erhitzt 16,5 Stunden am Rückfluß. Man filtriert das Reaktionsgemisch, verdünnt mit Ether, schüttelt mit verdünnter Natriumthiosulfatlösung und gesättigter Natriumchloridlösung. Den nach Trocknen über Natriumsulfat, Filtration und Lösungsmittelabzug erhaltenen Rückstand chromatographiert man an Kieselgel. Isoliert werden 58,3 mg (77 µmol, 96%) der Titelverbindung als farbloses Öl.

IR (Film): 2960, 2940, 2860, 1740, 1490, 1430, 1235, 1110, 1070, 825, 760 und 700 $cm^{-1}$.

Beispiel 84:

(8R,9S,11R,12R,15S)-9-(4-Phenylbenzyloxy)-11-(4-methylphenylsulfonyloxy)15-(tert.-butyldiphenylsilyloxy)-5(Z),13(E)-prostadiensäuremethylester:

241 mg (312 µmol) der nach Beispiel 85 dargestellten Verbindung setzt man in Analogie zu Beispiel 14c um und isoliert nach Aufarbeitung und Reinigung 138,5 mg (149 µmol, 48%) der Titelverbindung als farbloses Öl.

IR (Film): 2930, 2860, 1740, 1600, 1430, 1360, 1175, 1010, 925, 825, 765 und 700 $cm^{-1}$.

Beispiel 85:

(8R,9S,11R,12R,15S)-9-(4-Phenylbenzyloxy)-11-hydroxy-15-(tert.-butyldiphenylsilyloxy)-5(Z),13(E)-prostadiensäuremethylester:

425,3 mg (496 mol) der nach Beispiel 86 dargestellten Verbindung setzt man in Analogie zu Beispiel 56 um und isoliert nach Aufarbeitung und Reinigung 241 mg (312 µmol,63%) der Titelverbindung als farbloses Öl.

IR (Film): 3700-3180, 2930, 2860, 1740, 1595, 1425, 1360, 1015, 825, 760 und 700 $cm^{-1}$.

Beispiel 86:

(8R,9S, 11R,12R,15S)-9-(4-Phenylbenzyloxy)-11-(tetrahydropyranyloxy)-15-(tert.-butyldiphenylsilyloxy)-5(Z),13(E)-prostadiensäuremethylester:

329,1 mg (532 µmol) der nach Beispiel 78 dargestellten Verbindung (II, polarer Fleck) löst man in 2,1 ml wasserfreiem Dimethylformamid, versetzt mit 0,351 ml (1,349 µmol) tert.-Butyldiphenylsilylchlorid und 181 mg (2,660 mmol) Imidazol und rührt 2,5 Stunden bei Raumtemperatur. Man verdünnt mit Ether, wäscht mehrfach mit gesättigter Natriumhydrogencarbonatlösung und gesättigter Natriumchloridlösung, trocknet über Magnesiumsulfat und zieht das Lösungsmittel ab. Nach Chromatographie an Kieselgel isoliert man 439,1 mg (512 µmol,96%) der Titelverbindung als farbloses Öl.

IR (Film): 2940, 2855, 1740, 1600, 1430, 1360, 1010, 760 und 700 $cm^{-1}$.

Beispiel 87:

(8R,9S,12R,15R)-9-(4-Phenylbenzyloxy)-15-hydroxy-5(Z),13(E)-prostadiensäure:

63,2 mg (83 µmol) der nach Beispiel 88 dargestellten Verbindung setzt man in Analogie zu Beispiel 82 um und isoliert nach Aufarbeitung und Reinigung 13,4 mg (26 mol,32%) der Titelvebindung als farbloses Öl.

IR (Flsg. cap): 3600-2400, 2930, 2860, 1710, 1490, 1460, 1410, 1390, 1240, 1135, 1070, 970, 760 und 700 $cm^{-1}$.

Beispiel 88:

(8R,9S,12R,15R)-9-(4-Phenylbenzyloxy)-15-(tert.-butyldiphenylsilyloxy)-5(Z),13(E)-prostadiensäuremethylester:

80,4 mg (86 µmol) der nach Beispiel 89 dargestellten Verbindung setzt man in Analogie zu Beispiel 83 um und isoliert nach Aufarbeitung und Reinigung 63,2 mg (83 mol,96%) der Titelverbindung als farbloses Öl.

IR (Film): 2960, 2935, 2860, 1740, 1490, 1465, 1430, 1245, 1110, 1075, 825, 760, 740 und 700 $cm^{-1}$.

Beispiel 89:

(8R,9S,11R,12R,15R)-9-(4-Phenylbenzyloxy)-11-(4-methylphenylsulfonyloxy)15-(tert. butyldiphenylsilyloxy)-5(Z),13(E)-prostadiensäuremethylester:

206,4 mg (267 µmol) der nach Beispiel 90 dargestellten Verbindung setzt man in Analogie zu Beispiel 14c um und isoliert nach Aufarbeitung und Reinigung 139,9 mg (148 µmol, 57%) der Titelverbindung als farbloses Öl.

IR (Film): 2935, 2860, 1740, 1595, 1430, 1360, 1175, 1010, 925, 825, 765 und 700 $cm^{-1}$.

Beispiel 90:

(8R,9S,11R,12R,15R)-9-(4-Phenylbenzyloxy)-11-hydroxy-15-(tert.-butyldiphenylsilyloxy)-5(Z),13(E)-prostadiensäuremethylester:

490,7 mg (572 µmol) der nach Beispiel 91 dargestellten Verbindung setzt man in Analogie zu Beispiel 56 um und isoliert nach Aufarbeitung und Reinigung 206,4 mg (267 µmol,47%) der Titelverbindung als farbloses Öl.

IR (Film): 3700-3200, 2925, 2860, 1735, 1595, 1360, 1015, 825, 760 und 700 $cm^{-1}$.

Beispiel 91:

(8R,9S,11R,12R,15R)-9-(4-Phenylbenzyloxy)-11-(tetrahydropyranyloxy)-15-(tert.-butyldiphenylsilyloxy)-5(Z),13(E)-prostadiensäuremethylester:

370 mg (598 µmol) der nach Beispiel 78 dargestellten Verbindung (I, unpolarer Fleck) setzt man in Analogie zu Beispiel 86 um und isoliert nach Aufarbeitung und Reinigung 501,9 mg (585 µmol,98%) der Titelverbindung als farbloses Öl.

IR (Film): 3000, 2930, 2860, 1740, 1490, 1430, 1350, 1010, 1075, 1020, 975, 820, 760, 740 und 700 $cm^{-1}$.

Beispiel 92:

(8R,9S,11R,12R,15S)-9-(4-Phenylbenzyloxy)-11,15-dihydroxy-15-cyclohexyl-16,17,18,19,20-pentanor-5(Z),13(E)-prostadiensäure:

43,1 mg (79 µmol) der nach Beispiel 93 dargestellten Verbindung verseift man in Analogie zu Beispiel 1 und isoliert nach Aufarbeitung und Reinigung 31,6 mg (59 µmol, 75%) der Titelverbindung als farbloses Öl.

IR (Flsg. cap.): 3700-2300, 2920, 2850, 1710, 1490, 1450, 1410, 1345, 1075, 1010, 970, 760 und 700 $cm^{-1}$.

Beispiel 93:

(8R,9S,11R,12R,15S)-9-(4-Phenylbenzyloxy)-11,15-dihydroxy-15-cyclohexyl-16,17,18,19, 20-pentanor-5(Z),13(E)-prostadiensäuremethylester:

82,3 mg (130 mol) der nach Beispiel 94 dargestellten Verbindung (II, polarer Fleck) setzt man in Analogie zu Beispiel 56 um und isoliert nach Aufarbeitung und Reinigung 43,1 mg (79 µmol,60%) der Titelverbindung als farbloses Öl.

IR (Film): 3700-3100, 2830, 2750, 1740, 1490, 1450, 1345, 1215, 1170, 1075, 1010, 975, 760 und 700 $cm^{-1}$.

Beispiel 94:

(8R,9S,11R,12R,15R)-9-(4-Phenylbenzyloxy)-11-tetrahydropyranyloxy-15-hydroxy-15-cyclohexyl-16,17,18,19,20-pentanor-5(Z),13(E)-prostadiensäuremethylester (I) und (8R,9S,11R,1 2R, 155)-9-(4-Phenylbenzyloxy)-11-tetrahydropyranyloxy-15-hydroxy-15-cyclohexyl-16,17,18,19,20-pentanor-5(Z),13(E)-prostadiensäurem ethylester (II):

1,467 g (2,332 mmol) der nach Beispiel 95 dargestellten Verbindung wird in Analogie zu Beispiel 78 umgesetzt.

Isoliert werden 487,2 mg (773 µmol,33%) der Titelverbindung I (unpolarer Fleck) und 461,5 mg (732 µmol,32%) der Titelverbindung II (polarer Fleck). IR (Film),I: 3700-3200, 2930, 2860, 1740, 1490, 1450, 1350, 1245, 1200, 1135, 1075, 1025, 975, 765 und 700 $cm^{-1}$.

Beispiel 95:

(8R,9S,11R,12R)-9-(4-Phenylbenzyloxy)-11-(tetrahydropyranyloxy)-15-oxo-15-cyclohexyl-16,17,18,19,20-pentanor-5(Z),13(E)-prostadiensäuremethylester:

2 g (3,841 mmol) der nach Beispiel 57a dargestellten Verbindung setzt man in Analogie zu Beispiel 79 um und isoliert nach Aufarbeitung und Reinigung 1,467 g (2,332 mol,61%) der Titelverbindung als farbloses Öl.

IR (Film): 2940, 2860, 1740, 1695, 1670, 1630, 1450, 1350, 1250, 1205, 1135, 1080, 1035, 765 und 700 $cm^{-1}$.

Beispiel 96:

(8R,9S,11R,12R,15R)-9-(4-Phenylbenzyloxy)-11,15-dihydroxy-15-cyclohexyl-16,17,18,19, 20-pentanor-5(Z),13 (E)-prostadiensäure:

27,5 mg (50 µmol) der nach Beispiel 97 dargestellten Verbindung verseift man in Analogie zu Beispiel 1 und isoliert nach Aufarbeitung und Reinigung 26,1 mg (490 µmol,97%) der Titelverbindung als farbloses Öl.

IR (Flsg. cap.): 3700-2200, 2920, 2850, 1720, 1490, 1450, 1410, 1345, 1250, 1075, 1020, 760 und 700 $cm^{-1}$.

Beispiel 97:

(8R,9S,11R,12R,15R)-9-(4-Phenylbenzyloxy)-11,15-dihydroxy-15-cyclohexyl -16,17,18,19,20-pentanor-5(Z),13 (E)-prostadiensäuremethylester:

84,5 mg (134 mol) der nach Beispiel 94 dargestellten Verbindung (I, unpolarer Fleck) setzt man in Analogie zu Beispiel 56 um und isoliert nach Aufarbeitung und Reinigung 27,5 mg (50 µmol,38%) der Titelverbindung als farbloses Öl.

IR (Film): 3680-3100, 2825, 2750, 1740, 1490, 1445, 1345, 1215, 1070, 975, 760 und 700 $cm^{-1}$.

Beispiel 98:

(8R,9S,11S,12R,15S)-9-(4-Phenylbenzyloxy)-1 1 ,15-dihydroxy-15-cyclohexyl-16,17,18,19, 20-pentanor-5(Z),13 (E)-prostadiensäure:

45,5 mg (83 µmol) der nach Beispiel 99 dargestellten Verbindung verseift man in Analogie zu Beispiel 1 und isoliert nach Aufarbeitung 35,2 mg (66 µmol, 79%) der Titelverbindung als farbloses Öl.

IR (Flsg. cap.): 3700-2300, 2920, 2850, 1710, 1490, 1450, 1240, 1060, 760 und 700 $cm^{-1}$.

Beispiel 99:

(8R,9S,11S,12R,15S)-9-(4-Phenylbenzyloxy)-11,15-dihydroxy-15-cyclohexyl-16,17,18,19, 20-pentanor-5(Z),13(E) -prostadiensäuremethylester:

105,6 mg (134 mol) der nach Beispiel 100 dargestellten Verbindung setzt man in Analogie zu Beispiel 57b um und isoliert nach Aufarbeitung und Reinigung 45,5 mg (83 µmol,62%) der Titelverbindung als farbloses Öl.

IR (Film): 3700-3120, 2930, 2850, 1735, 1490, 1450, 1215, 1170, 1010, 975, 760 und 700 $cm^{-1}$.

Beispiel 100:

(8R,9S,11S,12R,15S)-9-(4-Phenylbenzyloxy)-11-hydroxy-15-(tert.-butyldiphenylsilyloxy)-15-cyclohexyl-16,17,18,19,20-pentanor-5(Z),13(E)-prostadiensäuremethylester:

187,1 mg (199 µmol) der nach Beispiel 101 dargestellten Verbindung löst man in 5,5 ml wasserfreiem Dimethylformamid, versetzt mit 248,7 mg (2,922 mmol) Kaliumnitrit und rührt 18 Stunden bei 85°C. Das Reaktionsgemisch wird mit Wasser und Methylenchlorid verdünnt und die organischen Phasen mehrfach mit gesättigter Natriumchloridlösung gewaschen. Nach Trocknen mit Natriumsulfat, Abzug des Lösungsmittels und Chromatographie des Rückstandes verbleiben 105,6 mg (134 µmol,69%) der Titelverbindung als farbloses Öl.

IR (Film): 3700-3130, 2925, 2850, 1735, 1490, 1455, 1175, 1010, 925, 760 und 700 $cm^{-1}$.

Beispiel 101:

(8R,9S,11R,12R,15S)-9-(4-Phenylbenzyloxy)-11-(4-methylphenylsulfonyloxy)-15-(tert.-butyldiphenylsilyloxy) -15-cyclohexyl-16,17,18,19,20-pentanor-5(Z),13(E)-prostadiensäuremethylester:

270,4 mg (344 mol) der nach Beispiel 102 dargestellten Verbindung setzt man in Analogie zu Beispiel 14c um und isoliert nach Aufarbeitung und Reinigung 298 mg (318 µmol,92%) der Titelverbindung als farbloses Öl.

IR (Film): 3120-2860, 2930, 2860, 1740, 1600, 1490, 1450, 1430, 1260, 1175, 1120, 925, 820, 740 und 700 $cm^{-1}$.

Beispiel 102:

(8R,9S,11R,12R,15S)-9-(4-Phenylbenzyloxy)-11-hydroxy-15-(tert.-butyldiphenylsilyloxy)-15-cyclohexyl-16,17,18,19,20-pentanor-5(Z),13(E)-prostadiensäuremethylester:

486,3 mg (559 µmol) der nach Beispiel 103 dargestellten Verbindung setzt man in Analogie zu Beispiel 56 um und isoliert nach Aufarbeitung und Reinigung 270,4 mg (344 µmol,62%) der Titelverbindung als farbloses Öl.

IR (Film): 3680-3120, 2925, 2840, 1740, 1485, 1455, 1175, 1020, 930, 760 und 700 $cm^{-1}$.

Beispiel 103:

(8R,9S,11R,12R,15S)-9-(4-Phenylbenzyloxy)-11-(tetrahydropyranyloxy)-15-(tert.-butyldiphenylsilyloxy)-15-cyclohexyl-16,17,18,19,20-pentanor-5(Z),13(E)-prostadiensäuremethyl ester:

368,2 mg (598 mol) der nach Beispiel 94 dargestellten Verbindung (II, polarer Fleck) setzt man in Analogie zu Beispiel 86 um und isoliert nach Aufarbeitung und Reinigung 498,4 mg (573 µmol,98%) der Titelverbindung als farbloses Öl.

IR (Film): 2930, 2860, 1740, 1485, 1460, 1440, 1360, 1160, 1110, 830, 760 und 700 $cm^{-1}$.

Beispiel 104:

(8R,9S,11S,12R,15R)-9-(4-Phenylbenzyloxy)-11,15-dihydroxy-15-cyclohexyl-16,17,18,19, 20-pentanor-5(Z),13(E) -prostadiensäure:

19,6 mg (36 µmol) der nach Beispiel 105 dargestellten Verbindung verseift man in Analogie zu Beispiel 1 und isoliert nach Aufarbeitung 17,5 mg (33 µmol,92%) der Titelverbindung als farbloses Öl.

IR (Flsg. cap.): 3700-2200, 2920, 2850, 1710, 1485, 1450, 1240, 1065, 1010, 760 und 700 $cm^{-1}$.

Beispiel 105:

(8R,9S,11S,12R,15R)-9-(4-Phenylbenzyloxy)-11,15-dihydroxy-15-cyclohexyl-16,17,18,19, 20-pentanor-5(Z),13(E) -prostadiensäuremethylester:

45,1 mg (57 mol) der nach Beispiel 106 dargestellten Verbindung setzt man in Analogie zu Beispiel 57b um und isoliert nach Aufarbeitung und Reinigung 19,6 mg (36 µmol,62%) der Titelverbindung als farbloses Öl.

IR (Film): 3680-3120, 2925, 2850, 1740, 1490, 1450, 1220, 1180, 975, 760 und 700 $cm^{-1}$.

Beispiel 106:

(8R,9S,11S,12R,15R)-9-(4-Phenylbenzyloxy)-11-hydroxy-15-(tert.-butyldiphenylsilyloxy)-15-cyclohexyl-16,17,18,19,20-pentanor-5(Z),13(E)-prostadiensäuremethylester:

167,6 mg (178 mol) der nach Beispiel 107 dargestellten Verbindung setzt man in Analogie zu Beispiel 100 um und isoliert nach Aufarbeitung und Reinigung 45,1 mg (57 µmol,32%) der Titelverbindung als farbloses Öl.

IR (Film): 3700-3120, 2930, 2850, 1740, 1490, 1455, 1175, 1020, 930, 760 und 700 $cm^{-1}$.

Beispiel 107:

(8R,9S,11R,12R,15R)-9-(4-Phenylbenzyloxy)-11-(4-methylphenylsulfonyloxy)-15-(tert.-butyldiphenylsilyloxy) -15-cyclohexyl-16,17,18,19,20-pentanor-5(Z),13(E)-prostadiensäure methylester:

264,2 mg (336 mol) der nach Beispiel 108 dargestellten Verbindung setzt man in Analogie zu Beispiel 14c um und isoliert nach Aufarbeitung und Reinigung 261,9 mg (278 µmol, 83%) der Titelverbindung als farbloses Öl.

IR (Film): 3100-2760, 2930, 2860, 1740, 1715, 1600, 1490, 1450, 1430, 1360, 1220, 1175, 1100, 820, 760 und 700 $cm^{-1}$.

Beispiel 108:

(8R,9S,11R,12R,15R)-9-(4-Phenylbenzyloxy)-11-hydroxy-15-(tert.-butyldiphenylsilyloxy)-15-cyclohexyl-16,17,18,19,20-pentanor-5(Z),13(E)-prostadiensäuremethylester:

507,7 mg (584 µmol) der nach Beispiel 109 dargestellten Verbindung setzt man in Analogie zu Beispiel 56 um und isoliert nach Aufarbeitung und Reinigung 264,2 mg (336 µmol,58%) der Titelverbindung als farbloses Öl.

IR (Film): 3680-3120, 2930, 2840, 1735, 1485, 1460, 1220, 1175, 1100, 760 und 700 $cm^{-1}$.

Beispiel 109:

(8R,9S,11R,12R,15R)-9-(4-Phenylbenzyloxy)-11(tetrahydropyranyloxy)-15-(tert.-butyldiphenylsilyloxy)-15-cyclohexyl-16,17,18,19,20-pentanor-5(Z),13(E)-prostadiensäuremethylester:

392,1 mg (621 µmol) der nach Beispiel 94 dargestellten Verbindung (I, unpolarer Fleck) setzt man in Analogie zu Beispiel 86 um und isoliert nach Aufarbeitung und Reinigung 520,9 mg (599 µmol,96%) der Titelverbindung als farbloses Öl.

IR (Film): 2930, 2860, 1735, 1485, 1455, 1440, 1355, 1110, 830, 760 und 700 $cm^{-1}$.

Beispiel 110:

(8R,9S,12R,15S)-9-(4-Phenylbenzyloxy)-15-hydroxy-15-cyclohexyl-16,17,18,19,20-pentanor-5(Z),13(E)-prostadiensäure:

86,9 mg (113 µmol) der nach Beispiel 111 dargestellten Verbindung setzt man in Analogie zu Beispiel 82 um und isoliert nach Aufarbeitung und Reinigung 19,5 mg (38 µmol,33%) der Titelverbindung als farbloses Öl.

IR (Flsg. cap.): 3700-2300, 3010, 2920, 2850, 1705, 1490, 1450, 1410, 1390, 1345, 1310, 1175, 1020, 1010, 760 und 695 $cm^{-1}$.

Beispiel 111:

(8R,9S,12R,15S)-9-(4-Phenylbenzyloxy)-15-(tert.-butyldiphenylsilyloxy)-15-cyclohexyl-16,17,18,19,20-pentanor-5(Z),13(E)-prostadiensäuremethylester:

110,9 mg (118 µmol) der nach Beispiel 101 dargestellten Verbindung setzt man in Analogie zu Beispiel 83 um und isoliert nach Aufarbeitung und Reinigung 86,9 mg (113 µmol,96%) der Titelverbindung als farbloses Öl.

IR (Film): 2930, 2860, 1740, 1490, 1450, 1430, 1240, 1110, 825, 760 und 700 $cm^{-1}$.

Beispiel 112:

(8R,9S,12R,15R)-9-(4-Phenylbenzyloxy)-15-hydroxy-15-cyclohexyl-16,17,18,19,20-pentanor-5(Z),13(E)-prostadiensäure:

76,4 mg (99 µmol) der nach Beispiel 113 dargestellten Verbindung setzt man in Analogie zu Beispiel 82 um und isoliert nach Aufarbeitung und Reinigung 23,2 mg (45 µmol,45%) der Titelverbindung als farbloses Öl.

IR (Flsg. cap.): 3700-2400, 2920, 2850, 1710, 1490, 1450, 1345, 1235, 1120, 1080, 1010, 970, 760 und 700 $cm^{-1}$.

Beispiel 113:

(8R,9S,12R,15R)-9-(4-Phenylbenzyloxy)-15-(tert.-butyldiphenylsilyloxy)-15-cyclohexyl16,17,18,19,20-pentanor-5 (Z),13(E)-prostadiensäuremethylester:

94,3 mg (100 µmol) der nach Beispiel 107 dargestellten Verbindung setzt man in Analogie zu Beispiel 83 um und isoliert nach Aufarbeitung und Reinigung 76,4 mg (99 µmol,99%) der Titelverbindung als farbloses Öl.

IR (Film): 2930, 2860, 1740, 1490, 1450, 1430, 1365, 1115, 1070, 765 und 700 $cm^{-1}$.

Beispiel 114:

(8R,9S,11R,12R,15S,16S)-9-(4-Phenylbenzyloxy)-11,15-dihydroxy-16,21-dimethyl-5(Z),13-(E)-prostadien-18-in-säure:

56,4 mg (101 µmol) der nach Beispiel 115 dargestellten Verbindung verseift man in Analogie zu Beispiel 1 und isoliert nach Aufarbeitung 34,9 mg (64 µmol,62%) der Titelverbindung als farbloses Öl.

IR (Flsg. cap.): 3700-2400, 2970, 2930, 1705, 1485, 1455, 1410, 1340, 1240, 1125, 1075, 1010, 970, 825, 760 und 700 $cm^{-1}$.

Beispiel 115:

(8R,9S,11R,12R,15S,16S)-9-(4-Phenylbenzyloxy)-11,15-dihydroxy-16,21-dimethyl-5(Z),13-(E)-prostadien-18-in-säuremethylester:

104,7 mg (163 mol) der nach Beispiel 116 dargestellten Verbindung (II, polarer Fleck) setzt man in Analogie zu Beispiel 56 um und isoliert nach Aufarbeitung und Reinigung 65 mg (116 µmol,71%) der Titelverbindung als farbloses Öl.

IR (Film): 3700-3120, 2980, 2940, 1740, 1490, 1455, 1440, 1220, 1175, 1130, 1080, 1015, 975, 770 und 700 $cm^{-1}$.

Beispiel 116:

(8R,9S,11R,12R,15R,16S)-9-(4-Phenylbenzyloxy)-(11-tetrahydropyranyloxy)-15-hydroxy-16,21-dimethyl-5(Z),13 (E)-prostadien-18-in-säuremethylester (I) und (8R,9S,IIR,12R,15S,16S)-9-(4-Phenylbenzyloxy)-(11-tetrahydropyranyloxy)-15-hydroxy 16,21-dimethyl-5(Z),13(E)-prostadien-18-in-säuremethylester (II):

1,85 g (2,887 mmol) der nach Beispiel 117 dargestellten Verbindung wird in Analogie zu Beispiel 78 umgesetzt. Isoliert werden 852,9 mg (1,32 mmol,46%) der Titelverbindung I (unpolarer Fleck) und 663 mg (1,03 mmol,36%) der Titelverbindung II (polarer Fleck). IR (Film), I: 3680-3200, 2940, 2880, 1740, 1490, 1455, 1440, 1360, 1240,

1140, 1080, 1025, 765 und 700 $cm^{-1}$.

IR (Film), II: 3680-3200, 2880, 1740, 1490, 1455, 1440, 1215, 1130, 760 und 700 $cm^{-1}$.

Beispiel 117:

(8R,9S,11R,12R,16S)-9-(4-Phenylbenzyloxy)-(11-tetrahydropyranyloxy-15-oxo-16,21-dimethyl-5(Z),13(E)-prostadien-18-in-säuremethylester:

2 g (3,841 mmol) der nach Beispiel 57a dargestellten Verbindung setzt man in Analogie zu Beispiel 79 unter Verwendung des entsprechenden Phosphonats um und isoliert nach Aufarbeitung und Reinigung 1,857 g (2,887 mmol,75%) der Titelverbindung als farbloses Öl.

IR (Film): 2940, 2880, 1740, 1695, 1670, 1630, 1490, 1455, 1440, 1375, 1360, 1245, 1135, 1075, 1035, 765 und 700 $cm^{-1}$.

Beispiel 118:

(8R,9S,11R,12R,15R,16S)-9-(4-Phenylbenzyloxy)-11,15-dihydroxy-16,21-dimethyl -5(Z), 13(E)-prostadien-18-in-säure:

76,64 mg (137 µmol) der nach Beispiel 119 dargestellten Verbindung verseift man in Analogie zu Beispiel 1 und isoliert nach Aufarbeitung 67,3 mg (122 µmol,89%) der Titelverbindung als farbloses Öl.

IR (Flsg. cap.): 3700-2400, 3380, 2970, 2920, 1705, 1490, 1450, 1410, 1370, 1345, 1240, 1050, 1015, 965, 760 und 700 $cm^{-1}$.

Beispiel 119:

(8R,9S,11R,12R,15R,16S)-9-(4-Phenylbenzyloxy)-11,15-dihydroxy-16,21-dimethyl-5(Z), 13(E)-prostadien-18-in-säuremethylester:

114,5 g (178 µmol) der nach Beispiel 116 dargestellten Verbindung (I, unpolarer Fleck) wird in Analogie zu Beispiel 56 umgesetzt. Isoliert werden nach Aufarbeitung und Reinigung 86,4 mg (86,4 µmol,87%) der Titelverbindung als farbloses Öl.

IR (Film): 3700-3120, 2970, 2940, 1740, 1490, 1455, 1440, 1350, 1250, 1215, 1075, 975, 835, 765 und 700 $cm^{-1}$.

Beispiel 120:

(8R,9S,11S,12R,15S,16S)-9-(4-Phenylbenzyloxy)-11,15-dihydroxy-16,21-dimethyl-5(Z),13-(E)-prostadien-18-in-säure:

76,9 mg (137 µmol) der nach Beispiel 121 dargestellten Verbindung verseift man in Analogie zu Beispiel 1 und isoliert nach Aufarbeitung 53 mg (96 µmol,69%) der Titelverbindung als farbloses Öl.

IR (Flsg. cap.): 3600-2800, 2970, 2940, 1710, 1485, 1410, 1340, 1125, 1060, 760 und 700 $cm^{-1}$.

Beispiel 121:

(8R,9S,11S,12R,15S,16S)-9-(4-Phenylbenzyloxy)-11,15-dihydroxy-16,21-dimethyl-5(Z),13-(E)-prostadien-18-in-säuremethylester:

185,5 mg (232 mol) der nach Beispiel 122 dargestellten Verbindung setzt man in Analogie zu Beispiel 36d um und isoliert nach Aufarbeitung und Reinigung 87,1 mg (156 µmol,67%) der Titelverbindung als farbloses Öl.

IR (Film): 3660-3100, 2970, 2930, 1735, 1490, 1455, 1435, 1240, 1220, 1120, 1015, 980, 760 und 700 $cm^{-1}$.

Beispiel 122:

(8R,9S,11S,12R,15S,16S)-9-(4-Phenylbenzyloxy)-11-hydroxy-15-(tert.butyldiphenylsilyloxy)-16,21-dimethyl-5(Z),13(E)-prostadien-18-in-säuremethylester:

313,4 mg (332 mol) der nach Beispiel 123 dargestellten Verbindung setzt man in Analogie zu Beispiel 100 um und isoliert nach Aufarbeitung und Reinigung 196 mg (246 µmol,75%) der Titelverbindung als farbloses Öl.

IR (Film): 3620-3200, 2960, 2930, 2860, 1740, 1490, 1430, 1245, 1120, 1060, 825, 765, 745 und 705 $cm^{-1}$.

Beispiel 123:

(8R,9S,11R,12R,15S,16S)-9-(4-Phenylbenzyloxy)-11-(4-methylphenylsulfonyloxy)-15(tert.butyldiphenylsilyloxy)-16,21-dimethyl-5(Z),13(E)-prostadien-18-in-säuremethylester:

377,7 mg (474 mol) der nach Beispiel 124 dargestellten Verbindung setzt man in Analogie zu Beispiel 14c um und isoliert nach Aufarbeitung und Reinigung 420,5 mg (442 µmol, 93%) der Titelverbindung als farbloses Öl.

IR (Film): 2960, 2930, 2860, 1735, 1600, 1490, 1430, 1360, 1220, 1175, 1110, 825, 765, 740 und 705 $cm^{-1}$.

Beispiel 124:

(8R,9S,11R,12R,15S,16S)-9-(4-Phenylbenzyloxy)-11-hydroxy-15-(tert.butyldiphenylsilyloxy)-16,21-dimethyl-5(Z),13(E)-prostadien-18-in-säuremethylester:

725,7 mg (823 mol) der nach Beispiel 125 dargestellten Verbindung setzt man in Analogie zu Beispiel 56 um und isoliert nach Aufarbeitung und Reinigung 388,2 mg (489 µmol,59%) der Titelverbindung als farbloses Öl.

IR (Film): 3630-3200, 2960, 2930, 2860, 1710, 1490, 1460, 1110, 1060, 975, 825, 760, 740 und 700 $cm^{-1}$.

Beispiel 125:

(8R,9S,11R,12R,15S,16S)-9-(4-Phenylbenzyloxy)-11-(tetrahydropyranyloxy)-15-(tert.-butyldiphenylsilyloxy)-16,21-dimethyl-5(Z),13(E)-prostadien-18-in-säuremethylester:

558,3 mg (868 mol) der nach Beispiel 116 (II, polarer Fleck) dargestellten Verbindung setzt man in Analogie zu Beispiel 86 um und isoliert nach Aufarbeitung und Reinigung 740,1 mg (840 µmol,97%) der Titelverbindung als farbloses Öl.

IR (Film): 2960, 2930, 2860, 1740, 1590, 1430, 1360, 1110, 1080, 1030, 820, 760 und 700 $cm^{-1}$.

Beispiel 126:

(8R,9S,11S,12R,15R,16S)-9-(4-Phenylbenzyloxy)-11,15-dihydroxy-16,21-dimethyl-5(Z),13-(E)-prostadien-18-in-säure:

65,1 mg (116 µmol) der nach Beispiel 127 dargestellten Verbindung verseift man in Analogie zu Beispiel 1 und isoliert nach Aufarbeitung und Reinigung 22,2 mg (41 µmol, 35%) der Titelverbindung als farbloses Öl.

IR (Flsg. cap.): 3700-2500, 2970, 2930, 1710, 1490, 1450, 1405, 1345, 1320, 1240, 1115, 1065, 1010, 970, 825, 760 und 700 $cm^{-1}$.

Beispiel 127:

(8R,9S,11S,12R,15R,16S)-9-(4-Phenylbenzyloxy)-11,15-dihydroxy-16,21-dimethyl-5(Z),13-(E)-prostadien-18-in-säuremethylester:

168,1 mg (211 mol) der nach Beispiel 128 dargestellten Verbindung setzt man in Analogie zu Beispiel 36d um und

isoliert nach Aufarbeitung und Reinigung 76,4 mg (119 µmol,65%) der Titelverbindung als farbloses Öl.

IR (Film): 3660-3120, 2970, 2930, 1735, 1490, 1455, 1435, 1320, 1215, 1120, 1070, 760 und 700 cm⁻ '.

Beispiel 128:

(8R,9S,11S,12R,15R,16S)-9-(4-Phenylbenzyloxy)-11-hydroxy-15-(tert.-butyldiphenylsilyloxy)-16,21-dimethyl-5(Z),13(E)-prostadien-18-in-säuremethylester:

309,9 mg (326 mol) der nach Beispiel 129 dargestellten Verbindung setzt man in Analogie zu Beispiel 100 um und isoliert nach Aufarbeitung 178 mg (223 µmol,69%) der Titelverbindung als farbloses Öl.

IR (Film): 3640-3200, 2960, 2930, 2860, 1740, 1490, 1460, 1430, 1375, 1245, 1110, 825, 765, 740 und 705 $cm^{-1}$.

Beispiel 129:

(8R,9S,11R,12R,15R,16S)-9-(4-Phenylbenzyloxy)-11-(4-methylphenylsulfonyloxy)-15-(tert.-butyldiphenylsilyloxy)-16,21-dimethyl-5(Z),13(E)-prostadien-18-in-säuremethylester:

363,2 mg (465 mol) der nach Beispiel 130 dargestellten Verbindung setzt man in Analogie zu Beispiel 14c um und isoliert nach Aufarbeitung und Reinigung 425,3 mg (447 µmol, 98%) der Titelverbindung als farbloses Öl.

IR (Film): 2960, 2930, 2860, 1740, 1715, 1600, 1490, 1430, 1360, 1190, 1175, 1110, 925, 820, 760 und 700 $cm^{-1}$.

Beispiel 130:

(8R,9S,11R,12R,15R,16S)-9-(4-Phenylbenzyloxy)-11-hydroxy-15-(tert.-butyldiphenylsilyloxy)-16,21-dimethyl-5(Z),13(E)-prostadien-18-in-säuremethylester:

657,3 mg (746 µmol) der nach Beispiel 131 dargestellten Verbindung setzt man in Analogie zu Beispiel 56 um und isoliert nach Aufarbeitung und Reinigung 373,3 mg (468 µmol,63%) der Titelverbindung als farbloses Öl.

IR (Film): 3630-3220, 2960, 2930, 1740, 1485, 1430, 1110, 1070, 975, 760 und 700 $cm^{-1}$.

Beispiel 131:

(8R,9S,11R,12R,15R,16S)-9-(4-Phenylbenzyloxy)-11-(tetrahydropyranyloxy)-15-(tert.-butyldiphenylsilyloxy)-16,21-dimethyl-5(Z),13(E)-prostadien-18-in-säuremethylester:

500 mg (778 mol) der nach Beispiel 116 (I, unpolarer Fleck) dargestellten Verbindung setzt man in Analogie zu Beispiel 86 um und isoliert nach Aufarbeitung und Reinigung 670,9 mg (762 µmol,98%) der Titelverbindung als farbloses Öl.

IR (Film): 2960, 2930, 2870, 2860, 1740, 1585, 1470, 1430, 1350, 1110, 1075, 1025, 755 und 700 $cm^{-1}$.

Beispiel 132:

(8R,9S,12R,15S,16S)-9-(4-Phenylbenzyloxy)-15-hydroxy-16,21-dimethyl-5(Z),13(E)-prostadien-18-in-säure:

30,1 mg (55 µmol) der nach Beispiel 133 dargestellten Verbindung verseift man in Analogie zu Beispiel 1 und isoliert nach Aufarbeitung und Reinigung 17,6 mg (33 µmol, 60%) der Titelverbindung als farbloses Öl.

IR (Flsg. cap.): 3600-2700, 2960, 2930, 2870, 1710, 1490, 1455, 1410, 1345, 1240, 1125, 1075, 1010, 975, 760 und 700 $cm^{-1}$.

Beispiel 133:

(8R,9S,12R,15S,16S)-9-(4-Phenylbenzyloxy)-15-hydroxy-16,21-dimethyl-5(Z),13(E)-prostadien-18-in-säuremethylester:

75,8 mg (97 mol) der nach Beispiel 134 dargestellten Verbindung setzt man in Analogie zu Beispiel 36d um und isoliert nach Aufarbeitung und Reinigung 30,1 mg (55µmol,57%) der Titelverbindung als farbloses Öl.

IR (Film): 3680-3160, 2960, 2930, 1735, 1490, 1460, 1430, 1365, 1120, 825, 755 und 700 $cm^{-1}$.

Beispiel 134:

(8R,9S,12R,15S,16S)-9-(4-Phenylbenzyloxy)-15-(tert.-butyldiphenylsilyloxy)-16,21-dimethyl-5(Z),13(E)-prostadien-18-in-säuremethylester:

107,1 mg (112 mol) der nach Beispiel 123 dargestellten Verbindung setzt man in Analogie zu Beispiel 83 um und isoliert nach Aufarbeitung und Reinigung 75,8 mg (97µmol,86%) der Titelverbindung als farbloses Öl.

IR (Film): 2960, 2930, 2860, 1735, 1480, 1455, 1440, 1360, 1110, 1055, 970, 760, 740 und 700 $cm^{-1}$.

Beispiel 135:

(8R,9S,12R,15R,16S)-9-(4-Phenylbenzyloxy)-15-hydroxy-16,21-dimethyl-5(Z),13(E)-prostadien-18-in-säure:

37,3 mg (68 µmol) der nach Beispiel 136 dargestellten Verbindung verseift man in Analogie zu Beispiel 1 und isoliert nach Aufarbeitung und Reinigung 25,8 mg (47 µmol, 71m%) der Titelverbindung als farbloses Öl.

IR (Flsg. cap.): 3700-2400, 2960, 2930, 2870, 1710, 1490, 1455, 1410, 1340, 1240, 1075, 1010, 970, 760 und 700 $cm^{-1}$.

Beispiel 136:

(8R,9S,12R,15R,16S)-9-(4-Phenylbenzyloxy)-15-hydroxy-16,21-dimethyl-5(Z),13(E)-prostadien-18-in-säuremethylester:

90,1 mg (115 mol) der nach Beispiel 137 dargestellten Verbindung setzt man in Analogie zu Beispiel 36d um und isoliert nach Aufarbeitung und Reinigung 37,3 mg (68 µmol,60%) der Titelverbindung als farbloses Öl.

IR (Film): 3700-3100, 2960, 2930, 2860, 1740, 1490, 1460, 1430, 1365, 1115, 1075, 975, 825, 765 und 700 $cm^{-1}$.

Beispiel 137:

(8R,9S,12R,15R,16S)-9-(4-Phenylbenzyloxy)-15-(tert.-butyldiphenylsilyloxy)-16,21-dimethyl-5(Z),13(E)-prostadien-18-in-säuremethylester:

115,4 mg (121 mol) der nach Beispiel 129 dargestellten Verbindung setzt man in Analogie zu Beispiel 83 um und isoliert nach Aufarbeitung und Reinigung 90,1 mg (115 µmol,95%) der Titelverbindung als farbloses Öl.

IR (Film): 2955, 2930, 2855, 1735, 1485, 1455, 1440, 1360, 1110, 1060, 970, 825, 760, 740 und 700 $cm^{-1}$.

Beispiel 138:

7-[(1R,2S,4R,5S)-2-(4-Phenylbenzyloxy)-4-hydroxy-5-(4-fluorbenzyloxymethyl)-cyclopentyl]-5(Z)-heptensäure:

62,7 mg (114 µmol) der nach Beispiel 139 dargestellten Verbindung verseift man in Analogie zu Beispiel 1 und isoliert nach Aufarbeitung und Reinigung 35,9 mg (68 µmol, 59%) der Titelverbindung als farbloses Öl.

IR (Flsg. cap.): 3680-2200, 3030, 3000, 2920, 2860, 1710, 1600, 1510, 1490, 1410, 1350, 1220, 1100, 820, 760 und 700 $cm^{-1}$.

Beispiel 139:

7-[(1R,2S,4R,5S)-2-(4-Phenylbenzyloxy)-4-hydroxy-5-(4-fluorbenzyloxymethyl)-cyclopentyl)-5(Z)-heptensäuremethylester:

403,9 mg (640 mol) der nach Beispiel 140 dargestellten Verbindung setzt man in Analogie zu Beispiel 56 um und isoliert nach Aufarbeitung und Reinigung 309,7 mg (567 μmol,88%) der Titelverbindung als farbloses Öl.

IR (Film): 3680-3200, 2980, 2890, 1735, 1590, 1490, 1210, 1125, 1110, 1020, 765 und 700 $cm^{-1}$.

Beispiel 140:

7-[(1R,2S,4R,5S)-2-(4-Phenylbenzyloxy)-4-(tetrahydropyranyloxy)-5-(4-fluorbenzyloxymethyl)-cyclopentyl]-5(Z) -heptensäuremethylester:

500 mg (956 μmol) der nach Beispiel 57b dargestellten Verbindung löst man in 0,58 ml 4-Fluorbenzylbromid, versetzt mit 0,95 ml 50%iger Kaliumhydroxydlösung und 44 mg Tetabutylammoniumhydrogensulfat und rührt stark 21 Stunden bei Raumtemperatur. Nach dem Ansäuern mit 10%iger Zitronensäure verdünnt man mit Ether, wäscht mit gesättigter Natriumchloridlösung und trocknet über Natriumsulfat. Den nach Filtration und Lösungsmittelabzug erhaltenen Rückstand reinigt man durch Chromatographie an Kieselgel. Man erhält 411,9 mg (653 μmol,68%) der Titelverbindung als farbloses Öl.

IR (Film): 2950, 2870, 1735, 1600, 1510, 1490, 1455, 1440, 1360, 1205, 1020, 975, 820, 760 und 700 $cm^{-1}$.

Beispiel 141:

7-[(1R,2S,4S,5S)-2-(4-Phenylbenzyloxy)-4-hydroxy-5-(4-fluorbenzyloxymethyl)-cyclopentyl]-5(Z)-heptensäure:

55,7 mg (102 μmol) der nach Beispiel 142 dargestellten Verbindung verseift man in Analogie zu Beispiel 1 und isoliert nach Aufarbeitung und Reinigung 34,7 mg (65 μmol,64%) der Titelverbindung als farbloses Öl.

IR (Flsg. cap.): 3680-2200, 3030, 2930, 2870, 1735, 1600, 1510, 1220, 1100, 830, 760 und 700 $cm^{-1}$.

Beispiel 142:

7-[(1R,2S,4S,5S)-2-(4-Phenylbenzyloxy)-4-hydroxy-5-(4-fluorbenzyloxymethyl)-cyclopentyl]-5(Z)-heptensäuremethylester:

160,4 mg (229 mol) der nach Beispiel 143 dargestellten Verbindung setzt man in Analogie zu Beispiel 100 um und isoliert nach Aufarbeitung und Reinigung 55,7 mg (102 μmol,45%) der Titelverbindung als farbloses Öl.

IR (Film): 3700-3180, 2960, 2890, 1740, 1600, 1480, 1380, 1210, 1130, 1110, 1020, 890, 760 und 700 $cm^{-1}$.

Beispiel 143:

7-[(1R,2S,4R,5S)-2-(4-Phenylbenzyloxy)-4-(4-methylphenylsulfonyloxy)-5-(4-fluorbenzyloxymethyl)-cyclopentyl] -5(Z)-heptensäuremethylester:

247 mg (452 mol) der nach Beispiel 139 dargestellten Verbindung setzt man in Analogie zu Beispiel 14c um und isoliert nach Aufarbeitung und Reinigung 260,9 mg (372 μmol, 82%) der Titelverbindung als farbloses Öl.

IR (Film): 2940, 2860, 1735, 1605, 1520, 1490, 1440, 1360, 1245, 1230, 1175, 1095, 830, 765 und 700 $cm^{-1}$.

Beispiel 144:

7-[(1R,2S,5S)-2-(4-Phenylbenzyloxy)-5-(4-fluorbenzyloxymethyl)-cyclopentyl]-5(Z)-heptensäure:

65,7 mg (123 μmol) der nach Beispiel 145 dargestellten Verbindung verseift man in Analogie zu Beispiel 1 und

isoliert nach Aufarbeitung und Reinigung 41 mg (80 µmol,64%) der Titelverbindung als farbloses Öl.

IR (Flsg. cap.): 3680-2400, 3040, 3010, 2940, 2860, 1710, 1600, 1510, 1410, 1220, 1080, 825, 760 und 700 $cm^{-1}$.

Beispiel 145:

7-[(1R,2S,5S)-2-(4-Phenylbenzyloxy)-5-(4-fluorbenzyloxymethyl)-cyclopentyl]-5(Z)-heptensäuremethylester:

100,5 mg (143 mol) der nach Beispiel 143 dargestellten Verbindung setzt man in Analogie zu Beispiel 83 um und isoliert nach Aufarbeitung und Reinigung 65,7 mg (123 µmol,86%) der Titelverbindung als farbloses Öl.

IR (Film): 2940, 2860, 1740, 1610, 1490, 1450, 1440, 1360, 1210, 1025, 760 und 700 $cm^{-1}$.

Beispiel 146:

7-[(1R,2S,4R,5S)-2-(4-Phenylbenzyloxy)-4-hydroxy-5-(4-cyanobenzyloxymethyl)-cyclopentyl] -5(Z)-heptensäure:

80 mg (144 µmol) der nach Beispiel 147 dargestellten Verbindung verseift man in Analogie zu Beispiel 1 und isoliert nach Aufarbeitung und Reinigung 41,5 mg (77 µmol,53%) der Titelverbindung.

IR (Flsg. cap.): 3680-2400, 3030, 3000, 2910, 2860, 2230, 1710, 1610, 1490, 1410, 1250, 1100, 820, 760 und 700 $cm^{-1}$.

Beispiel 147:

7-[(1R,2S,4R,5S)-2-(4-Phenylbenzyloxy)-4-hydroxy-5-(4-cyanobenzyloxymethyl)cyclopentyl ]-5(Z)-heptensäuremethylester:

130,6 mg (206 mol) der nach Beispiel 148 dargestellten Verbindung setzt man in Analogie zu Beispiel 56 um und isoliert nach Aufarbeitung und Reinigung 80 mg (144 µmol,70%) der Titelverbindung.

IR (Film): 3700-3200, 2950, 2880, 2215, 1740, 1610, 1490, 1455, 1370, 1210, 1025, 765 und 700 $cm^{-1}$.

Beispiel 148:

7-[(1R,2S,4R,5S)-2-(4-Phenylbenzyloxy)-4-(tetrahydropyranyloxy)-5-(4-cyanobenzyloxymethyl)-cyclopentyl]-5(Z) -heptensäuremethylester:

159,3 mg (304 µmol) der nach Beispiel 57b dargestellten Verbindung löst man in 0,5 ml Toluol, versetzt mit 299,4 mg (1,52 mmol) 4-Cyanobenzylbromid, 0,3 ml 50%iger Kaliumhydroxydlösung und 13,7 mg Tetabutylammoniumhydrogensulfat und rührt stark 19,5 Stunden bei Raumtemperatur. Nach dem Ansäuern mit 10%iger Zitronensäure verdünnt man mit Ethylacetat, wäscht mit gesättigter Natriumchloridlösung und trocknet über Natriumsulfat. Den nach Filtration und Lösungsmittelabzug erhaltenen Rückstand reinigt man durch Chromatographie an Kieselgel. Man erhält 130,6 mg (206 µmol,67%) der Titelverbindung.

IR (Film): 2950, 2870, 2215, 1735, 1615, 1490, 1455, 1440, 1360, 1205, 1130, 1110, 1020, 975, 820, 765 und 700 $cm^{-1}$.

## Patentansprüche

### Patentanspruche für folgende Vertragsstaaten : BE, CH, LI, DE, DK, FR, GB, IT, LU, NL, SE

**1.** Cyclopentanetherderivate der Formel I,

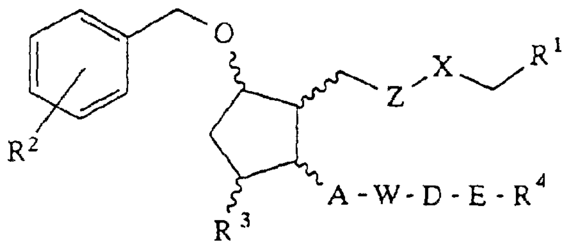

(I),

worin

$R^1$ 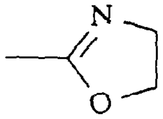, 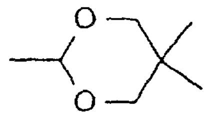, 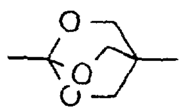, 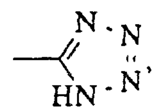,

$COOR^5$, wobei $R^5$ Wasserstoff oder gegebenenfalls durch Halogen, Phenyl, $C_1$-$C_4$-Alkoxy oder Di-($C_1$-$C_4$)-alkylamino substituiertes $C_1$-$C_{10}$-Alkyl, $C_5$-$C_6$-Cycloalkyl, $C_7$-$C_{16}$-Aralkyl, durch Y substituiertes Phenacyl oder $C_6$-$C_{12}$-Aryl oder einen 5- oder 6- gliedrigen heterocyclischen Rest mit wenigstens einem N-, O- oder S-Atom bedeuten kann, oder $-CONHR^7$ mit $R^7$ in der Bedeutung Wasserstoff, $C_1$-$C_{10}$-Alkanoyl oder $C_1$-$C_{10}$-Alkansulfonyl sein kann,

Z eine Direktbindung, (Z)-CH=CH-, (E)-CH=CH-, -C≡C-,

X $-(CH_2)_p-$, $-CH_2-O-$, $-CH_2-S-$,

p 0 bis 5,

$R^2$ Y oder 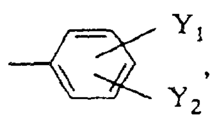,

$R^3$ Wasserstoff, F, $R^6$ oder $OR^6$,

A eine Direktbindung, (Z)-CH=CH-, (E)-CH=CH-, -C≡C-,

W eine Direktbindung, eine $-[(CH_2)_n-V]_q$-Gruppe oder eine $-(CH_2)_n-V-(CH_2)_q-V$-Gruppe, eine freie oder funktionell abgewandelte Hydroxymethylengruppe, eine freie oder funktionell abgewandelte

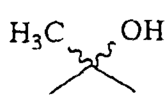 -Gruppe,

wobei die Hydroxygruppe jeweils α- oder β-ständig sein kann,

q 1 oder 2,

n 0 bis 2,

D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, $-(CH_2)_m-NH-SO_2-$,

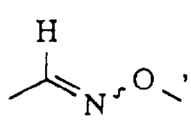, 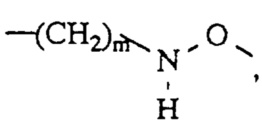, 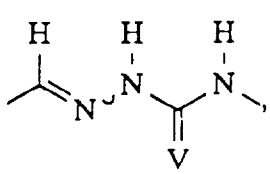,

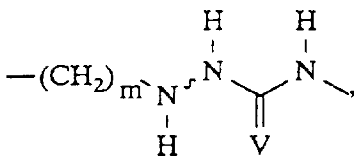 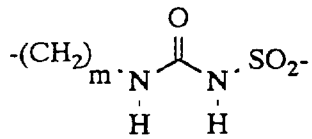,

m 0 bis 2

V ein O- oder S-Atom,

E eine Direktbindung, -C≡C- oder -CH=CR$^8$-, wobei R$^8$ Wasserstoff, $C_1$-$C_5$-Alkyl, Halogen oder Trifluormethyl,

AW, DE unabhängig voneinander eine Direktbindung,

R$^4$ durch Y substituiertes $C_1$-$C_{10}$-Alkyl-, $C_3$-$C_{10}$-Cycloalkyl,

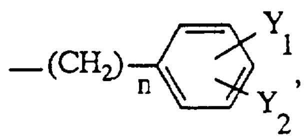,

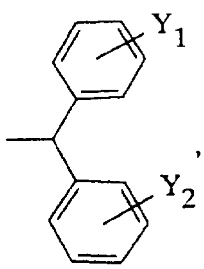, 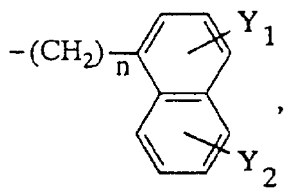, 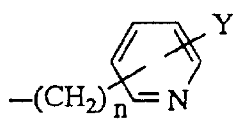, 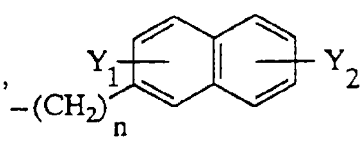,

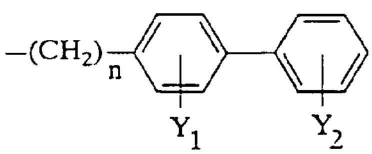, 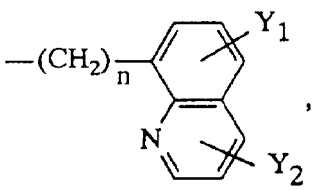, 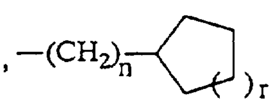,

r 1 oder 2,

$Y_1$ und $Y_2$ gleich oder verschieden sind und Y bedeuten,

Y Wasserstoff, Halogen, CN, $N_3$, $CF_3$, OR$^6$, $NO_2$, -$CH_2$-OR$^6$, COOR$^6$ oder $C_1$-$C_{10}$-Alkyl,

R$^6$ Wasserstoff, $C_1$-$C_{10}$-Alkyl, durch Halogen substituiertes $C_6$-$C_{12}$-Aryl oder $C_7$-$C_{16}$-Aralkyl sein kann und, falls R$^5$ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten,

wobei (9α, 11α, 15S)-9-Benzyloxy-11,15-dihydroxy-5(Z), 13(E)-prostadiensäure, deren 15-Epi-Verbindung sowie 9α-Benzyloxy-15-(t-butyldimethylsilyloxy)-11α-hydroxy-13(E)-prostensäure ausgeschlossen sind.

**2.** Arzneimittel bestehend aus einer oder mehreren Verbindungen des Anspruchs 1 und üblichen Hilfs-, Träger- und Zusatzstoffen.

**3.** Verfahren zur Herstellung von Cyclopentanether-Derivaten der Formel I, dadurch gekennzeichnet, daß man die Hydroxy-Verbindung der Formel II

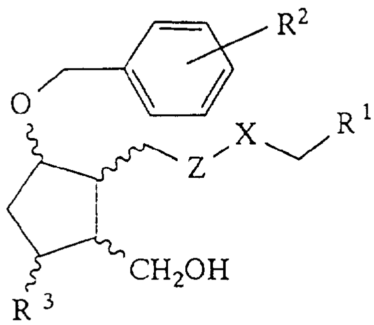
(II),

worin $R^2$, $R^3$, X und Z die oben angegebenen Bedeutungen haben und $R^1$ eine $-COOR^5$-Estergruppe mit $R^5$ in der mit Ausnahme von Wasserstoff oben angegebenen Bedeutung darstellt, mit einer Halogenverbindung der Formel $Hal-W-R^4$ (III), worin Hal, W und $R^4$ die oben genannten Bedeutungen haben oder nach Oxidation mit Oxalylchlorid/DMSO mit einem Dimethylphosphonat der Formel V

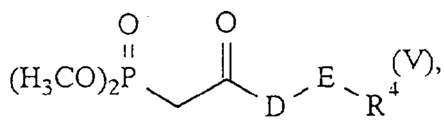
(V),

worin D, E und $R^4$ die oben genannte Bedeutung haben,
wobei für $D-E-R^4$ die Bedeutung $-(CH_2)_4CH_3$ ausgeschlossen ist, wenn -Z-X die Bedeutung (Z)-$CH=CH-(CH_2)_p$- oder die Bedeutung Direktbindung-$(CH_2)_p$- aufweist, in Gegenwart von Natriumhydrid oder Natriumhydrid/Brom umsetzt und anschliessend reduziert und gegebenenfalls Bromwasserstoff abspaltet oder das Oxidationsprodukt aus II und Oxalylchlorid/DMSO mit einem Amin der Formel $H_2N-O-R^4$ (X) oder

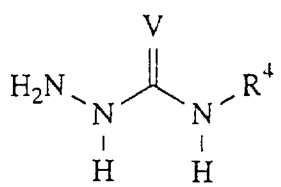
(XI) ,

worin $R^4$ die oben angegebene Bedeutung hat, umsetzt oder nach Oxidation und Umsetzung mit Phosphorsäurediphenylesterazid, 2-(Trimethylsilyl)-ethanol und Tetrabutylammoniumfluorid das intermediäre Amin der Formel VIII

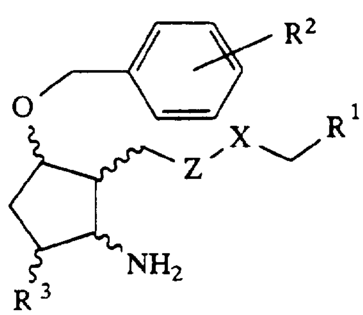
(VIII),

mit einer Verbindung der Formel $Hal-SO_2-R^4$ (IX), worin Hal und $R^4$ die oben genannte Bedeutung hat, oder nach Tosylatbildung, Substitution durch Azid und Reduktion das intermediäre Amin der Formel XII

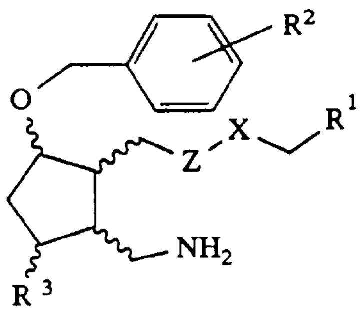

(XII)

mit einer Verbindung der Formel Hal-$SO_2$-$R^4$ (IX), worin Hal und $R^4$ die oben genannte Bedeutung hat umsetzt und die erhaltenen Ester verseift, in Salze überführt, in Cyclodextrinclathrate umwandelt oder mit Liposomen verkapselt.

**Patentanspruche für folgende Vertragsstaaten : AT, GR, ES**

1. Verfahren zur Herstellung von Cyclopentanether-Derivaten der allgemeinen Formel I

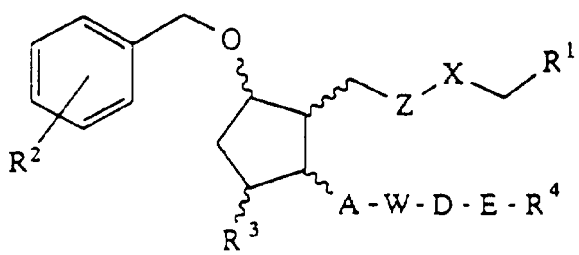

(I),

worin

$R^1$ 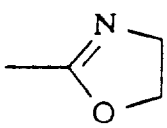 , 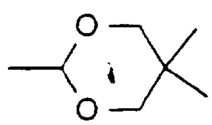 , 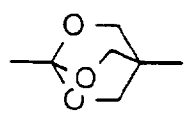 , 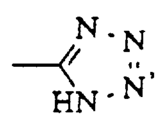

$COOR^5$, wobei $R^5$ Wasserstoff oder gegebenenfalls durch Halogen, Phenyl, $C_1$-$C_4$-Alkoxy oder Di-($C_1$-$C_4$)-alkylamino substituiertes $C_1$-$C_{10}$-Alkyl, $C_5$-$C_6$-Cycloalkyl, $C_7$-$C_{16}$-Aralkyl, durch Y substituiertes Phenacyl oder $C_6$-$C_{12}$-Aryl oder einen 5- oder 6- gliedrigen heterocyclischen Rest mit wenigstens einem N-, O- oder S-Atom bedeuten kann, oder -$CONHR^7$ mit $R^7$ in der Bedeutung Wasserstoff, $C_1$-$C_{10}$-Alkanoyl oder $C_1$-$C_{10}$-Alkansulfonyl sein kann,

Z eine Direktbindung, (Z)-CH=CH-, (E)-CH=CH-, -C≡C-,

X -$(CH_2)_p$-, -$CH_2$-O-, -$CH_2$-S-,

p 0 bis 5,

$R^2$ Y oder 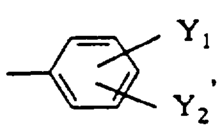 ,

$R^3$ Wasserstoff, F, $R^6$ oder $OR^6$,

A eine Direktbindung, (Z)-CH=CH-, (E)-CH=CH-, -C≡C-,

W eine Direktbindung, eine -[$(CH_2)_n$-V]$_q$-Gruppe oder eine -$(CH_2)_n$-V-$(CH_2)_q$-V-Gruppe, eine freie oder funktionell abgewandelte Hydroxymethylengruppe, eine freie oder funktionell abgewandelte

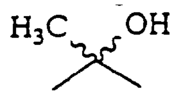 -Gruppe,

wobei die Hydroxygruppe jeweils α- oder β-ständig sein kann,

q 1 oder 2,

n 0 bis 2,

D eine Direktbindung, eine geradkettige gesättigte Alkylengruppe mit 1-5 C-Atomen,

eine verzweigte gesättigte Alkylengruppe oder eine geradkettige oder verzweigte ungesättigte Alkylengruppe mit 2-5 C-Atomen, die gegebenenfalls durch Fluoratome substituiert sein können, $-(CH_2)_m-NH-SO_2-$,

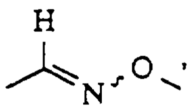, 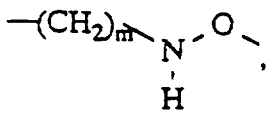, 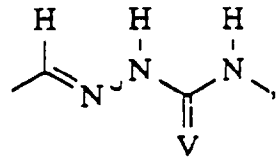,

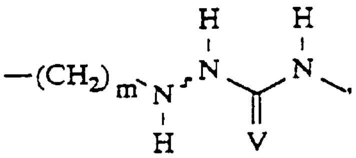, 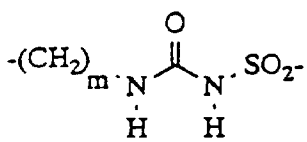,

m 0 bis 2

V ein O- oder S-Atom,

E eine Direktbindung, -C≡C- oder $-CH=CR^8-$, wobei $R^8$ Wasserstoff, $C_1$-$C_5$-Alkyl, Halogen oder Trifluormethyl,

AW, DE unabhängig voneinander eine Direktbindung,

$R^4$ durch Y substituiertes $C_1$-$C_{10}$-Alkyl, $C_3$-$C_{10}$-Cycloalkyl,

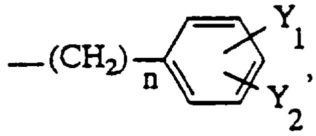,

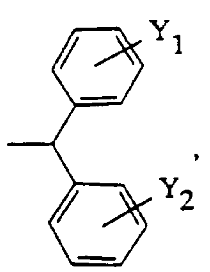, 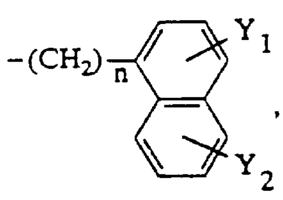, 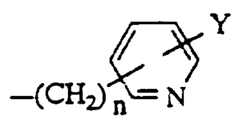, 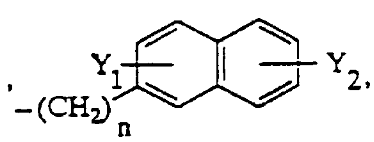,

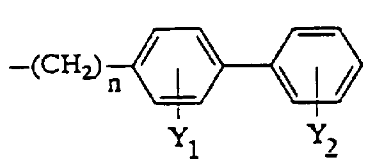, 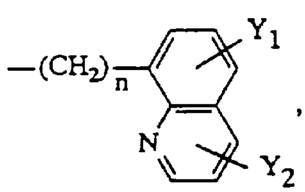, 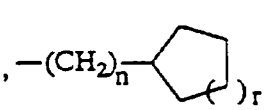,

r 1 oder 2,

$Y_1$ und $Y_2$ gleich oder verschieden sind und Y bedeuten,

Y Wasserstoff, Halogen, CN, $N_3$, $CF_3$, $OR^6$, $NO_2$, $-CH_2-OR^6$, $COOR^6$ oder $C_1-C_{10}$-Alkyl,

$R^6$ Wasserstoff, $C_1-C_{10}$-Alkyl, durch Halogen substituiertes $C_6-C_{12}$-Aryl oder $C_7-C_{16}$-Aralkyl sein kann und, falls $R^5$ Wasserstoff bedeutet, deren Salze mit physiologisch verträglichen Basen , sowie die α-, β- oder γ-Cyclodextrinclathrate, sowie die mit Liposomen verkapselten Verbindungen der Formel I bedeuten,
wobei (9α, 11α, 15S)-9-Benzyloxy-11,15-dihydroxy-5(Z), 13(E)-prostadiensäure, deren 15-Epi-Verbindung sowie 9α-Benzyloxy-15-(t-butyldimethylsilyloxy)-11α-hydroxy-13(E)-prostensäure ausgeschlossen sind.

dadurch gekennzeichnet, daß man die Hydroxy-Verbindung der Formel II

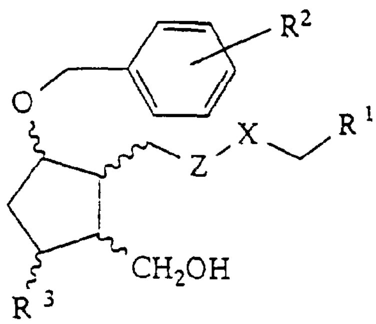
(II),

worin $R^2$, $R^3$, X und Z die oben angegebenen Bedeutungen haben und $R^1$ eine $-COOR^5$-Estergruppe mit $R^5$ in der mit Ausnahme von Wasserstoff oben angegebenen Bedeutung darstellt, mit einer Halogenverbindung der Formel $Hal-W-R^4$ (III), worin Hal, W und $R^4$ die oben genannten Bedeutungen haben oder nach Oxidation mit Oxalylchlorid/DMSO mit einem Dimethylphosphonat der Formel V

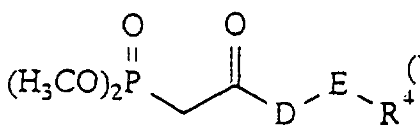
(V),

worin D, E und $R^4$ die oben genannte Bedeutung haben,
wobei für $D-E-R^4$ die Bedeutung $-(CH_2)_4CH_3$ ausgeschlossen ist, wenn -Z-X die Bedeutung (Z)-$CH=CH-(CH_2)_p$- oder die Bedeutung Direktbindung-$(CH_2)$p- aufweist, in Gegenwart von Natriumhydrid oder Natriumhydrid/Brom umsetzt und anschliessend reduziert und gegebenenfalls Bromwasserstoff abspaltet oder das Oxidationsprodukt aus II und Oxalylchlorid/DMSO mit einem Amin der Formel $H_2N-O-R^4$ (X) oder

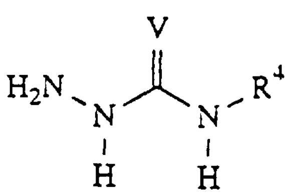
(XI) ,

worin $R^4$ die oben angegebene Bedeutung hat, umsetzt oder nach Oxidation und Umsetzung mit Phosphorsäurediphenylesterazid, 2-(Trimethylsilyl)-ethanol und Tetrabutylammoniumfluorid das intermediäre Amin der Formel VIII

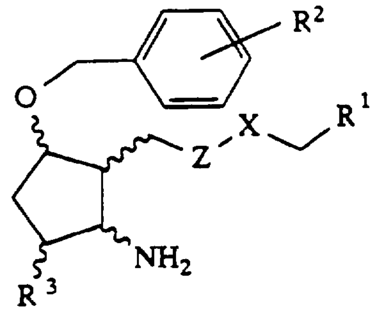

(VIII),

mit einer Verbindung der Formel Hal-$SO_2$-$R^4$ (IX), worin Hal und $R^4$ die oben genannte Bedeutung hat, oder nach Tosylatbildung, Substitution durch Azid und Reduktion das intermediäre Amin der Formel XII

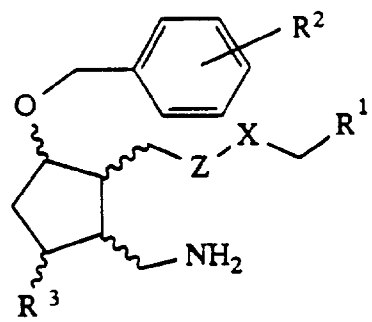

(XII)

mit einer Verbindung der Formel Hal-$SO_2$-$R^4$ (IX), worin Hal und $R^4$ die oben genannte Bedeutung hat umsetzt und die erhaltenen Ester verseift, in Salze überführt, in Cyclodextrinclathrate umwandelt oder mit Liposomen verkapselt.

**Claims**

**Claims for the following Contracting States : BE, CH, LI, DE, DK, FR, GB, IT, LU, NL, SE**

1. Cyclopentane ether derivatives of the formula I

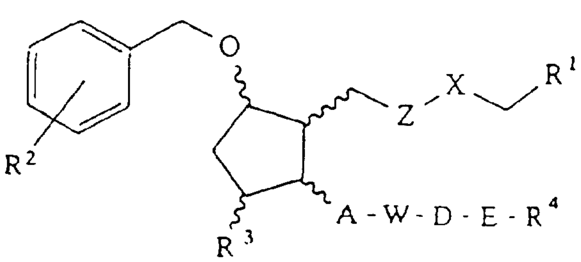

(I),

wherein $R^1$ is

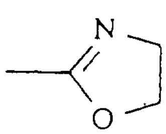 , 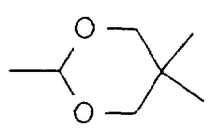 , 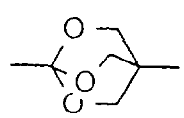 , 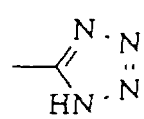 ,

$COOR^5$ wherein $R^5$ is hydrogen or optionally halo-, phenyl-, $C_1$-$C_4$alkoxy- or di($C_1$-$C_4$)alkylamino-substituted $C_1$-$C_{10}$alkyl, $C_5$-$C_6$cycloalkyl, $C_7$-$C_{16}$aralkyl, Y-substituted phenacyl or $C_6$-$C_{12}$aryl, or a 5- or 6-membered heterocyclic radical having at least one N, O or S atom, or $R^1$ is -$CONHR^7$ wherein $R^7$ may be hydrogen, $C_1$-$C_{10}$alkanoyl or $C_1$-$C_{10}$ alkanesulphonyl,

Z is a direct bond, (Z)-CH=CH-, (E)-CH=CH-, or -C≡C-,

X is $-(CH_2)_p-$, $-CH_2-O-$ or $-CH_2-S-$,

p is from 0 to 5,

$R^2$ is Y or

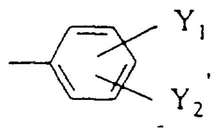

,

$R^3$ is hydrogen, F, $R^6$ or $OR^6$,

A is a direct bond, (Z)-CH=CH-, (E)-CH=CH- or -C≡C-,

W is a direct bond, a $-[(CH_2)_n-V]_q-$ group or

a $-(CH_2)_n-V-(CH_2)_q-V-$ group, a free or functionally modified hydroxymethylene group or a free or functionally modified

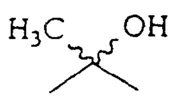

group,

it being possible for the hydroxy group in each case to be in the α- or β- configuration,

q is 1 or 2,

n is from 0 to 2,

D is a direct bond, a straight-chained saturated alkylene group having from 1 to 5 carbon atoms, a branched saturated alkylene group or a straight-chained or branched unsaturated alkylene group having from 2 to 5 carbon atoms which may optionally be substituted by fluorine atoms, or is $-(CH_2)_m-NH-SO_2-$,

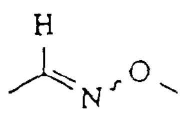

,

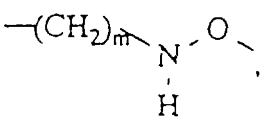

,

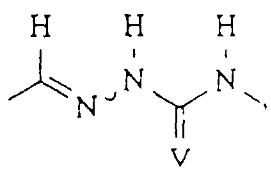

,

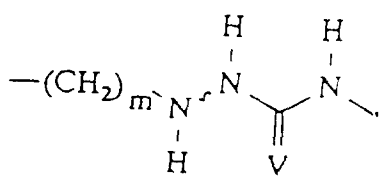

or

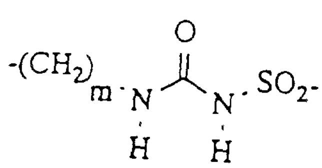

,

m is from 0 to 2,

V is an O or S atom,

E is a direct bond, -C≡C- or $-CH=CR^8-$ wherein $R^8$ is hydrogen, $C_1$-$C_6$alkyl, halogen or trifluoromethyl,

AW and DE are, each independently of the other, a direct bond,

$R^4$ is Y-substituted $C_1$-$C_{10}$alkyl, $C_3$-$C_5$cycloalkyl,

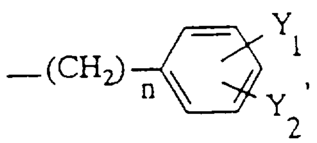
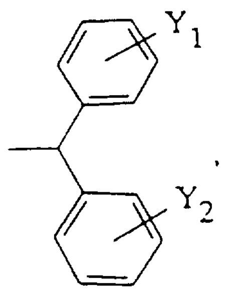
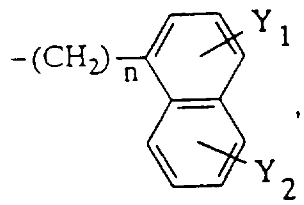
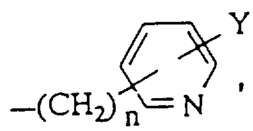

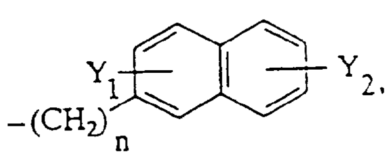
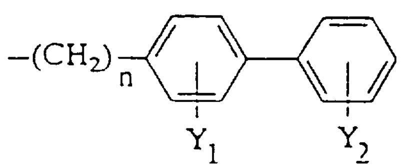
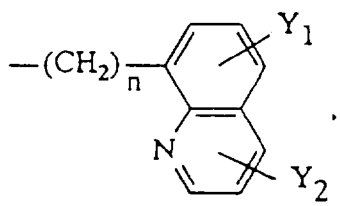

or

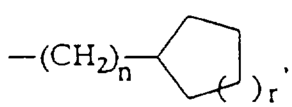

r is 1 or 2,

$Y_1$ and $Y_2$ are identical or different and represent Y,

Y is hydrogen, halogen, CN, $N_3$, $CF_3$, $OR^6$, $NO_2$, $-CH_2-OR^6$, $COOR^6$ or $C_1$-$C_{10}$alkyl,

$R^6$ may be hydrogen, $C_1$-$C_{10}$alkyl, halo-substituted $C_6$-$C_{12}$aryl or $C_7$-$C_{16}$aralkyl, and, when $R^5$ is hydrogen, salts thereof with physiologically tolerable bases, and also the α-, β- or γ-cyclodextrin clathrates and the compounds of formula I encapsulated with liposomes,

(9α,11α,15S)-9-benzyloxy-11,15-dihydroxy-5(Z),13(E)prostadienoic acid, its 15-epi compound and also 9α-benzyloxy-15-(tert-butyldimethylsilyloxy)-11α-hydroxy13(E)prostenoic acid being excluded.

**2.** Medicaments comprising one or more compounds of claim 1 and customary excipients, carriers and adjuvants.

**3.** Process for the preparation of cyclopentane ether derivatives of the formula I, characterised in that the hydroxy compound of the formula II

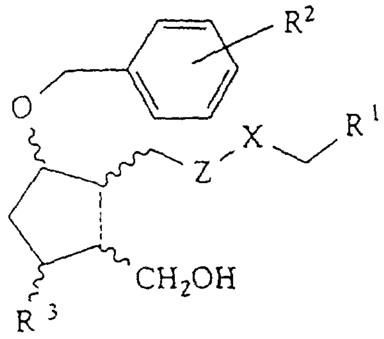

(II),

wherein $R^2$, $R^3$, X and Z have the meanings given above and $R^1$ is a $-COOR^5$ ester group wherein $R^5$ has the meanings given above with the exception of hydrogen, is reacted in the presence of sodium hydride or sodium hydride/bromine with a halogen compound of the formula $Hal-W-R^4$ (III) wherein Hal, W and $R^4$ have the meanings given above, or, after oxidation with oxalyl chloride/-DMSO, with a dimethyl phosphonate of the formula V

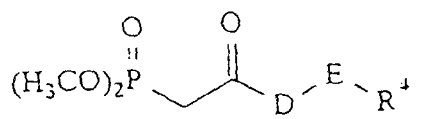

(V),

wherein D, E and $R^4$ have the meanings given above, the meaning $-(CH_2)_4CH_3$ for $D-E-R^4$ being excluded when -Z-X is (Z)-CH=CH-$(CH_2)_p$- or is a direct bond $-(CH_2)_p-$, and the reaction product is then reduced and optionally hydrogen bromide is removed, or the product of the oxidation of II and oxalyl chloride/DMSO is reacted with an amine of the formula $H_2N-O-R^4$ (X) or

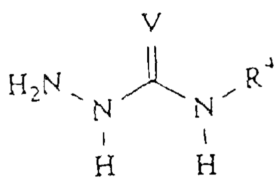

(XI)

wherein $R^4$ has the meaning given above or, after oxidation and reaction with phosphoric acid diphenyl ester azide, 2-(trimethylsilyl)ethanol and tetrabutylammonium fluoride, the intermediate amine of the formula VIII

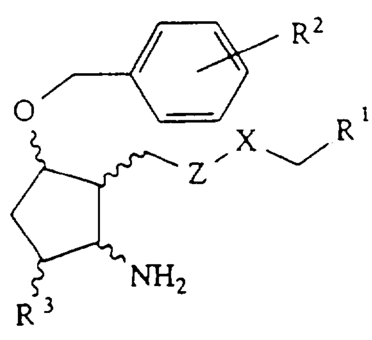

(VIII)

is reacted with a compound of the formula $Hal-SO_2-R^4$ (IX) wherein Hal and $R^4$ have the meanings given above or, after tosylate formation, substitution by azide and reduction, the intermediate amine of the formula XII

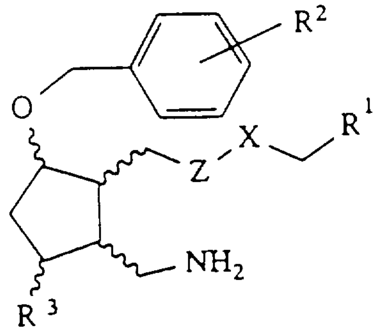

(XII)

is reacted with a compound of the formula Hal-$SO_2$-$R^4$ (IX) wherein Hal and $R^4$ have the meanings given above, and the resulting esters are hydrolysed, converted into salts, converted into cyclodextrin clathrates or encapsulated with liposomes.

**Claims for the following contracting States : AT, GR,ES**

1. Process for the preparation of cyclopentane ether derivatives of the general formula I

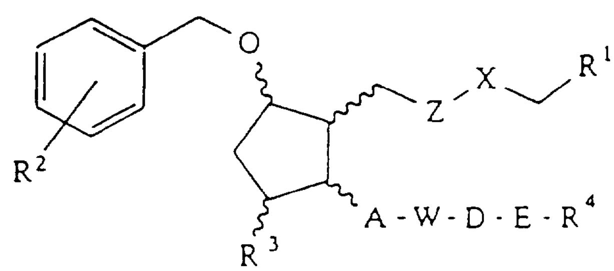

(I),

wherein

$R^1$ is

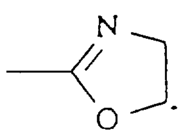 , 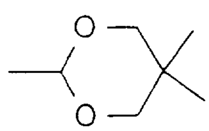 , 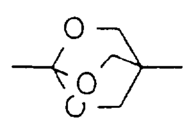 , 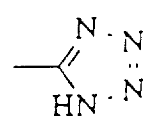 ,

COOR$^5$ wherein $R^5$ is hydrogen or optionally halo-, phenyl-, $C_1$-$C_4$alkoxy- or di($C_1$-$C_4$)alkylamino-substituted $C_1$-$C_{10}$alkyl, $C_5$-$C_6$cycloalkyl, $C_7$-$C_{16}$aralkyl, Y-substituted phenacyl or $C_6$-$C_{12}$aryl, or a 5- or 6-membered heterocyclic radical having at least one N, O or S atom, or $R^1$ is -CONHR$^7$ wherein $R^7$ may be hydrogen, $C_1$-$C_{10}$alkanoyl or $C_1$-$C_{10}$alkanesulphonyl, Z is a direct bond, (Z)-CH=CH-, (E) -CH=CH-, or -C≡C-, X is -$(CH_2)_p$-, -$CH_2$-O- or -$CH_2$-S-, p is from 0 to 5,

$R^2$ is Y or

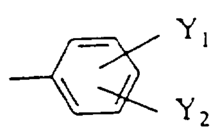 ,

$R^3$ is hydrogen, F, $R^6$ or OR$^6$,

A is a direct bond, (Z)-CH=CH-, (E)-CH=CH- or -C≡C-,

W is a direct bond, a $-[(CH_2)_n-V]_q-$ group or

a $-(CH_2)_n-V-(CH_2)_q-V-$ group, a free or functionally modified hydroxymethylene group or a free or functionally modified

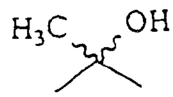 group,

it being possible for the hydroxy group in each case to be in the α- or β- configuration,

q is 1 or 2,

n is from 0 to 2,

D is a direct bond, a straight-chained saturated alkylene group having from 1 to 5 carbon atoms, a branched saturated alkylene group or a straight-chained or branched unsaturated alkylene group having from 2 to 5 carbon atoms which may optionally be substituted by fluorine atoms, or is $-(CH_2)_m-NH-SO_2-$,

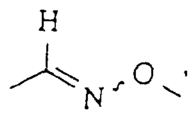

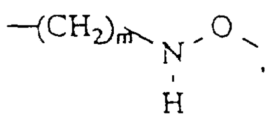, 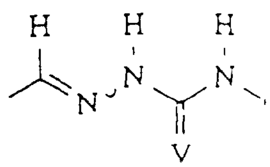, 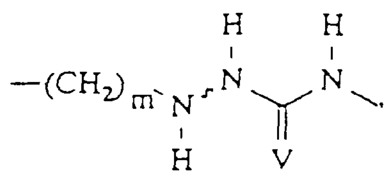

or

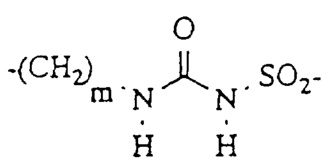,

m is from 0 to 2,

V is an O or S atom,

E is a direct bond, -C≡C- or $-CH=CR^8-$ wherein $R^8$ is hydrogen, $C_1$-$C_5$alkyl, halogen or trifluoromethyl, AW and DE are, each independently of the other, a direct bond,

$R^4$ is Y-substituted $C_1$-$C_{10}$alkyl, $C_3$-$C_{10}$cycloalkyl,

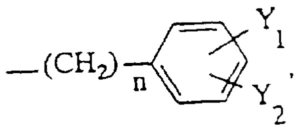,
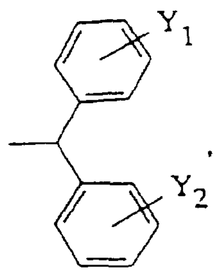,
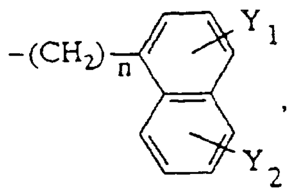,
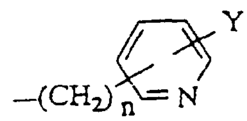,

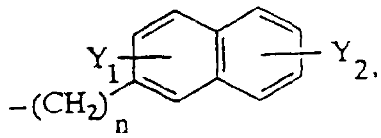.
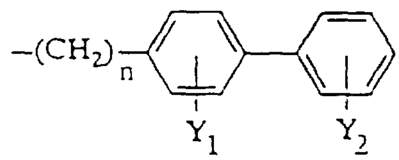,
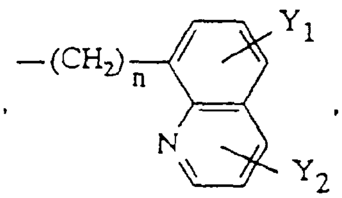,

or

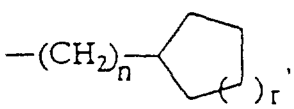,

| | |
|---|---|
| r | is 1 or 2, |
| $Y_1$ and $Y_2$ | are identical or different and represent Y, |
| Y | is hydrogen, halogen, CN, $N_3$, $CF_3$, $OR^6$, $NO_2$, $-CH_2-OR^6$, $COOR^6$ or $C_1$-$C_{10}$alkyl, |
| $R^6$ | may be hydrogen, $C_1$-$C_{10}$alkyl, halo-substituted $C_6$-$C_{12}$aryl or $C_7$-$C_{16}$aralkyl, and, when $R^5$ is hydrogen, salts thereof with physiologically tolerable bases, and also the α-, β- or γ-cyclodextrin clathrates and the compounds of formula I encapsulated with liposomes, |

(9α,11α,15S)-9-benzyloxy-11,15-dihydroxy-5(Z),13(E)prostadienoic acid, its 15-epi compound and also 9α-benzyloxy-15-(tert-butyldimethylsilyloxy)-11α-hydroxy-13(E)prostenoic acid being excluded, which process is characterised in that the hydroxy compound of the formula II

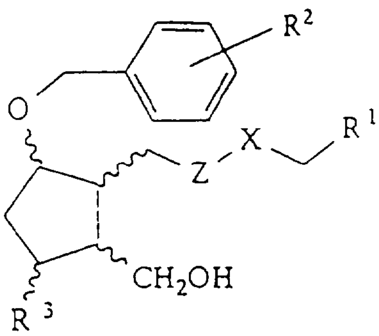

(II)

wherein $R^2$, $R^3$, X and Z have the meanings given above and $R^1$ is a $-COOR^5$ ester group wherein $R^5$ has the

meanings given above with the exception of hydrogen, is reacted in the presence of sodium hydride or sodium hydride/bromine with a halogen compound of the formula Hal-W-$R^4$ (III) wherein Hal, W and $R^4$ have the meanings given above, or, after oxidation with oxalyl chloride/DMSO, with a dimethyl phosphonate of the formula V

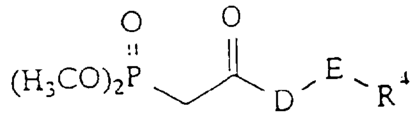

(V)

wherein D, E and $R^4$ have the meanings given above, the meaning $-(CH_2)_4CH_3$ for D-E-$R^4$ being excluded when -Z-X is (Z)-CH=CH-$(CH_2)_p$- or is a direct bond -$(CH_2)_p$-, and the reaction product is then reduced and optionally hydrogen bromide is removed, or the product of the oxidation of II and oxalyl chloride/DMSO is reacted with an amine of the formula $H_2N$-O-$R^4$ (X) or

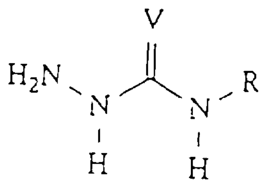

(XI)

wherein $R^4$ has the meaning given above or, after oxidation and reaction with phosphoric acid diphenyl ester azide, 2-(trimethylsilyl)ethanol and tetrabutylammonium fluoride, the intermediate amine of the formula VIII

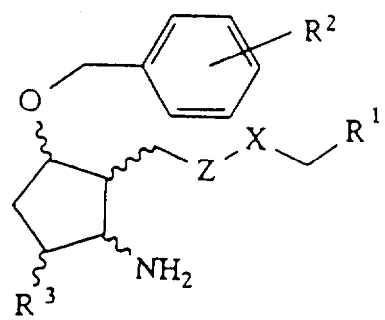

(VIII)

is reacted with a compound of the formula Hal-$SO_2$-$R^4$ (IX) wherein Hal and $R^4$ have the meanings given above or, after tosylate formation, substitution by azide and reduction, the intermediate amine of the formula XII

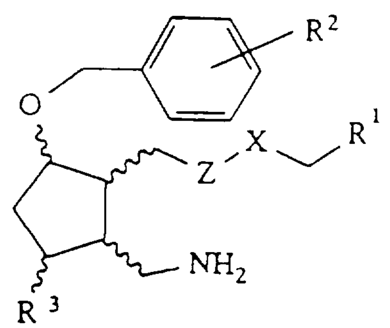

(XII)

is reacted with a compound of the formula Hal-$SO_2$-$R^4$ (IX) wherein Hal and $R^4$ have the meanings given above, and the resulting esters are hydrolysed, converted into salts, converted into cyclodextrin clathrates or encapsulated with liposomes.

## Revendications

**Revendications pour les Etats contractants suivants : BE, CH, LI, DE, DK, FR, GB, IT, LU, NL, SE**

**1.** Dérivés d'éthers de cyclopentane de formule I

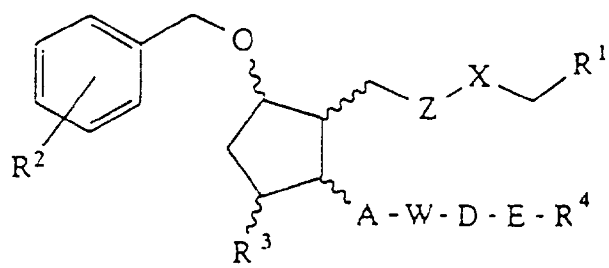

(I),

dans laquelle

$R^1$ est

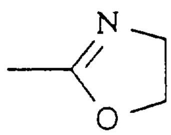, 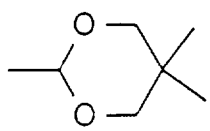, 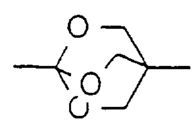, 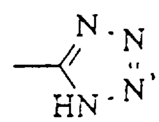

$COOR^5$, où $R^5$ peut être un atome d'hydrogène ou un radical alkyle en $C_1$-$C_{10}$, cycloalkyle en $C_5$-$C_6$, aralkyle en $C_7$-$C_{16}$, éventuellement substitués par des substituants halogéno, phényle, alcoxy en $C_1$-$C_4$, ou di-(alkyle en $C_1$-$C_4$)amino, phénacyle substitué par Y ou aryle en $C_6$-$C_{12}$, ou encore un radical hétérocyclique à 5 ou 6 chaînons ayant au moins un atome N, O ou S, ou encore -$CONHR^7$, où $R_7$ peut être un hydrogène ou un radical alcanoyle en $C_1$-$C_{10}$ ou alcanesulfonyle en $C_1$-$C_{10}$,
Z est une liaison directe, (Z)-CH=CH-, (E)-CH=CH-, -C≡C-,
X est $-(CH_2)_p-$, $-CH_2-O-$, $-CH_2-S-$,
p vaut de 0 à 5,
$R^2$ est Y ou

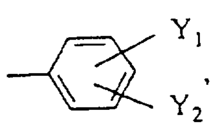

$R^3$ est un hydrogène, F, $R^6$ , ou $OR^6$,
A est une liaison directe, (Z)-CH=CH-, (E)-CH=CH-, -C≡C-,
W est une liaison directe, un groupe $-[(CH_2)_n-V]_q-$ ou un groupe $-(CH_2)n-V-(CH_2)_q-V-$, un groupe hydroxyméthylène libre ou fonctionnalisé, un groupe

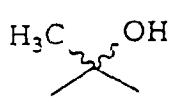

libre ou fonctionnalisé, le groupe hydroxy pouvant être en position α ou en position β,
q vaut 1 ou 2,
n vaut 0 à 2,
D est une liaison directe, un groupe alkylène saturé à chaîne droite ayant de 1 à 5 atomes de carbone, un groupe alkylène saturé ramifié, ou encore un groupe alkylène insaturé à chaîne droite ou ramifiée ayant de 2 à 5 atomes de carbone, qui peuvent être éventuellement substitués par des atomes de fluor,

$-(CH_2)_m-NH-SO_2-$, 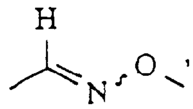 , 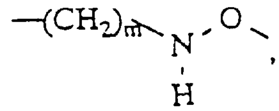 , 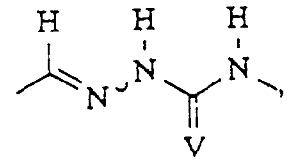 ,

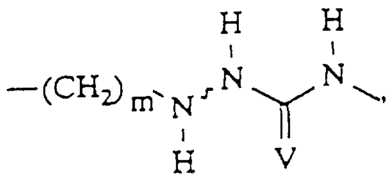 , 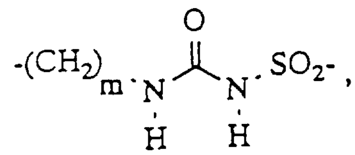 ,

m vaut de 0 à 2,

V est un atome d'oxygène ou de soufre,

E est une liaison directe, -C≡C- ou $-CH=CR^{8}-$, où $R^8$ est un hydrogène, un radical alkyle en $C_1$-$C_5$, halogéno ou trifluoro-méthyle,

AW, DE, indépendamment l'un de l'autre, représentent une liaison directe,

$R^4$ est un radical alkyle en $C_1$-$C_{10}$, cycloalkyle en $C_3$-$C_{10}$ substitué par Y,

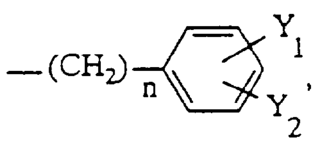 ,

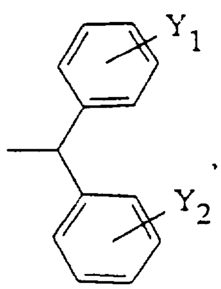 , 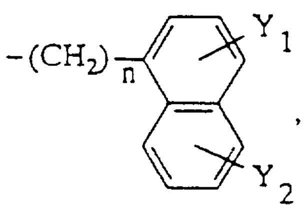 , 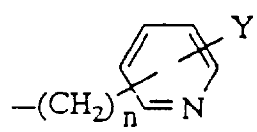 , 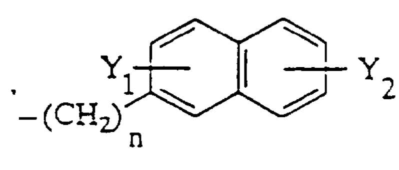

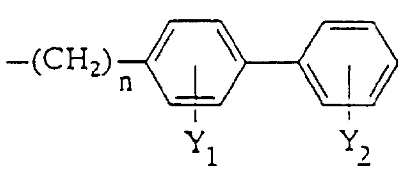 , 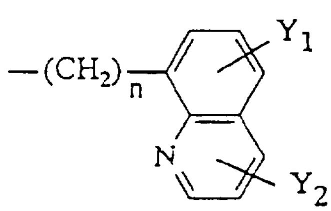 , 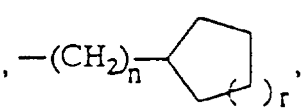 ,

r vaut 1 ou 2,

$Y_1$ ou $Y_2$ sont identiques ou différents et représentent Y,

Y peut être un hydrogène ou un radical halogéno, CN, $N_3$, $CF_3$, $OR^6$, $NO_2$, $-CH_2-OR^6$, $COOR_6$ ou alkyle en $C_1$-$C_{10}$,

$R^6$ peut être un hydrogène ou un radical alkyle en $C_1$-$C_{10}$, aryle en $C_6$-$C_{12}$ ou aralkyle en $C_7$-$C_{16}$ substitués par des halogènes, et, si $R_5$ est un hydrogène, leurs sels avec des bases physiologiquement tolérées, ainsi que les clathrates d'α-, de β- ou de γ-cyclodextrine, ainsi que les composés de formule I encapsulés avec des liposomes,

à l'exclusion de l'acide (9α,11α,15S)-9-benzyloxy-11,15-dihydroxy-5(Z),13(E)-prostadiénoïque, son dérivé 15-épi, ainsi que l'acide 9α-benzyloxy-15-(tert-butyldiméthylsilyloxy)-11a-hydroxy-13(E)-prosténoïque.

2. Médicament constitué d'un ou plusieurs composés selon la revendication 1, et d'adjuvants, excipients et additifs usuels.

3. Procédé pour préparer des dérivés d'éthers de cyclopentane de formule I, caractérisé en ce qu'on fait réagir le composé hydroxylé de formule II

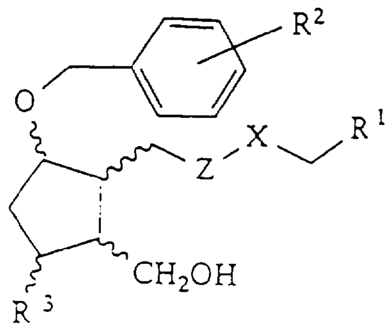

(II),

dans laquelle $R^2$, $R^3$, X et Z ont les significations données ci-dessus, et $R^1$ est un groupe ester $-COOR^5$, $R^5$ ayant les significations données ci-dessus, à l'exception de l'hydrogène, avec un composé halogéné de formule $Hal-W-R^4$ (III), dans laquelle Hal, W et $R^4$ ont les significations données ci-dessus, ou encore, après oxydation avec du chlorure d'oxalyle/DMSO, avec un diméthylphosphonate de formule V :

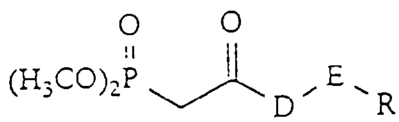

(V),

dans laquelle D, E et $R^4$ ont les significations données ci-dessus,

où la signification $-(CH_2)_4CH_3$ est exclue pour $D-ER^4$ quand -Z-X représente (Z)-$CH=CH-(CH_2)_p$-, ou encore représente une liaison directe $-(CH_2)_p$-, en présence d'hydrure de sodium ou d'hydrure de sodium/brome, puis on procède à une réduction, et on élimine éventuellement le bromure d'hydrogène, ou encore on fait réagir le produit d'oxydation du composé (II) et du chlorure d'oxalyle/DMSO avec une amine de formule $H_2N-O-R^4$ (X) ou

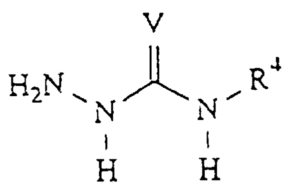

(XI) ,

où $R^4$ a les significations données ci-dessus, ou encore, après oxydation et réaction avec du phosphate de diphényle-azide, du 2-(triméthylsilyl)-éthanol et du fluorure de tétrabutylammonium, on fait réagir l'amine intermédiaire de formule VIII :

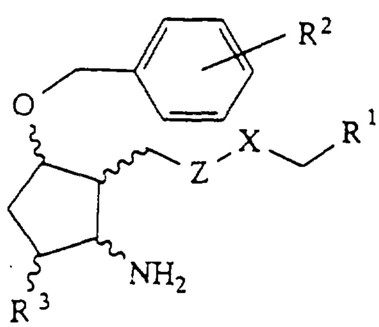

(VIII),

avec un composé de formule $Hal-SO_2-R^4$ (IX), dans laquelle Hal et $R^4$ ont les significations données ci-dessus, ou encore, après formation d'un tosylate, substitution par un azide et réduction, on fait réagir l'amine intermédiaire de formule XII :

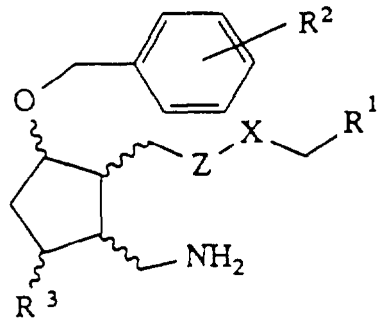

(XII)

avec un composé de formule Hal-$SO_2$-$R^4$ (IX) dans laquelle Hal et $R^4$ ont les significations données ci-dessus, et on saponifie les esters obtenus, on les transforme en des sels, on les convertit en clathrates de cyclodextrine ou encore on les encapsule à l'aide de liposomes.

**Revendications pour les Etats contractants suivants : AT, GR, ES**

1. Procédé pour préparer des dérivés d'éthers de cyclopentane de formule I

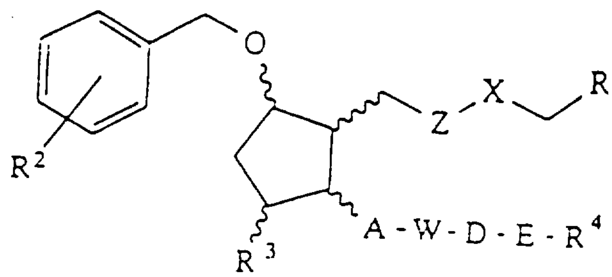

(I),

dans laquelle

$R^1$ est

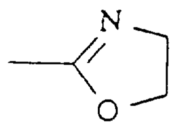, 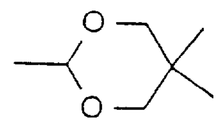, 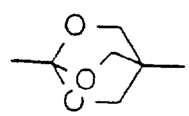, 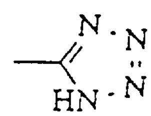

COOR$^5$, où $R^5$ peut être un atome d'hydrogène ou un radical alkyle en $C_1$-$C_{10}$, cycloalkyle en $C_5$-$C_6$, aralkyle en $C_7$-$C_{16}$, éventuellement substitués par des substituants halogéno, phényle, alcoxy en $C_1$-$C_4$, ou di-(alkyle en $C_1$-$C_4$)amino, phénacyle substitué par Y ou aryle en $C_6$-$C_{12}$, ou encore un radical hétérocyclique à 5 ou 6 chaînons ayant au moins un atome N, O ou S, ou encore -CONHR$^7$, où $R^7$ peut être un hydrogène ou un radical alcanoyle en $C_1$-$C_{10}$ ou alcanesulfonyle en $C_1$-$C_{10}$,
Z est une liaison directe, (Z)-CH=CH-, (E)-CH=CH-, -C≡C-,
X est -$(CH_2)_p$-, -$CH_2$-O-, -$CH_2$-S-,
p vaut de 0 à 5,
R $^2$est Y ou

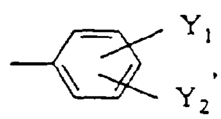,

$R^3$ est un hydrogène, F, $R^6$, ou OR$^6$,
A est une liaison directe, (Z)-CH=CH-, (E)-CH=CH-, -C≡C-,
W est une liaison directe, un groupe -[$(CH_2)_n$-V]$_q$- ou un groupe -$(CH_2)_n$-V-$(CH_2)_q$-V-, un groupe hydroxyméthylène libre ou fonctionnalisé, un groupe

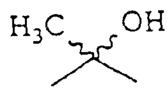

libre ou fonctionnalisé, le groupe hydroxy pouvant être en position α ou en position β,

q vaut 1 ou 2,

n vaut 0 à 2,

D est une liaison directe, un groupe alkylène saturé à chaîne droite ayant de 1 à 5 atomes de carbone, un groupe alkylène saturé ramifié, ou encore un groupe alkylène insaturé à chaîne droite ou ramifiée ayant de 2 à 5 atomes de carbone, qui peuvent être éventuellement substitués par des atomes de fluor,

$-(CH_2)_m-NH-SO_2-$, 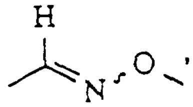 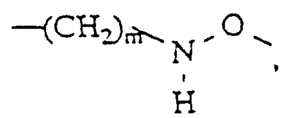, 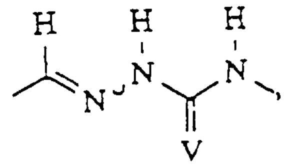

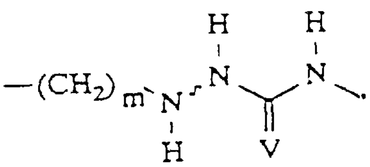 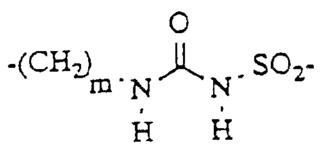

m vaut de 0 à 2,

V est un atome d'oxygène ou de soufre,

E est une liaison directe, -C≡C- ou $-CH=CR^8-$, où $R^8$ est un hydrogène, un radical alkyle en $C_1$-$C_5$, halogéno ou trifluoro-méthyle,

AW, DE, indépendamment l'un de l'autre, représentent une liaison directe,

$R^4$ est un radical alkyle en $C_1$-$C_{10}$ , cycloalkyle en $C_3$-$C_{10}$, substitué par Y,

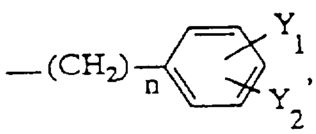

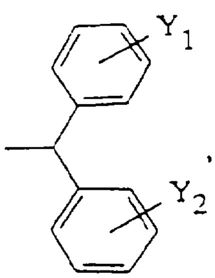, 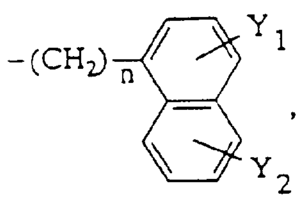, 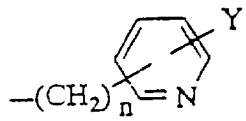, 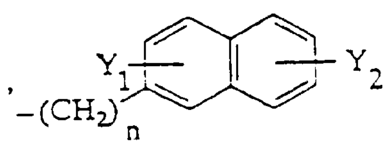

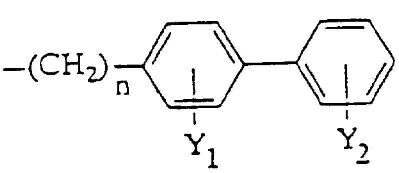, 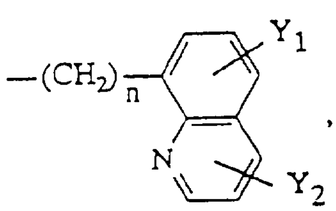, 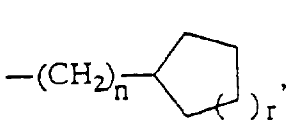

r vaut 1 ou 2,

$Y_1$ ou $Y_2$ sont identiques ou différents et représentent Y,

Y peut être un hydrogène ou un radical halogéno, CN, $N_3$, $CF_3$, $OR^6$, $NO_2$, $-CH_2-OR^6$, $COOR^6$ ou alkyle en $C_1$-$C_{10}$,

$R^6$ peut être un hydrogène ou un radical alkyle en $C_1$-$C_{10}$, aryle en $C_6$-$C_{12}$ ou aralkyle en $C_7$-$C_{16}$ substitués par des halogènes, et, si $R^5$ est un hydrogène, leurs sels avec des bases physiologiquement tolérées, ainsi que les clathrates d'α-, de β- ou de γ-cyclodextrine, ainsi que les composés de formule I encapsulés avec des

liposomes,
à l'exclusion de l'acide (9α,11α,15S)-9-benzyloxy-11,15-dihydroxy-5(Z),13(E)-prostadiénoïque, son dérivé 15-épi, ainsi que l'acide 9α-benzyloxy-15-(tert-butyldiméthylsilyloxy)-11α-hydroxy-13(E)-prosténoïque, caractérisé en ce qu'on fait réagir le composé hydroxylé de formule II

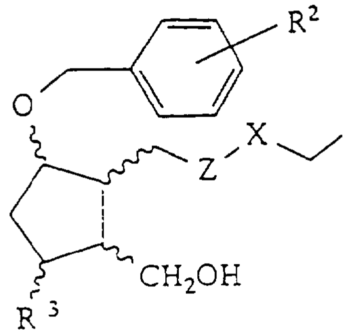 (II),

dans laquelle $R^2$, $R^3$, X et Z ont les significations données ci-dessus, et $R^1$ est un groupe ester $-COOR^5$, $R^5$ ayant les significations données ci-dessus, à l'exception de l'hydrogène, avec un composé halogéné de formule $Hal-W-R^4$ (III), dans laquelle Hal, W et $R^4$ ont les significations données ci-dessus, ou encore, après oxydation avec du chlorure d'oxalyle/DMSO, avec un diméthylphosphonate de formule V

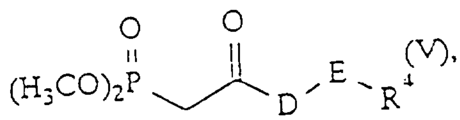 (V),

dans laquelle D, E et $R^4$ ont les significations données ci-dessus,
où la signification $-(CH_2)_4CH_3$ est exclue pour $D-E-R^4$ quand -Z-X représente (Z)-CH=CH-$(CH_2)_p$-, ou encore représente une liaison directe $-(CH_2)_p-$,
en présence d'hydrure de sodium ou d'hydrure de sodium/brome, puis on procède à une réduction, et on élimine éventuellement le bromure d'hydrogène, ou encore on fait réagir le produit d'oxydation du composé (II) et du chlorure d'oxalyle/DMSO avec une amine de formule $H_2N-O-R^4$ (X) ou

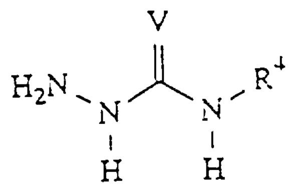 (XI) ,

où $R^4$ a les significations données ci-dessus, ou encore, après oxydation et réaction avec du phosphate de diphényle-azide, du 2-(triméthylsilyl)-éthanol et du fluorure de tétrabutylammonium, on fait réagir l'amine intermédiaire de formule VIII

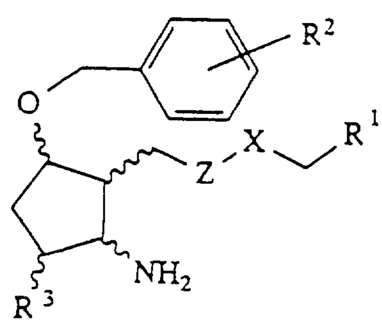 (VIII),

avec un composé de formule $Hal-SO_2-R^4$ (IX), dans laquelle Hal et $R^4$ ont les significations données ci-dessus, ou encore, après formation d'un tosylate, substitution par un azide et réduction, on fait réagir l'amine intermédiaire de formule XII :

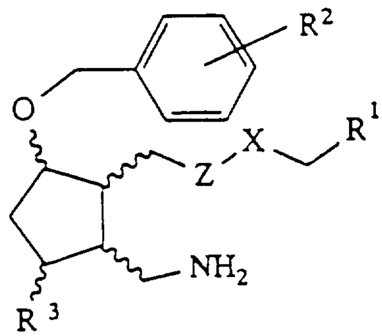

(XII)

avec un composé de formule Hal-$SO_2$-$R^4$ (IX) dans laquelle Hal et $R^4$ ont les significations données ci-dessus, et on saponifie les esters obtenus, on les transforme en des sels, on les convertit en clathrates de cyclodextrine ou encore on les encapsule à l'aide de liposomes.